# EUROPEAN PATENT APPLICATION

(11) **EP 4 317 433 A1**
(43) Date of publication of application: **07.02.2024**
(21) Application number: 22778970.8
(22) Date of filing: 29.03.2022
(51) Int. Cl.: C12N 15/113, A61K 31/7105, A61P 35/00

(54) **NUCLEIC ACID DELIVERY SYSTEM AND APPLICATION THEREOF**

(30) Priority: 29.03.2021 CN 202110336983; 29.03.2021 CN 202110335617; 29.03.2021 CN 202110336982
(71) Applicant: Nanjing University, Nanjing, Jiangsu 210023 (CN)
(72) Inventor: ZHANG, Chenyu, Nanjing, Jiangsu 210023 (CN); CHEN, Xi, Nanjing, Jiangsu 210023 (CN); FU, Zheng, Nanjing, Jiangsu 210023 (CN); LI, Jing, Nanjing, Jiangsu 210023 (CN); ZHANG, Xiang, Nanjing, Jiangsu 210023 (CN); ZHOU, Xinyan, Nanjing, Jiangsu 210023 (CN); ZHANG, Li, Nanjing, Jiangsu 210023 (CN); YU, Mengchao, Nanjing, Jiangsu 210023 (CN); GUO, Hongyuan, Nanjing, Jiangsu 210023 (CN)
(74) Representative: Cabinet Laurent & Charras
(86) International application number: PCT/CN2022/083876
(87) International publication number: WO 2022/206788

(57) **Abstract**

An isolated nucleic acid, a plasmid or viral vector thereof and a pharmaceutical composition thereof, and a method for treating a disease. The nucleic acid and the vector comprise at least one RNA fragment capable of inhibiting gene expression and/or a targeting tag having a targeting function, can be delivered into a host body, are enriched in an organ tissue of a host, and are self-assembled to form and secrete an exosome and target a target tissue, thereby treating the disease.

## Description

This application claims the priority of Chinese Patent Application No. 202110335617.9, filed with the China National Intellectual Property Administration on March 29, 2021, and titled with "RNA PLASMID DELIVERY SYSTEM AND USE THEREOF", Chinese Patent Application No. 202110336982.1, filed with the China National Intellectual Property Administration on March 29, 2021, and titled with "VIRAL VECTOR-BASED RNA DELIVERY SYSTEM AND USE THEREOF", and Chinese Patent Application No. 202110336983.6, filed with the China National Intellectual Property Administration on March 29, 2021, and titled with "GENE CIRCUIT, RNA DELIVERY SYSTEM AND USE THEREOF", which are hereby incorporated by reference in entirety.

### FIELD

The present invention relates to the fields of molecular biology and medicine. Specifically, the present invention relates to a nucleic acid molecule that can treat diseases, a delivery system and use thereof in disease treatment.

### BACKGROUND

RNA interference (RNAi) therapy has been considered a promising strategy to treat human diseases since its invention. However, it has faced numerous issues during clinical practice, and the progress of the therapy's development has fallen short of expectations.

It is generally believed that RNA cannot remain stable outside the cell for a long time, because RNA will be degraded into fragments due to the abundance of RNase in the extracellular environment. It is therefore crucial to find a method that can stabilize RNA outside the cell and enable it to enter targeted tissues, thereby enhancing the effect of RNAi therapy.

There are currently many studies concerning siRNA, mainly focusing on the following aspects: 1. Designing siRNA with therapeutic effects. 2. Chemically modifying siRNA in order to enhance its stability *in vivo* and increase the yield. 3. Designing various artificial carriers, including lipid nanoparticles, cationic polymers and plasmids, to improve the efficiency of siRNA delivery *in vivo.* Among them, there are a number of patents regarding the third aspect. The primary cause for this is that researchers have realized the absence of suitable siRNA delivery systems for safely, accurately and efficiently deliver siRNA to target tissues. This challenge has become the fundamental limitation of RNAi therapy.

Chinese patent CN108624590A discloses an siRNA capable of inhibiting the expression of DDR2 gene. Chinese patent CN108624591A discloses an siRNA capable of silencing ARPC4 gene, and the siRNA is modified with α-phosphorus-selenium. Chinese patent CN108546702A discloses an siRNA targeting a long non-coding RNA, DDX11-AS1. Chinese patent CN106177990A discloses an siRNA precursor that can be used for the treatment of various tumors. These patents devise specific siRNAs and target certain diseases caused by genetic mutations.

Chinese patent CN108250267A discloses a polypeptide and a polypeptide-siRNA induced co-assembly, where the polypeptide acts as a carrier of siRNA. Chinese patent CN108117585A discloses a polypeptide to target and to introduce siRNA for promoting apoptosis of breast cancer cells, also utilizing the polypeptide as a carrier of siRNA. Chinese patent CN108096583A discloses a nanoparticle carrier, which can be loaded with siRNA that has curative effect on breast cancer while containing chemotherapeutic drugs. These patents are all inventions on siRNA carriers, whose technical solutions share the common feature of pre-assembling the carrier and siRNA *in vitro* before introducing them to the host. In fact, this is characteristic for most of the currently designed delivery techniques. However, these delivery systems pose a common issue that such artificially synthesized exogenous delivery system is prone to be cleared by the host's circulatory system, cause immunogenic reactions, and even potentially be toxic to certain cell types and tissues.

The research team of the present invention found that endogenous cells can encapsulate miRNAs into exosomes. Exosomes can deliver miRNA to receptor cells, and the secreted miRNA can effectively block the expression of target genes at a relatively low concentration. Exosomes are biocompatible with the host immune system and possess the inherent ability to protect and transport miRNA across biological barriers in vivo, thereby having the potential to overcome problems associated with siRNA delivery. For example, Chinese patent CN1106993 82A discloses a method for preparing exosomes delivering siRNA, which involves techniques of isolating exosomes from plasma and encapsulating siRNA into exosomes by electroporation.

However, such techniques for isolating or preparing exosomes in vitro often necessitate obtaining a large amount of exosomes through cell culture, together with the step of siRNA encapsulation, which makes the clinical cost of large-scale application of the product too high for patients to afford. More importantly, the intricate production/purification process of exosomes makes it almost impossible to comply with GMP standards.

So far, medicinal products containing exosomes as active ingredients have not received approval from CFDA. The main challenge lies in ensuring the consistency of exosome products, which results in these products failing to obtain drug production licenses. If this issue can be resolved, it would significantly advance RNAi therapy.

Therefore, the development of a safe, precise and efficient siRNA delivery system is a crucial part to improve the efficacy of RNAi therapy and advance it further.

### SUMMARY

The present invention provides an effective, safe and convenient method and medicament for assembling RNA that inhibits gene expression in an organ or tissue to form a complex structure and delivering it to a target tissue or its cells to treat diseases as needed.

Specifically, in one aspect of the present invention, an isolated nucleic acid is provided, which comprises a nucleotide sequence encoding an RNA capable of inhibiting gene expression, comprising
(a) a nucleotide sequence encoding one or more RNAs that inhibit gene expression, and the RNA is selected from the group consisting of miRNA, shRNA, siRNA, mRNA, ncRNA, sgRNA, and a combination thereof.

In one embodiment of the present invention, the above-mentioned isolated nucleic acid further comprises: (b) a nucleotide sequence encoding a targeting protein. In one embodiment of the present invention, the targeting protein is a tissue-specific protein.

In one embodiment of the present invention, (a) is a nucleotide sequence encoding an RNA that inhibits gene expression.

In one embodiment of the present invention, (a) is a nucleotide sequence encoding a plurality of RNAs that inhibit gene expression. For example, the plurality of RNAs that inhibit gene expression are 2-4 RNAs that inhibit gene expression

As used herein, "isolated" means that a substance has been separated from its original environment (in the case of a natural substance, the original environment is the natural environment). For example, polynucleotides and polypeptides in their natural state within living cells are not isolated and purified. However, if the same polynucleotide or polypeptide is separated from other substances that exist in its natural state, it is isolated and purified..

In one embodiment of the present invention, the RNA that inhibits gene expression is RNA that inhibits the expression of the following genes: EGFR gene, KRAS gene, VEGFR gene, mTOR gene, TNF-α gene, integrin-α gene, B7 gene, TGF-β1 gene, H2-K gene, H2-D gene, H2-L gene, HLA gene, GDF15 gene, miRNA-21, miRNA-214, TNC gene, PTP1B gene, mHTT gene, Lrrk2 gene, and/or α-synuclein gene.

In one embodiment of the present invention, (a) is siRNA. siRNA, also known as short interfering RNA or silencing RNA, is a type of double-stranded RNA molecule, generally 20-29 base pairs in length, with its double strands extending 2 nucleotides beyond the other end at both ends of the RNA. siRNA is generally produced by simulating the production mechanism of miRNA. Such siRNA can be processed from precursor RNA (Pre-RNA). Precursor RNA can be folded into a stable stem-loop (hairpin) structure, and the length of the stem-loop structure is generally between 50-100 bp. The stem of a stem-loop structure contains two substantially complementary sequences on either side.

The siRNA can be substantially complementary to at least a portion of the sequence of the mRNA encoding the gene. "Substantially complementary" means that the sequences of the nucleotides are sufficiently complementary to interact in a predictable manner, such as to form secondary structure. Generally, at least 70% of the nucleotides in two "substantially complementary" nucleotide sequences are complementary to each other; preferably, at least 80% of the nucleotides are complementary; more preferably, at least 90% of the nucleotides are complementary; further preferably, at least 95% of the nucleotides are complementary; such as 98%, 99% or 100%. Functionally, siRNA interferes with post-transcriptional degradation of the mRNA expressed by a specific gene with a complementary nucleotide sequence, thereby preventing translation.

In one embodiment of the present invention, (a) is selected from the group consisting of siRNA of EGFR gene, siRNA of KRAS gene, siRNA of VEGFR gene, siRNA of mTOR gene, siRNA of TNF-α gene, siRNA of integrin-α gene, siRNA of B7 gene, siRNA of TGF-β1 gene, siRNA of H2-K gene, siRNA of H2-D gene, siRNA of H2-L gene, siRNA of HLA gene, siRNA of GDF15 gene, an antisense strand of miRNA-21, an antisense strand of miRNA-214, siRNA of TNC gene, siRNA of PTP1B gene, siRNA of mHTT gene, siRNA of Lrrk2 gene, and siRNA of α-synuclein gene.

The siRNAs of the above-mentioned genes are RNA sequences that have the function of inhibiting the expression of the gene. Any RNA sequence that has the function of inhibiting the expression of the above-mentioned genes can be used in the present invention. The following are some RNA sequences with better effects:
The siRNA of the EGFR gene includes UGUUGCUUCUCUUAAUUCCU, AAAUGAUCUUCAAAAGUGCCC, UCUUUAAGAAGGAAAGAUCAU, AAUAUUCGUAGCAUUUAUGGA, UAAAAAUCCUCACAUAUACUU.
The siRNA of the KRAS gene includes UGALTCTCTAGUALTCTALTCTCTAUGGC, AAUUUGUUCUCUAUAAUGGUG, UAAUUUGUUCUCUAUAAUGGU, UUAUGUUUUCGAAUUUCUCGA, and UGUAUUUACAUAAUUACACAC.
The siRNA of the VEGFR gene includes AULTCTGAAGAGUUGUALTCTAGCC, UAAUAGACUGGUAACUUUCAU, ACAACUAUGUACAUAAUAGAC, UUUAAGACAAGCUUUUCUCCA, and AACAAAAGGUUUUUCAUGGAC.
The siRNA of the mTOR gene includes AGAUAGUUGGCAAAUCUGCCA, ACUAUUUCAUCCAUAUAAGGU, AAAAUGUUGUCAAAGAAGGGU, AAAAAUGUUGUCAAAGAAGGG, and UGAUUUCUUCCAUUUCUUCUC.
The siRNA of the TNF-α gene includes AAAACAUAAUCAAAAGAAGGC, UAAAAAACAUAAUCAAAAGAA, AAUAAUAAAUAAUCACAAGUG, UUUUCACGGAAAACAUGUCUG, and AAACAUAAUCAAAAGAAGGCA.
The siRNA of the integrin-α gene includes AUAAUCAUCUCCAUUAAUGUC, AAACAAUUCCUUUUUUAUCUU, AUUAAAACAGGAAACUUUGAG, AUAAUGAAGGAUAUACAACAG, and UUCUUUAUUCAUAAAAGUCUC.
The siRNA of the B7 gene includes LTCTCTCTCLTCTUGGGUAAUCLTCTCAG, AGAAAAAUUCCACUUUUUCUU, AUUUCAAAGUCAGAUAUACUA, ACAAAAAUUCCAUUUACUGAG, and AUUAUUGAGUUAAGUAUUCCU.
The siRNA of the TGF-β1 gene includes ACGGAAAUAACCUAGAUGGGC, UGAACUUGUCAUAGAUUUCGU, UUGAAGAACAUAUAUAUGCUG, UCUAACUACAGUAGUGUUCCC, and UCUCAGACUCUGGGGCCUCAG.
The siRNA of the H2-K gene includes AAAAACAAAUCAAUCAAACAA, UCAAAAAAACAAAUCAAUCAA, UAUGAGAAGACAUUGUCUGUC, AACAAUCAAGGUUACAUUCAA, and ACAAAACCUCUAAGCAUUCUC.
The siRNA of the H2-D gene includes AAUCUCGGAGAGACALTCTCTCAG, AAUGUUGUGUAAAGAGAACUG, AACAUCAGACAAUGUUGUGUA, UGUUAACAAUCAAGGUCACUU, and AACAAAAAAACCUCUAAGCAU.
The siRNA of the H2-L gene includes GAUCCGCUCCCAAUACUCCGG, AUCUGCGUGAUCCGCUCCCAA, UCGGAGAGACAUUUCAGAGCU, UCUCGGAGAGACAUUUCAGAG, and AAUCUCGGAGAGACAUUUCAG
The siRNA of the HLA gene includes AUCUGGAUGGUGUGAGAACCG, UGUCACUGCUUGCAGCCUGAG, UCACAAAGGGAAGGGCAGGAA, UUGCAGAAACAAAGUCAGGGU, and ACACGAACACAGACACAUGCA.
The siRNA of the GDF15 gene includes UAUAAAUACAGCUGLTCTCTGGGC, AGACUUAUAUAAAUACAGCUG, AAUUAAUAAUAAAUAACAGAC, AUCUGAGAGCCAUUCACCGUC, and UGCAACUCCAGCUGGGGCCGU.
The siRNA of the TNC gene includes AUGAAAUGUAAAAAAAGGGA, AAUCAUAUCCUUAAAAUGGAA, UAAUCAUAUCCUUAAAAUGGA, UGAAAAAUCCUUAGUUUUCAU, and AGAAGUAAAAAACUAUUGCGA.
The siRNA of the PTP1B gene includes UGAUAUAGUCALTCTAUCLTCTCLTCT, UCCAUUUUUAUCAAACUAGCG, AUUGUUUAAAUAAAUAUGGAG, AAU UUU AAU AC AUU AUUGGU U, and UUUAUUAUUGUACUUUUUGAU.
The siRNA of the mHTT gene includes UAUGLTCTCTCTCACAUALTCTGUCAG, AUUUAGUAGCCAACUAUAGAA, AUGUUUUUCAAUAAAUGUGCC, UAUGAAUAGCAUUCUUAUCUG, and UAUUUGUUCCUCUUAAUACAA.
The siRNA of the Lrrk2 gene includes ALTCTAACAUGAAAAUAUCACLTCT, UUAACAAUAUCAUAUAAUCUU, AUCUUUAAAAUUUGUUAACGC, UUGAUUUAAGAAAAUAGUCUC, and UUUGAUAACAGUAUUUUUCUG.
The siRNA of the α-synuclein gene includes AUAUALTCTAACAAALTCTUCACAA, AAGUAUUAUAUAUAUUAACAA, AUAACUUUAUAUUUUUGUCCU, UAACUAAAAAAUUAUUUCGAG, and UCGAAUAUUAUUUAUUGUCAG

Those skilled in the art can understand that the RNA sequences that can be used in the present invention also include RNA sequences that have a homology of more than 80% with the aforementioned RNA. For example, the homology is 85%, 88%, 90%, 95%, 98%, etc.

In one embodiment of the present invention, the nucleotide sequence encoding one or more RNAs that inhibit gene expression in the isolated nucleic acid comprises an RNA fragment sequence targeting the gene. The RNA fragment sequence is usually an RNA sequence complementary to the target nucleotide sequence of the gene. In the case where the RNA is siRNA, the RNA fragment sequence is the sense strand sequence of the siRNA.

In one embodiment of the present invention, the one or more RNAs that inhibit gene expression in the isolated nucleic acid has 15-29 nucleotides (nt) in length, preferably 18-22 nt, such as 18 nt, 19 nt, 20 nt, 21 nt, or 22 nt. A large number of experiments have proved that in the case where the length of the RNA sequence is less than 18 nt, especially less than 15 nt, the RNA sequence is mostly invalid and will not play a role. In the case where the length of the RNA sequence is greater than 22 nt, especially greater than 25 nt, not only does the cost of the circuit increase greatly, but the effect is no better than that of an RNA sequence with a length of 18-22 nt, and the economic benefits are poor. Therefore, in the case where the length of the RNA sequence is 15-25 nt, especially 18-22 nt, the cost and function can be balanced with the best effect.

In the present invention, the isolated nucleic acid also includes variants and derivatives thereof. Those skilled in the art can use common methods to modify the nucleic acid. Such modification includes (but not limited to) methylation modification, hydrocarbon modification, glycosylation modification (such as 2-methoxy-glycosyl modification, hydroxyl-glycosyl modification, saccharide ring modification), nucleic acid modification, peptide fragment modification, lipid modification, halogen modification, and nucleic acid modification (such as "TT" modification). In one embodiment of the present invention, the modification is an internucleotide linkage, for example selected from the group consisting of phosphorothioate, 2'-O methoxyethyl (MOE), 2'-fluoro, alkyl phosphonate, phosphorodithioate, alkyl phosphonothioate, phosphoramidate, carbamate, carbonate, phosphotriester, acetamidate, carboxymethyl ester, and combinations thereof. In one embodiment of the present invention, the modification is a modification of nucleotides, for example, selected from the group consisting of peptide nucleic acid (PNA), locked nucleic acid (LNA), arabinose nucleic acid (FANA), analogs and derivatives thereof, and combinations thereof. Preferably, the modification is 2' fluoropyrimidine modification. 2' Fluoropyrimidine modification is to replace the 2'-OH of the pyrimidine nucleotide of RNA with 2'-F. 2'-F modification can make RNA difficult to be recognized by RNase in vivo, thereby increasing the stability of RNA fragment during transportation in vivo.

In one embodiment of the present invention, the nucleotide sequence encoding one or more RNAs that inhibit gene expression in the isolated nucleic acid also comprises one or more of a flanking sequence (such as a 5' flanking sequence and a 3' flanking sequence), a stem-loop sequence and a compensation sequence of the RNA sequence.

The compensation sequence is the reverse complementary sequence of the RNA fragment sequence. In one embodiment of the present invention, the compensation sequence is the reverse complementary sequence of the RNA fragment sequence with any 1-5 bases deleted. In another embodiment of the present invention, the compensation sequence is the reverse complementary sequence of the RNA fragment sequence with any 1-3 bases deleted, especially 1-3 contiguous bases deleted. Most preferably, the compensation sequence is the reverse complementary sequence of the RNA fragment with the base at position 9 and/or 10 deleted. Generally speaking, the RNA sequence can be expressed in the target receptor, and the compensation sequence cannot be expressed in the target receptor.

The flanking sequence is a sequence used to help RNA molecules such as siRNA molecules be sheared into the correct final sequence. In the present invention, the flanking structure of the natural miRNA precursor can be used as the flanking structure of the RNA molecule of the present invention. For example, the flanking structure of pre-miR-155 is used.

In one embodiment of the present invention, the 5' flanking sequence is ggatcctggaggcttgctgaaggctgtatgctgaattc or a sequence with more than 80% homology thereto, for example, 85%, 90%, 92%, 95%, 98%, 99% homology thereto.

In one embodiment of the present invention, the 3' flanking sequence is accggtcaggacacaaggcctgttactagcactcacatggaacaaatggcccagatctggccgcactcgag or a sequence with more than 80% homology thereto, for example, 85%, 90%, 92%, 95%, 98%, 99% homology thereto.

The stem-loop structure is a spacer sequence encoding a hairpin structure that maintains the stability of the RNA. In one embodiment of the present invention, the sequence of the stem-loop structure is preferably gttttggccactgactgac or a sequence with more than 80% homology thereto.

In one embodiment of the present invention, the one or more RNAs that inhibit gene expression in the isolated nucleic acid comprises successively: 5' flanking sequence, RNAfragment sequence, stem-loop sequence, compensation sequence and 3' flanking sequence. In one embodiment of the present invention, the RNA has a promoter at 5' end.

The inventor of the present application unexpectedly discovered and proved through experiments that with the cooperation of the above-mentioned specific flanking sequence, compensation sequence, and stem-loop sequence, the required RNA sequence can be transcribed and sheared to the greatest extent, and encapsulated in exosomes.

In one embodiment of the present invention, (a) in the nucleic acid is a nucleotide sequence encoding a plurality of RNAs that inhibit gene expression. For example, the plurality of RNAs that inhibit gene expression are 2-4 RNAs that inhibit gene expression. In the case where (a) is a nucleotide sequence encoding a plurality of RNAs that inhibit gene expression, the plurality of RNAs are linked via a linker. The linker has a structure of, for example, sequence 1-sequence 2-sequence 3. Wherein, sequence 1 is preferably CAGATC; sequence 2 may be a sequence of 5-80 bases, preferably a sequence of 10-50 bases, more preferably a sequence of 20-40 bases; and sequence 3 is preferably TGGATC. In one embodiment of the present invention, the sequence of the linker is CAGATCTGGCCGCACTCGAGGTAGTGAGTCGACCAGTGGATC.

In one embodiment of the present invention, the targeting protein is a target tissue-specific targeting peptide.

In one embodiment of the present invention, the targeting protein is a fusion protein of a target tissue-specific targeting peptide and a membrane protein.

In one embodiment of the present invention, the specific targeting peptide is selected from the group consisting of RVG targeting peptide, GE11 targeting peptide, PTP targeting peptide, TCP-1 targeting peptide, and MSP targeting peptide.

In one embodiment of the present invention, the targeting protein is a membrane protein, which is, for example, selected from the group consisting of a receptor protein (such as a growth factor receptor), LAMP1 or LAMP2 (such as LAMP2B), and an antibody or an antigen-binding fragment thereof.

In one embodiment of the present invention, the targeting protein is RVG-LAMP2B fusion protein, GE11-LAMP2B fusion protein, PTP-LAMP2B fusion protein, TCP-1-LAMP2B fusion protein, or MSP-LAMP2B fusion protein.

In one embodiment of the present invention, the target or tissue is the brain, pineal gland, pituitary gland, eye, ear, nose, mouth, pharynx, parotid gland, tonsil, esophagus, trachea, thyroid, thymus, breast, lung, heart, stomach, intestines, appendix, liver, gallbladder, spleen, pancreas, kidney, ureter, bladder, urethra, uterus, ovary, fallopian tube, vagina, vas deferens, prostate, penis, testicle, anus, bone, muscle, connective tissue, nerve, lymph, colorectum, blood, bone marrow and/or skin, etc. In one embodiment of the present invention, the target cells are cells of the above-mentioned target or tissue.

In one aspect of the present invention, after the nucleic acid is administered to a mammal, it is enriched in tissues (including liver, lung, gastrointestinal tract, breast, kidney, brain, spleen, lymph, thyroid, reproductive organ, blood cells or lymphocytes, especially liver), and its expressed products are encapsulated in exosomes produced and secreted by cells in these tissues and delivered to target tissues to exert therapeutic effects. Therefore, on the one hand, it is necessary to select available targeting tags according to the target tissue for the targeting protein encoded by the nucleic acid, and on the other hand, it is also necessary to ensure that the targeting tag can stably appear on the surface of exosomes to achieve targeting function.

Targeting peptides suitable for use in the present invention include, but are not limited to, RVG targeting peptide (with the nucleotide sequence as shown in SEQ ID No: 1), GE11 targeting peptide (with the nucleotide sequence as shown in SEQ ID No: 2), PTP targeting peptide (with the nucleotide sequence as shown in SEQ ID No: 3), TCP-1 targeting peptide (with the nucleotide sequence as shown in SEQ ID No: 4), or MSP targeting peptide (with the nucleotide sequence as shown in SEQ ID No: 5); targeting proteins include but are not limited to RVG-LAMP2B fusion protein (with the nucleotide sequence as shown in SEQ ID No: 6), GE11-LAMP2B fusion protein (with the nucleotide sequence as shown in SEQ ID No: 7), PTP-LAMP2B fusion protein (with the nucleotide sequence as shown in SEQ ID No: 8), TCP-1-LAMP2B fusion protein (with the nucleotide sequence as shown in SEQ ID No: 9), or MSP-LAMP2B fusion protein (with the nucleotide sequence as shown in SEQ ID No: 10).

Among them, RVG targeting peptide and RVG-LAMP2B fusion protein can precisely target brain tissue; GE11 targeting peptide and GE11-LAMP2B fusion protein can precisely target organs or tissues with high expression of EGFR, such as lung cancer tissue with EGFR mutation; PTP targeting peptide and PTP-LAMP2B fusion protein can precisely target the pancreas, especially the plectin-1 protein specifically expressed in human and mouse pancreatic cancer tissues; TCP-1 targeting peptide and TCP-1-LAMP2B fusion protein can precisely target the colon; and MSP targeting peptide and MSP-LAMP2B fusion protein can precisely target muscle tissue.

In practical applications, the targeting protein can be combined with various RNAs that inhibit gene expression to inhibit their specific target genes in different tissues. For example, RVG targeting peptide and RVG-LAMP2B fusion protein can be combined with siRNA of EGFR gene, siRNA of TNC gene or a combination of the two to treat glioblastoma, be combined with siRNA of PTP1B gene to treat obesity, be combined with siRNA of mHTT gene to treat Huntington's disease, or be combined with siRNA of LRRK2 gene to treat Parkinson's disease; GE11 targeting peptide and GE11-LAMP2B fusion protein can be combined with siRNA of EGFR gene to treat lung cancer and other diseases induced by high expression or mutation of EGFR gene; TCP-1 targeting peptide or TCP-1-LAMP2B fusion protein can be combined with siRNA of TNF-α gene, siRNA of integrin-α gene, siRNA of B7 gene or any combination of the above three to treat colitis or colon cancer.

In one embodiment of the present invention, (a) in the nucleic acid is a nucleotide sequence encoding a plurality of RNAs that inhibit gene expression. The plurality of RNAs that inhibit gene expression can be administered to the subject in need of treatment simultaneously or separately. In one embodiment of the present invention, the plurality of RNAs can be located in different plasmid vectors or viral vectors. For example, one of the plasmids or viral vectors contains a promoter and targeting tag, and the other plasmid contains a promoter and an RNA fragment. That is, the targeting tag and the RNA fragment are loaded into different vectors, and two or more vectors are injected into the body simultaneously or separately. More preferably, in the case where the two or more different vectors are injected into the host, the vector containing the RNA sequence can be injected first, and then (such as after 1-2 hours) the vector containing the targeting tag is injected, so that a better targeting effect can be achieved.

In one embodiment of the present invention, the nucleic acid is enriched in the liver of a mammalian and its product is encapsulated in exosomes in hepatocytes.

In one aspect of the present invention, a vector of RNA that inhibits gene expression is provided, which comprises:
(a) a nucleotide sequence encoding one or more RNAs that inhibit gene expression, and the RNA is selected from the group consisting of miRNA, shRNA, siRNA, mRNA, ncRNA, sgRNA, and a combination thereof; and
optionally, (b) a nucleotide sequence encoding a targeting protein.

In one embodiment of the present invention, the vector comprises the aforementioned isolated nucleic acid provided by the present invention, which encodes a nucleotide sequence of RNA capable of inhibiting gene expression.

In one embodiment of the present invention, the vector is a plasmid. In one embodiment of the present invention, after the plasmid is administered to a mammal, it can be expressed in tissues (including liver, lung, gastrointestinal tract, breast, kidney, brain, spleen, lymph, thyroid, reproductive organ, blood cells or lymphocytes, especially liver), and the RNA fragment of the present invention is transcribed and/or expressed, and encapsulated in exosomes in the cells of the tissue.

In one embodiment of the present invention, the vector is a viral vector. For example, it may be a baculovirus expression vector, an adenoviral vector, a retroviral vector, a herpes viral vector or a lentiviral vector.

In one embodiment of the present invention, the vector is an adenoviral vector. Preferably, the adenovirus is adenovirus-associated virus type 5, adenovirus-associated virus type 8 or adenovirus-associated virus type 9. More preferably, the adenovirus is adenovirus-associated virus type 5.

In one embodiment of the present invention, the plasmid or viral vector is enriched and expressed in the liver after administration to mammals, and its products are encapsulated in exosomes in large quantities.

In one aspect of the present invention, an exosome with RNA that inhibits gene expression is provided, which comprises the nucleic acid or vector as described above. In one embodiment of the present invention, the exosome is an exosome derived from human tissues or cells. The tissue includes liver, lung, gastrointestinal tract, breast, kidney, brain, spleen, lymph, thyroid, reproductive organ, blood cells or lymphocytes. In one embodiment of the present invention, the exosomes is an exosome derived from liver or liver cells.

In one aspect of the present invention, a pharmaceutical composition is provided, which comprises the nucleic acid, vector or exosome as described above. The pharmaceutical composition further comprises a pharmaceutically acceptable carrier or excipient for delivering the nucleic acid, vector or exosome to a subject.

The administration methods of the pharmaceutical composition include oral administration, inhalation, subcutaneous injection, intramuscular injection, and intravenous injection. That is, the pharmaceutical composition can be administered orally, inhaled, subcutaneously, intramuscularly or intravenously. The dosage form of the pharmaceutical composition may be tablets, capsules, powders, granules, pills, suppositories, ointments, solutions, suspensions, lotions, gels, pastes, etc. After the plasmid or viral vector in the pharmaceutical composition is administered to a mammal, it will be enriched in tissues (including liver, lung, gastrointestinal tract, breast, kidney, brain, spleen, lymph, thyroid, reproductive organ, blood cells or lymphocytes, especially liver), and its expressed products are encapsulated in exosomes in large quantities in the cells of the tissue and delivered to the target tissue to exert a therapeutic effect.

The pharmaceutical composition can be used to treat various diseases, including tumors, acute and chronic infectious diseases, or other acute and chronic diseases. The acute and chronic infectious diseases mentioned therein include viral influenza, viral hepatitis, AIDS, SARS viral diseases, bacterial diseases (such as tuberculosis, bacterial pneumonia), and other acute and chronic infectious diseases caused by various pathogenic microorganisms. The other acute and chronic diseases include respiratory diseases, immune system diseases, blood and hematopoietic system diseases, circulatory system diseases such as cardiovascular and cerebrovascular diseases, endocrine system metabolic diseases, digestive system diseases, nervous system diseases, urinary system diseases, reproductive system diseases and motor system diseases. For example, the disease is cancer, pulmonary fibrosis, colitis, obesity, cardiovascular disease caused by obesity, type 2 diabetes, Huntington's disease, Parkinson's disease, myasthenia gravis, Alzheimer's disease or graft-versus-host disease.

In one aspect of the present invention, a method of treating a disease is provided, comprising administering to a subject a nucleic acid, vector or exosome as described above. The diseases include tumors, acute and chronic infectious diseases, or other acute and chronic diseases.

It will be understood by those skilled in the art that the actual dosage administered will vary depending on various factors such as the vector, target cells or tissues, the general condition of the subject to be treated, the degree of transformation/modification sought, the administration route, mode of administration, type of transformation/modification sought, etc.

### BRIEF DESCRIPTION OF DRAWINGS

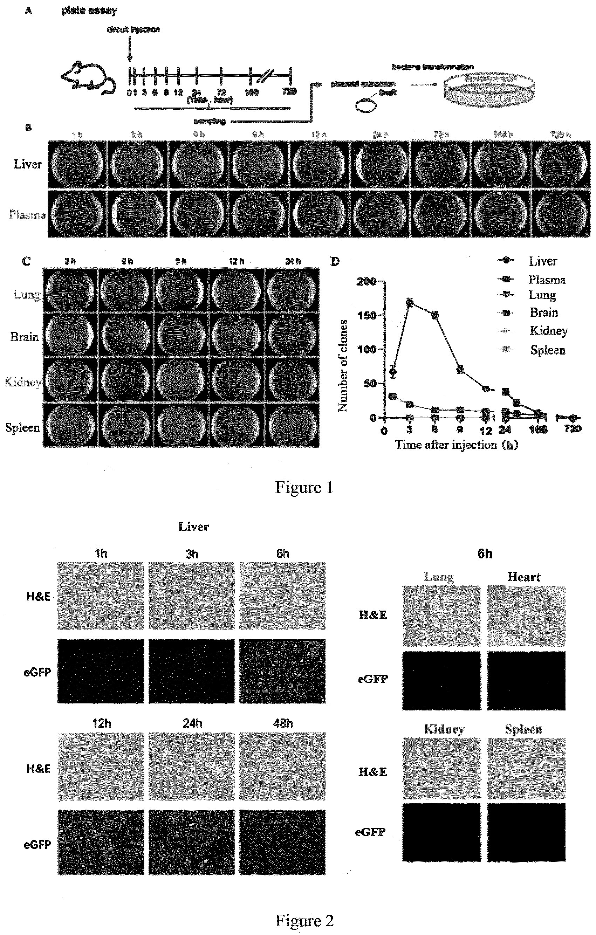

Figure 1 shows the comparison of plasmid distribution and metabolism in mice provided by an embodiment of the present application.

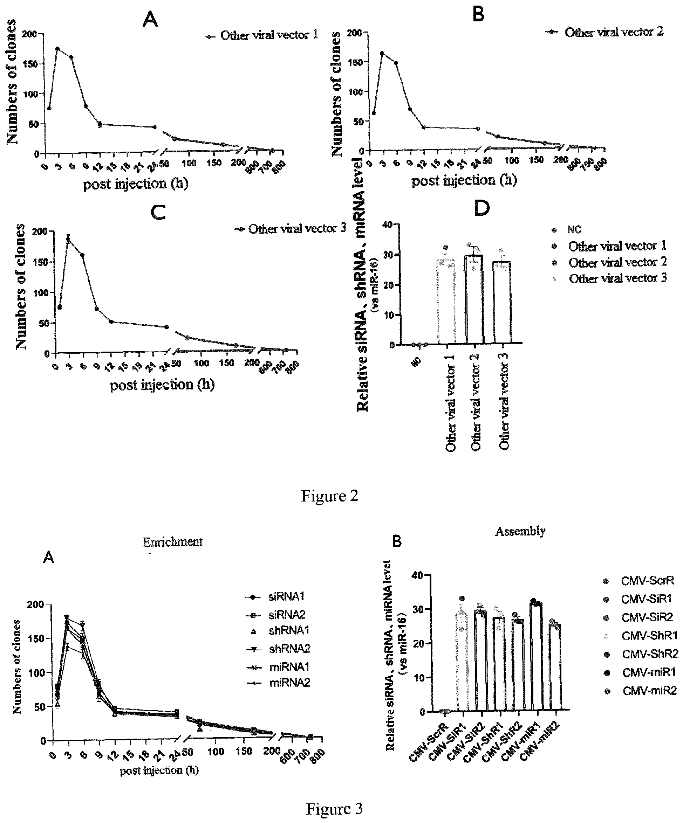

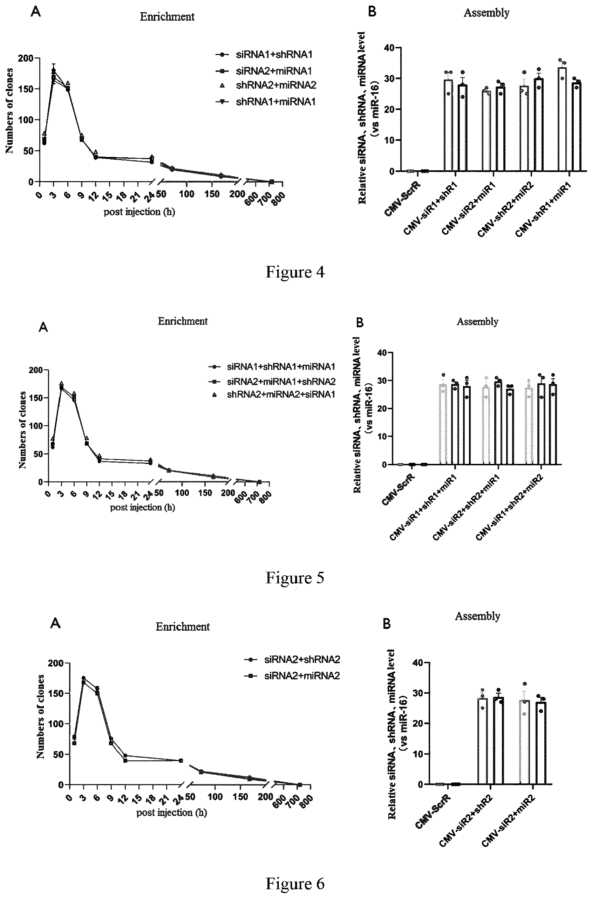

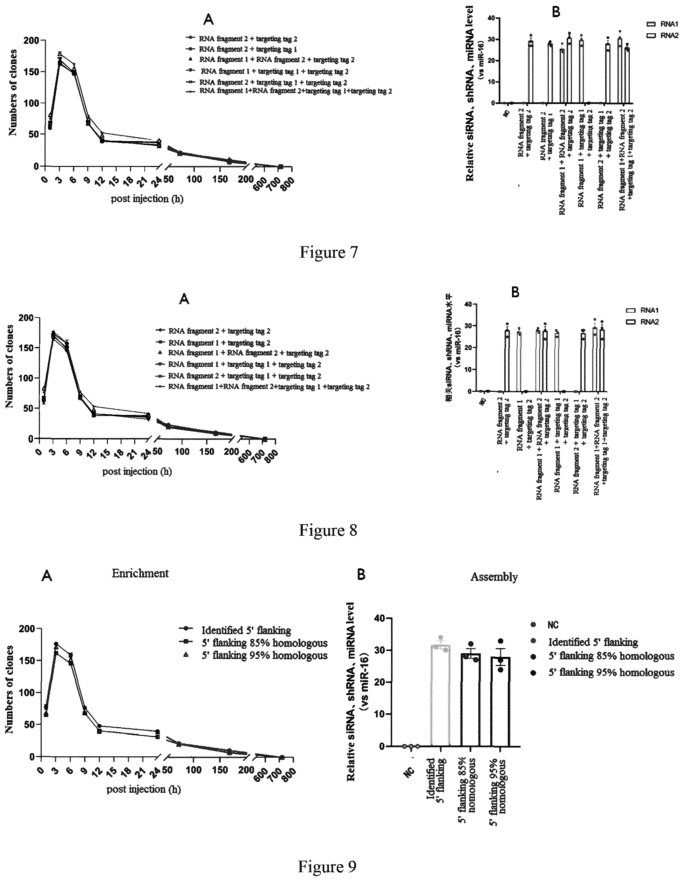

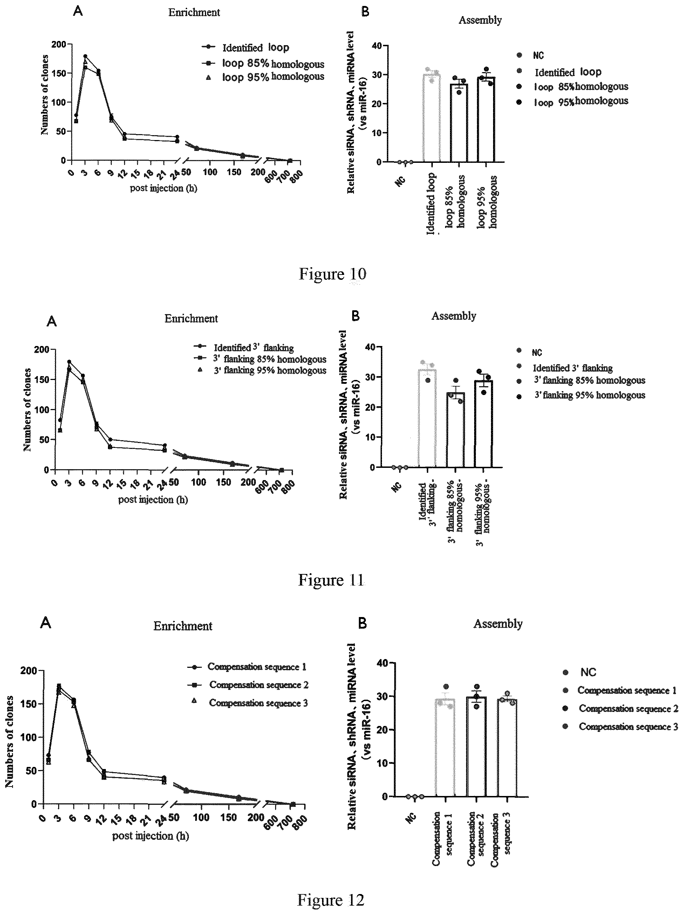

Figure 2 shows the comparison of protein expression levels in mice provided by an embodiment of the present application.

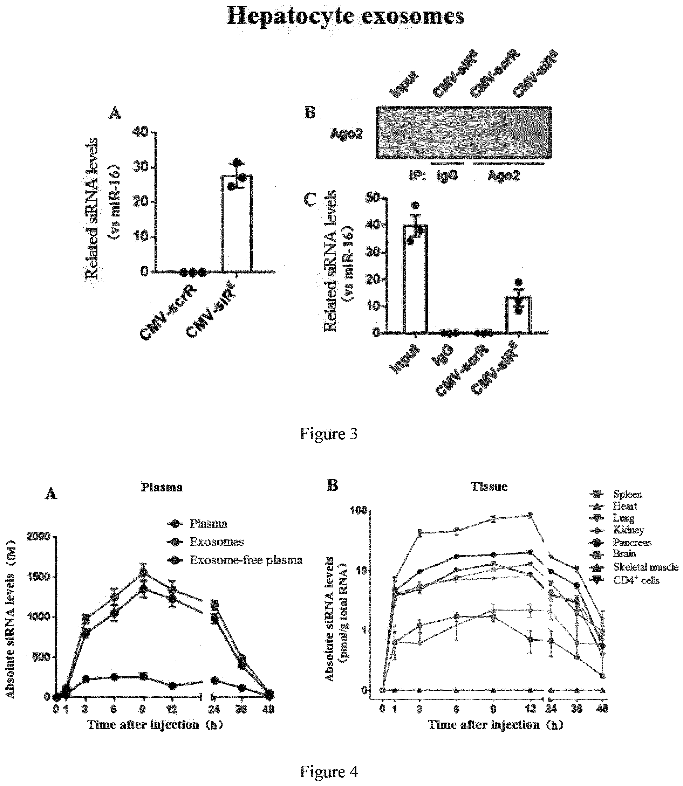

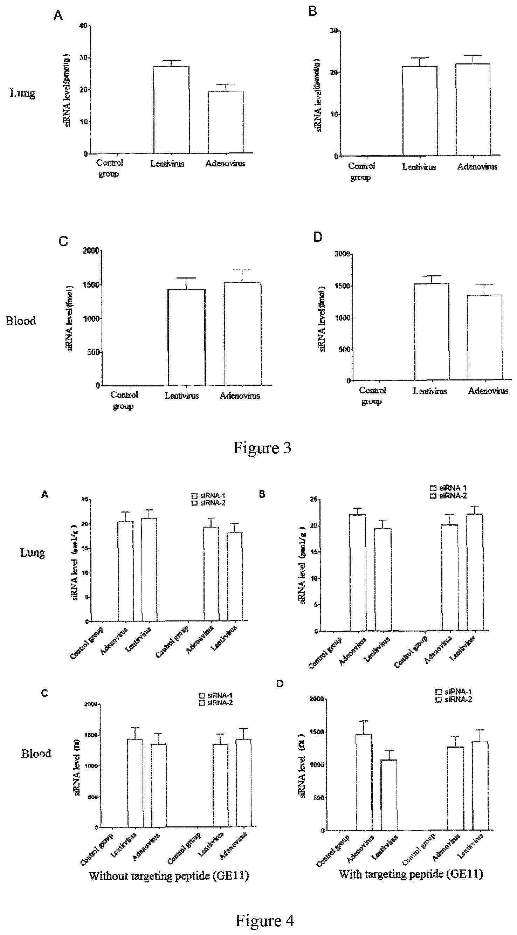

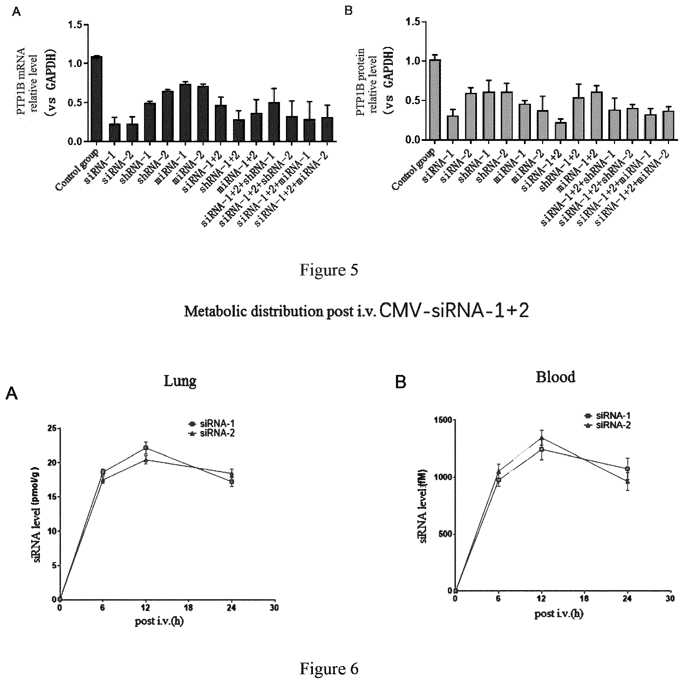

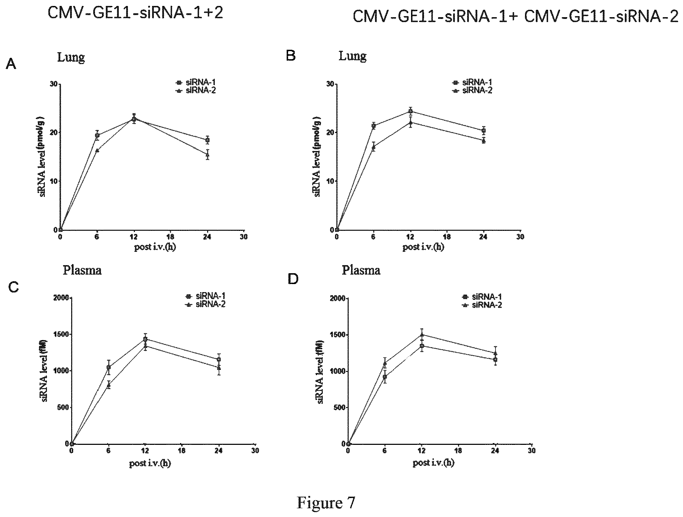

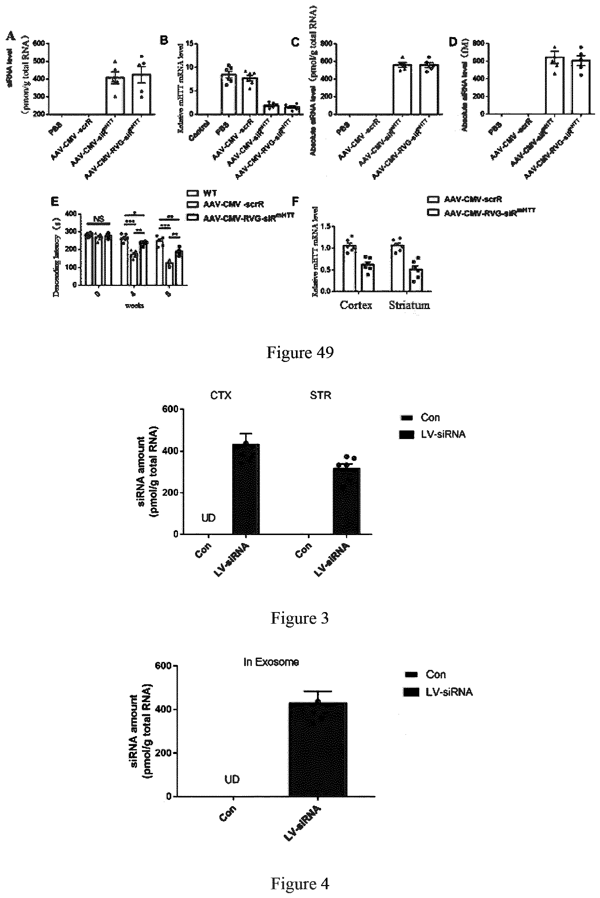

Figure 3 shows the comparison of related siRNA levels in mice provided by an embodiment of the present application.

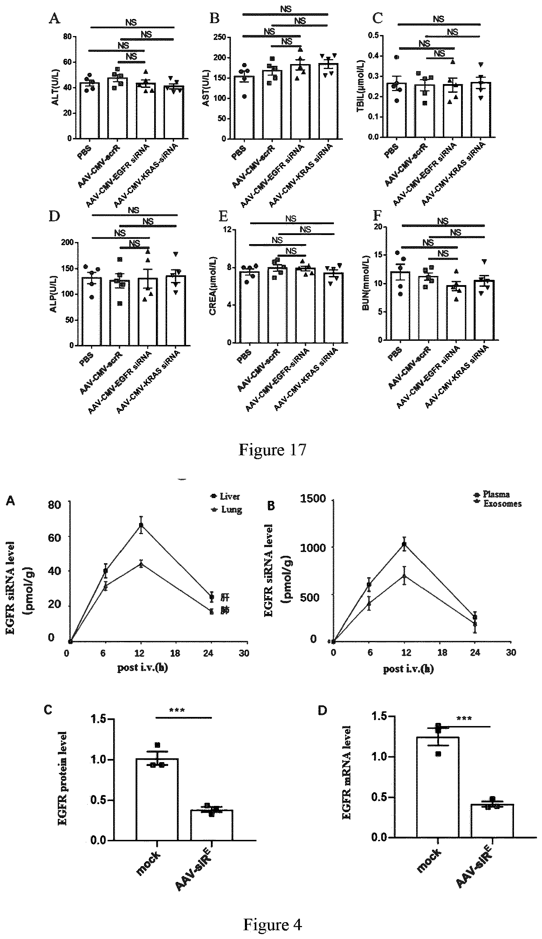

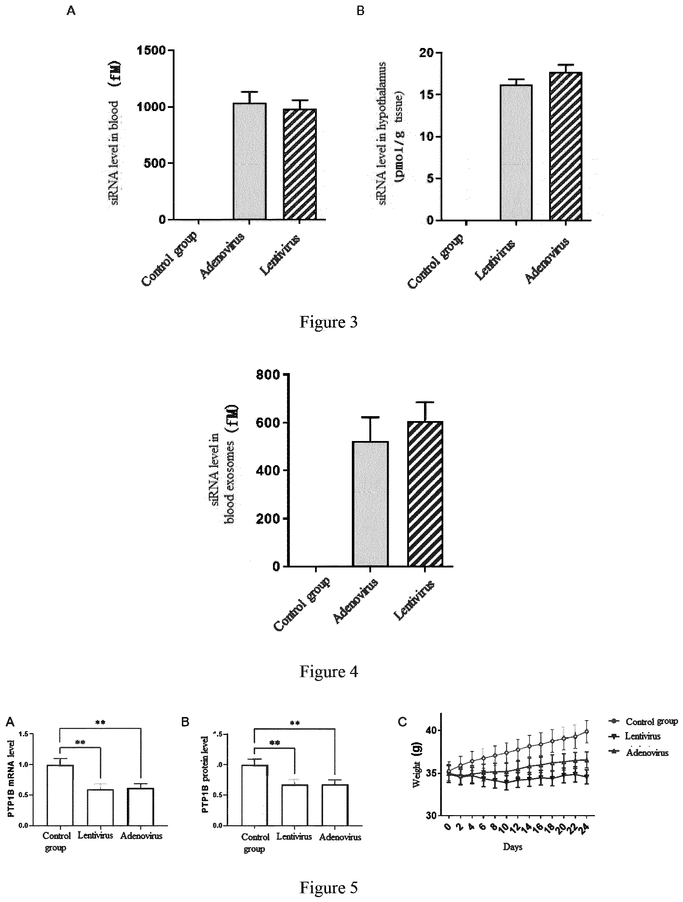

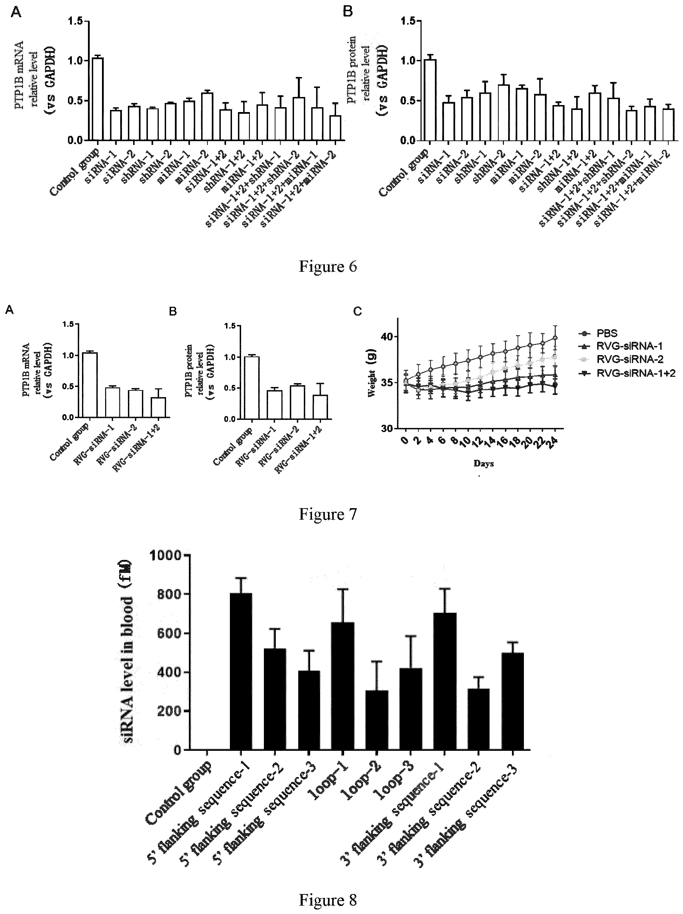

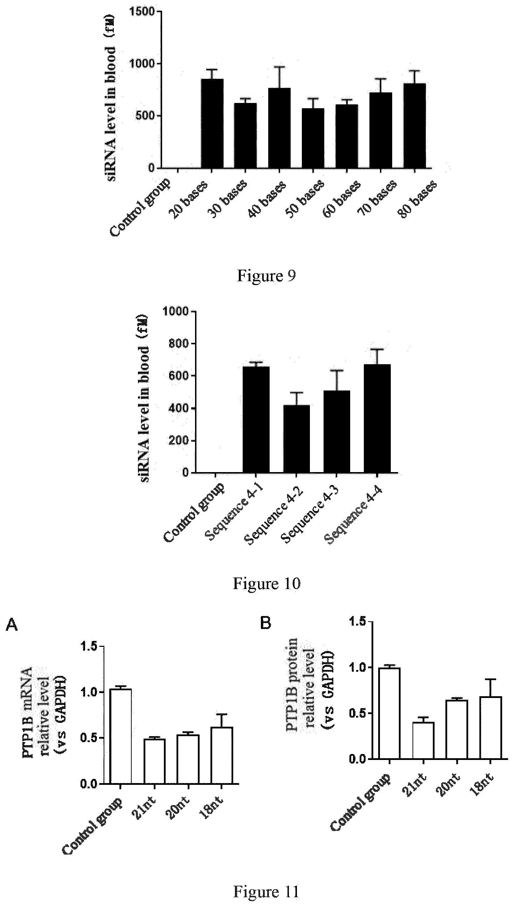

Figure 4 shows the comparison of absolute siRNA levels in various tissues of mice provided by an embodiment of the present application.

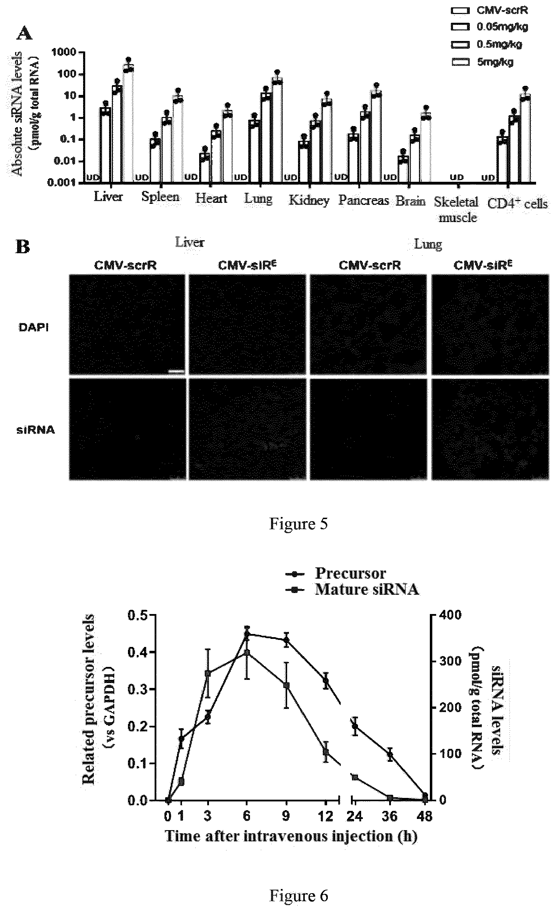

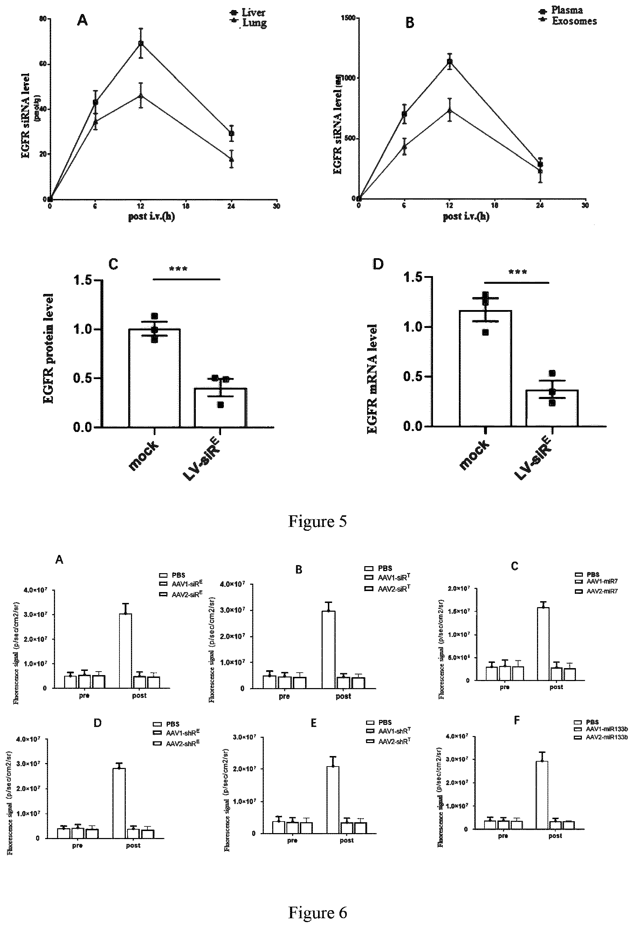

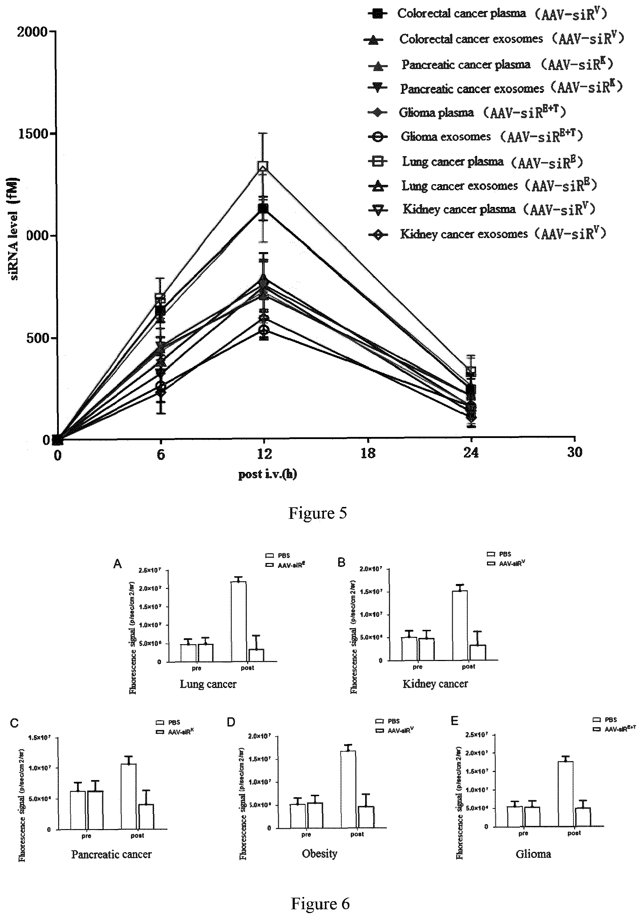

Figure 5 shows the comparison of the impact of plasmid doses on mouse siRNA levels provided by an embodiment of the present application.
Figure 6 shows the comparison of the metabolism of precursors and mature bodies in the mouse liver after plasmid injection provided by an embodiment of the present application.

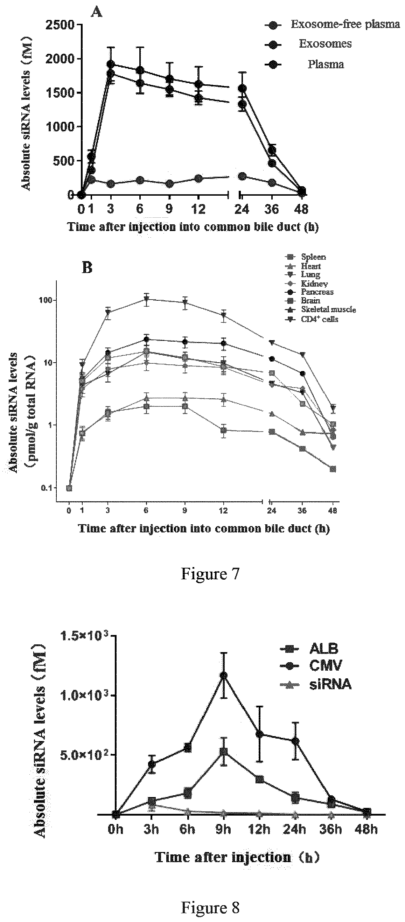

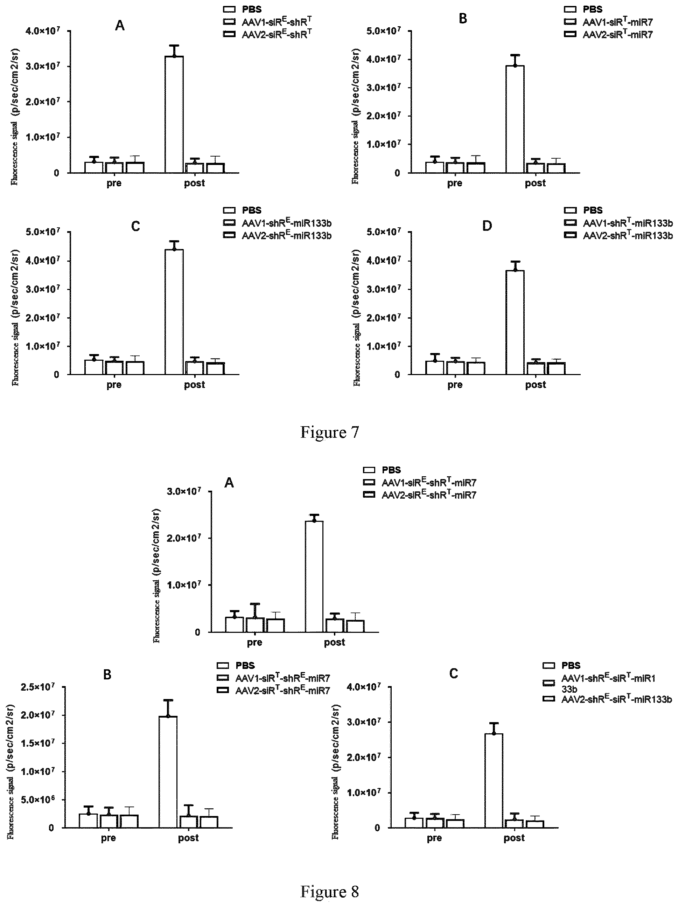

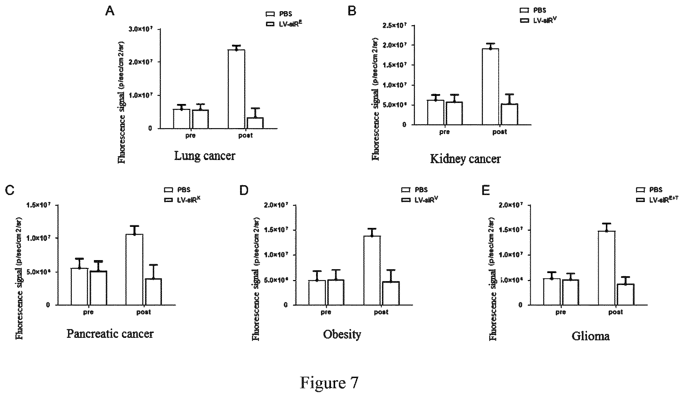

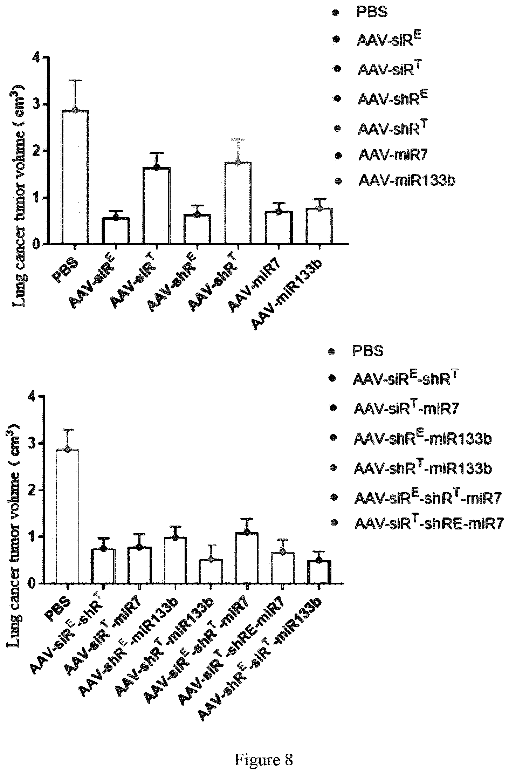

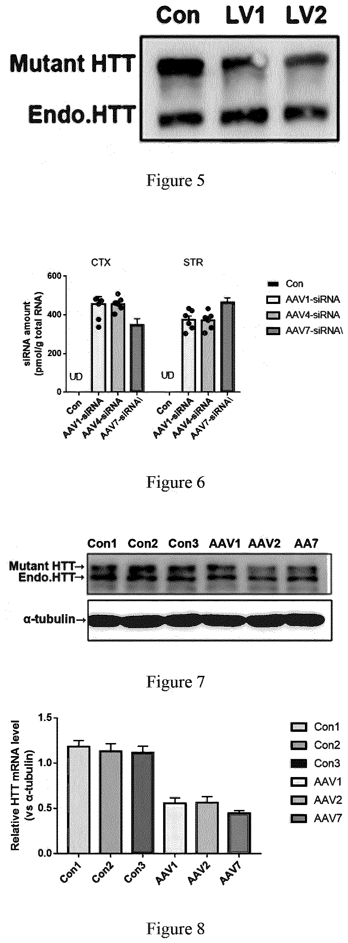

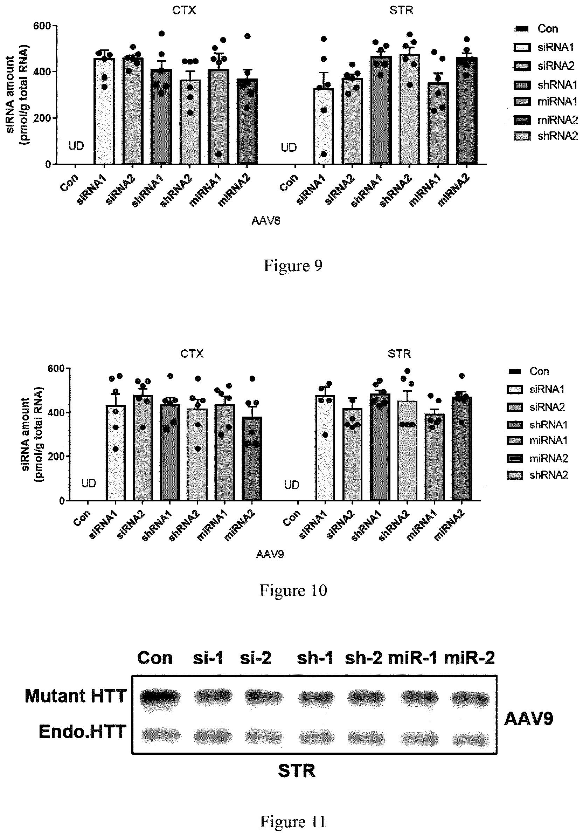

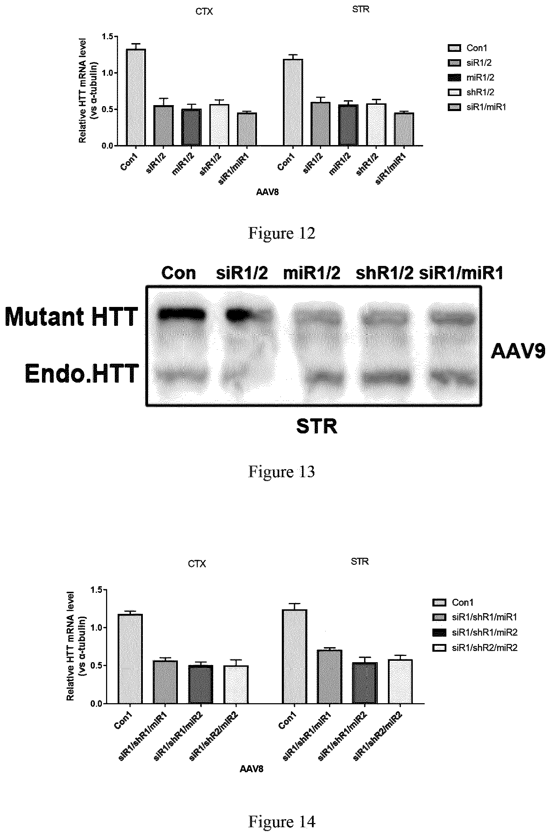

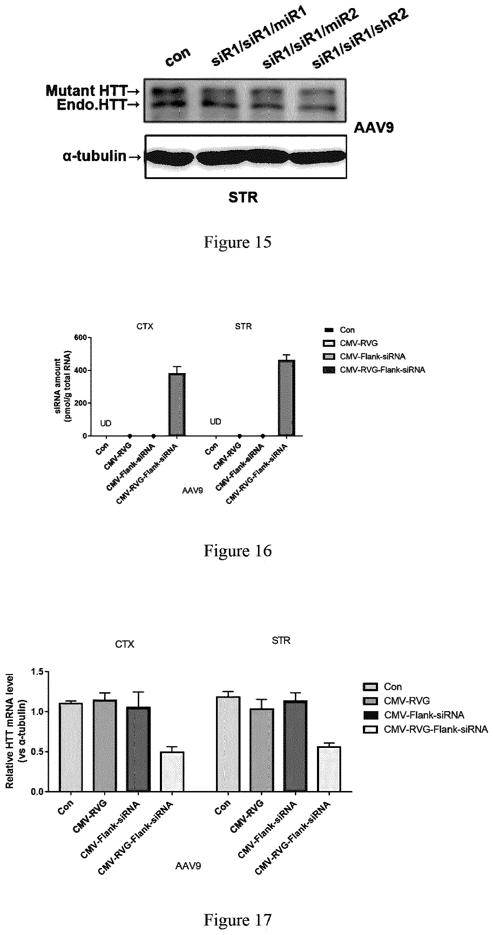

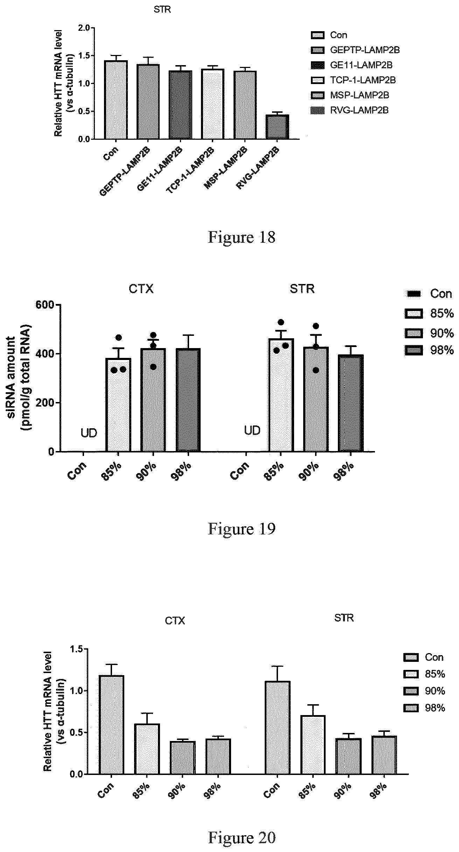

Figure 7 shows the comparison of siRNA kinetics and distribution in different tissues of mice provided by an embodiment of the present application.

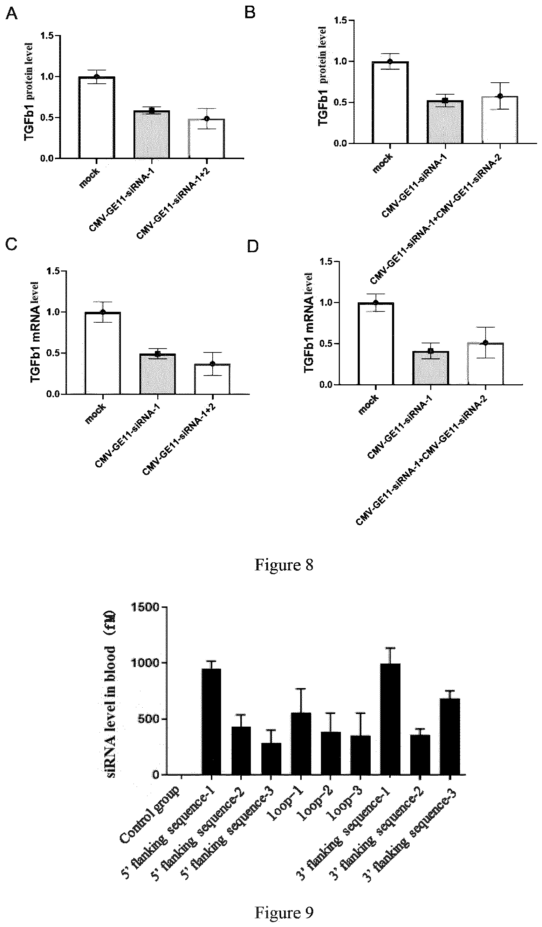

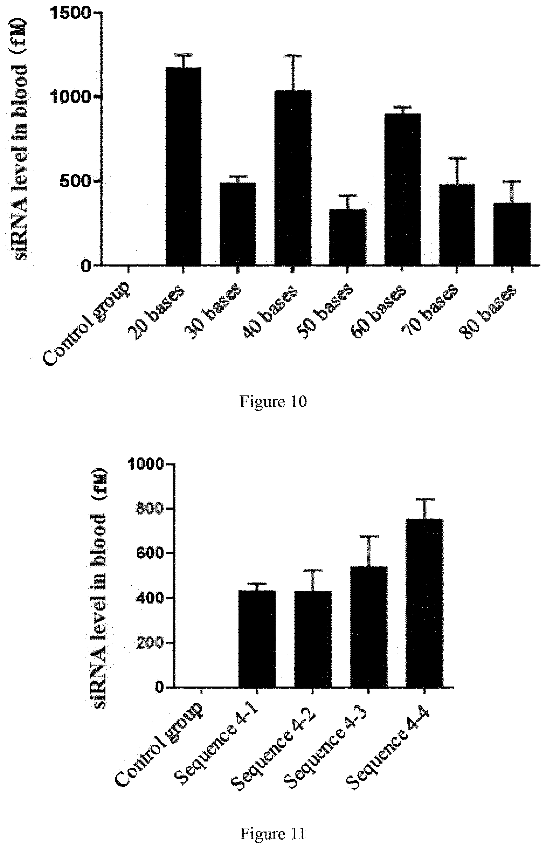

Figure 8 shows the comparison of the influence of different promoters on siRNA provided by an embodiment of the present application.

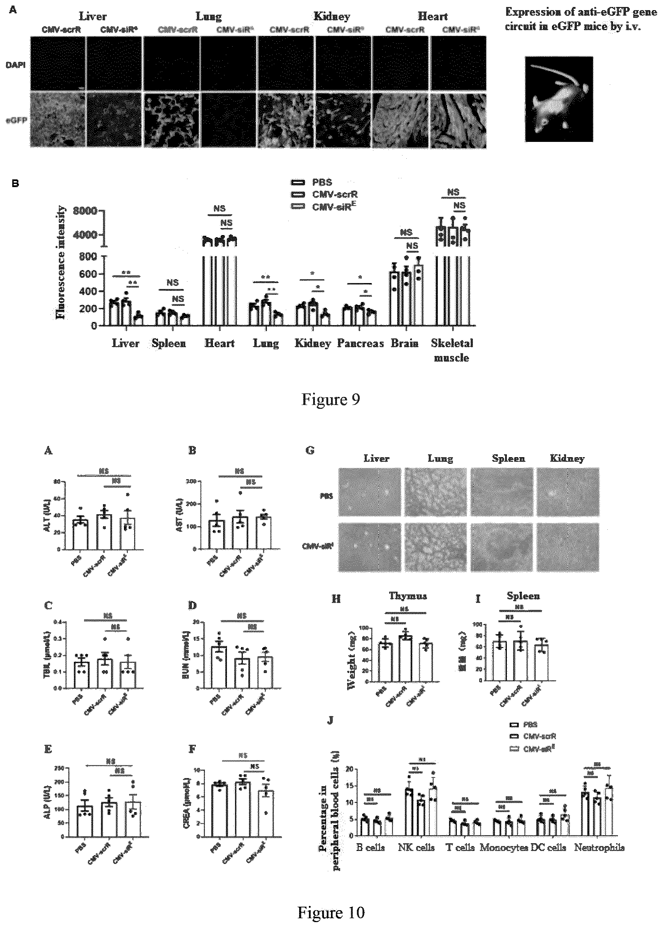

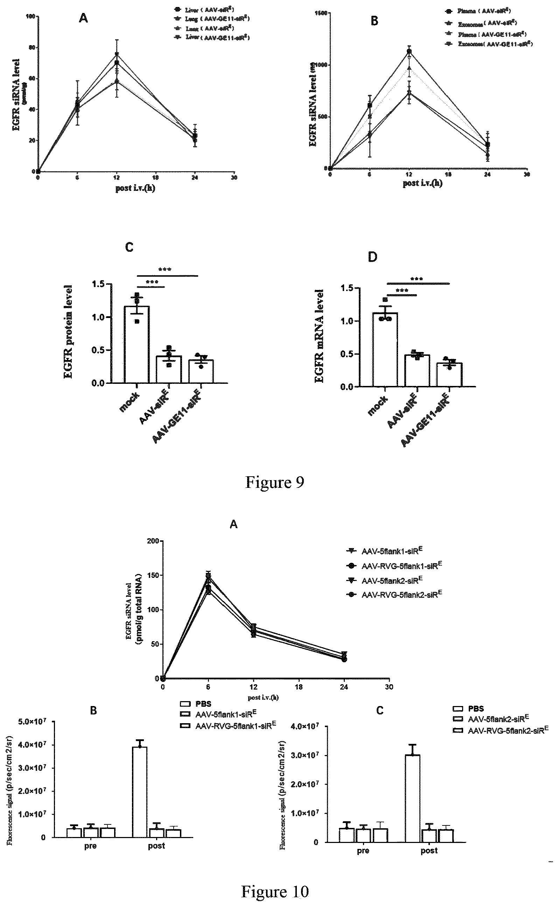

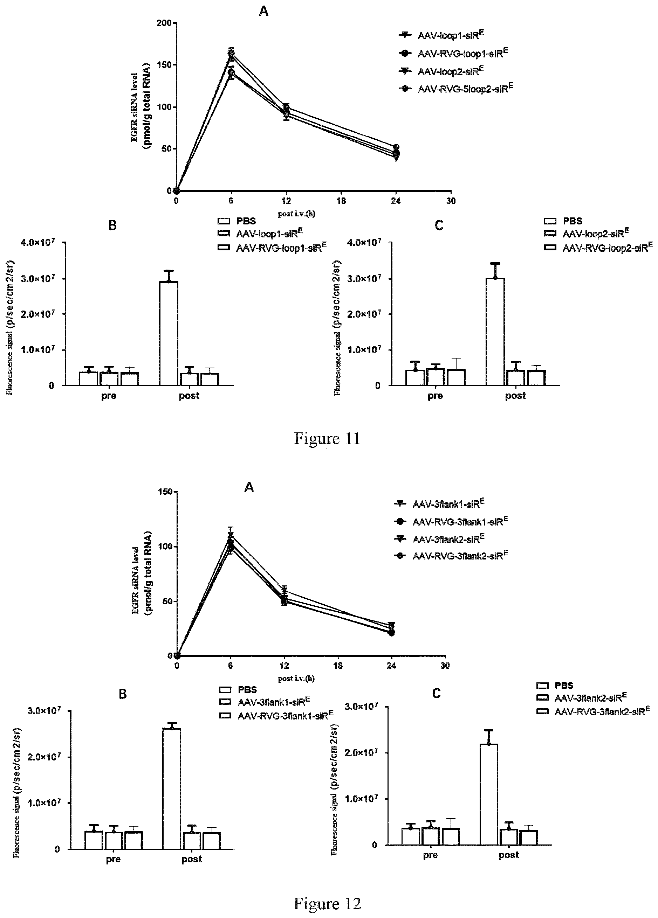

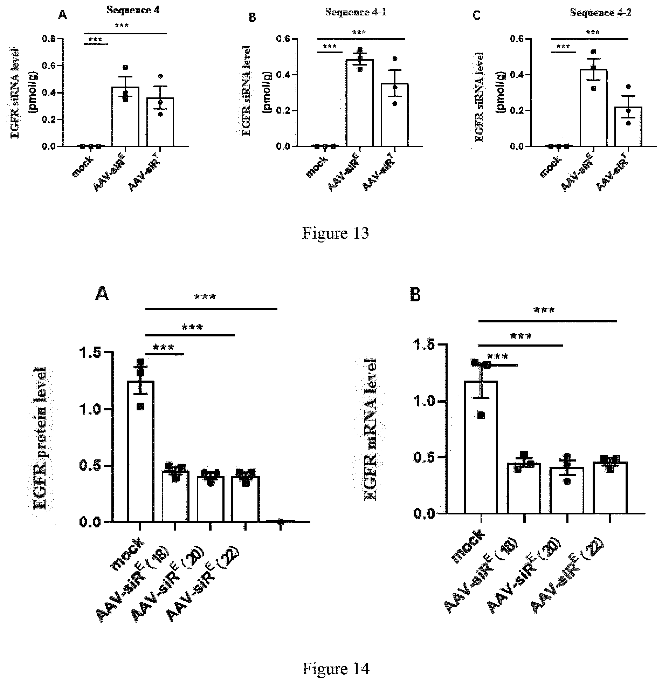

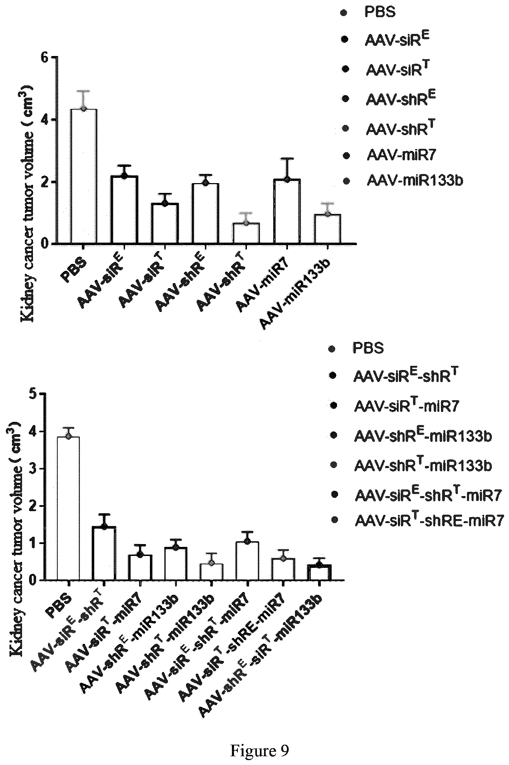

Figure 9 shows the comparison of eGFP fluorescence intensity in different tissues of mice provided by an embodiment of the present application.

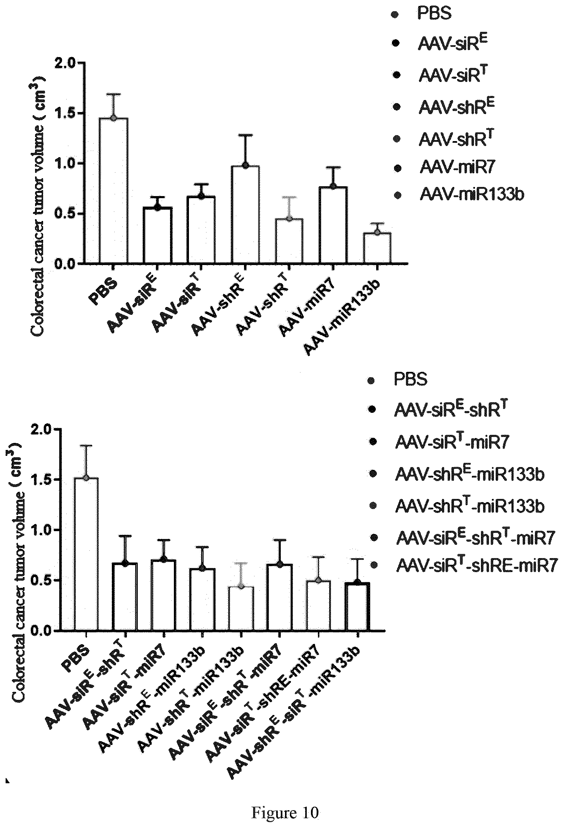

Figure 10 shows the comparison of mouse alanine aminotransferase, aspartate aminotransferase, total bilirubin, blood urea nitrogen, serum alkaline phosphatase, creatinine contents, thymus weight, spleen weight, and percentage in peripheral blood cells provided by an embodiment of the present application.

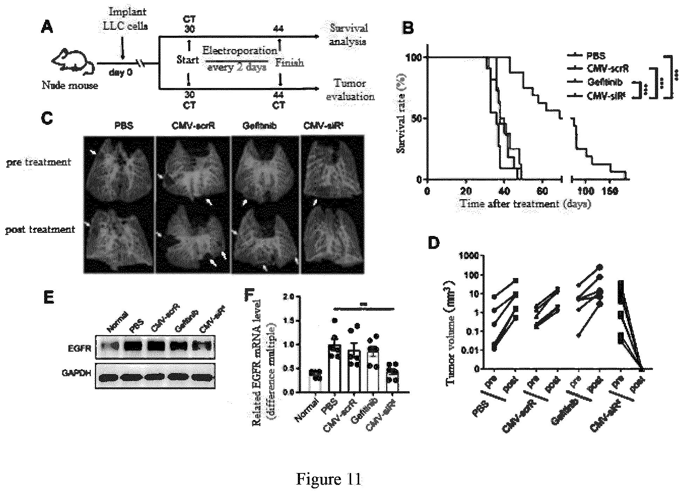

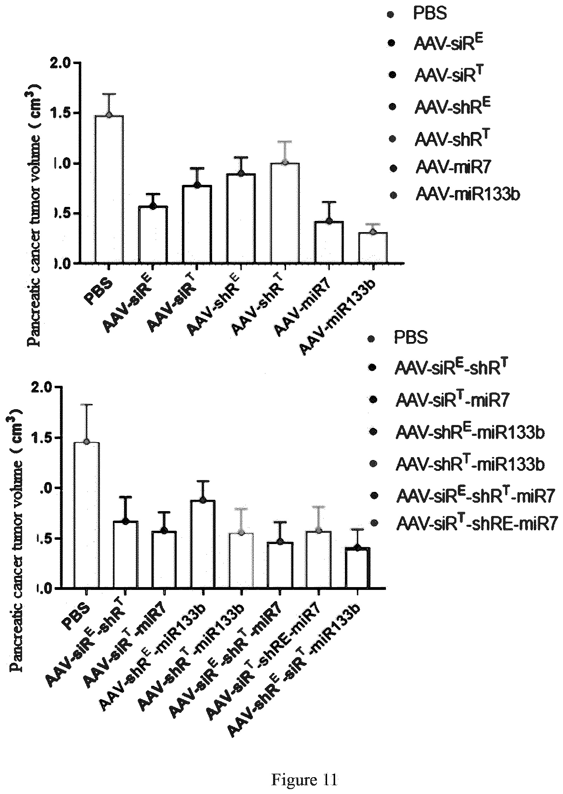

Figure 11 shows the comparison of the therapeutic effects of mouse EGFR mutant lung cancer tumors provided by an embodiment of the present application.

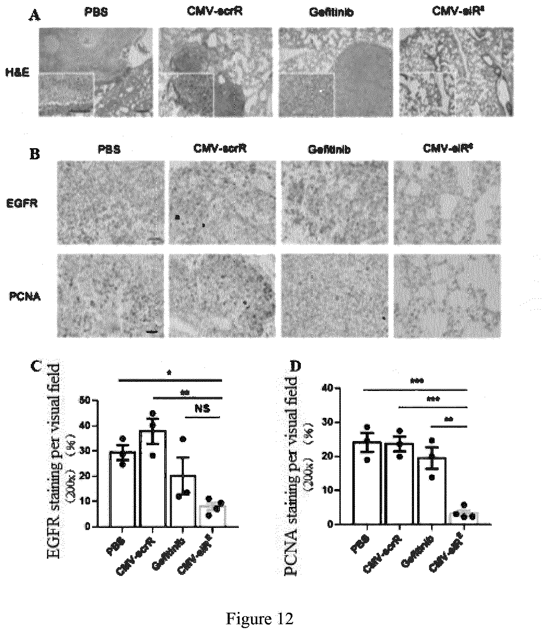

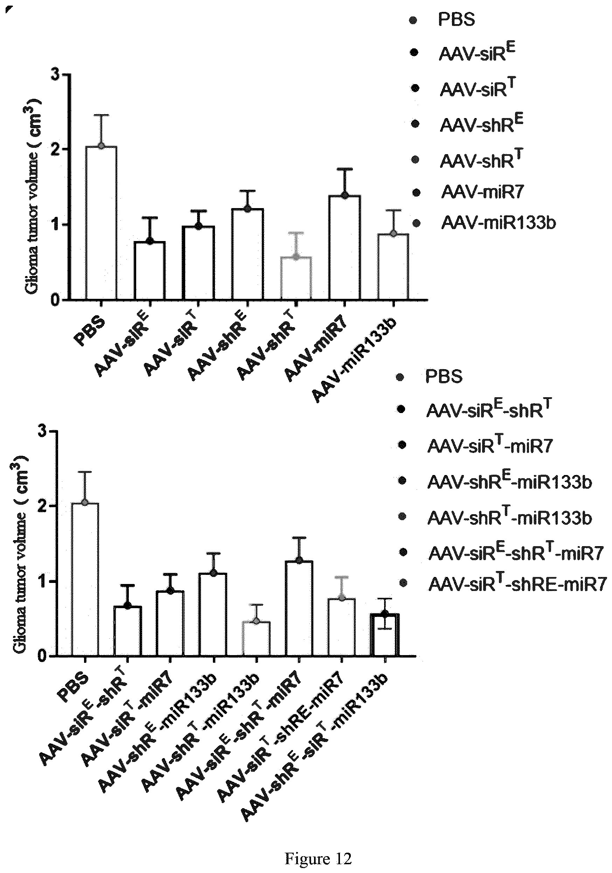

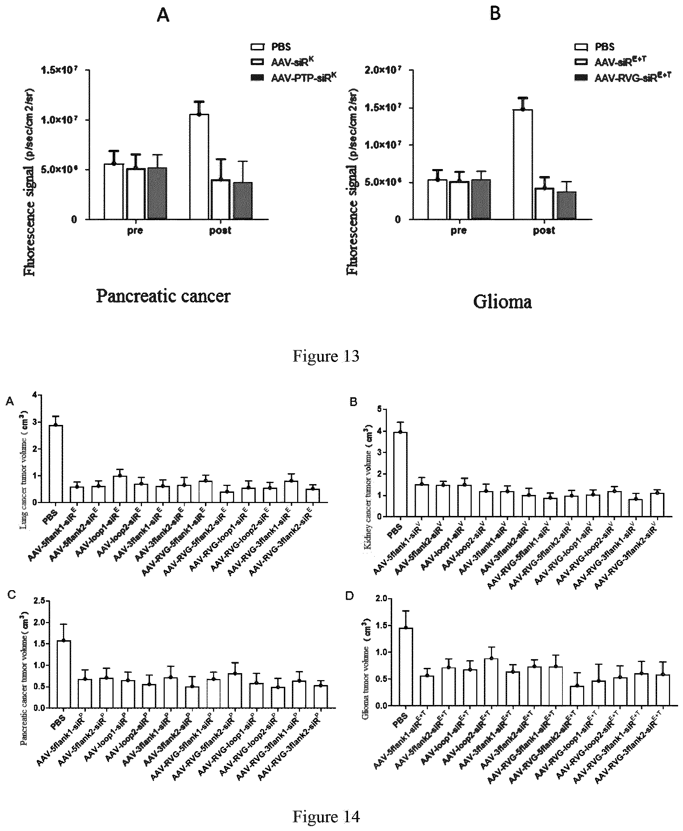

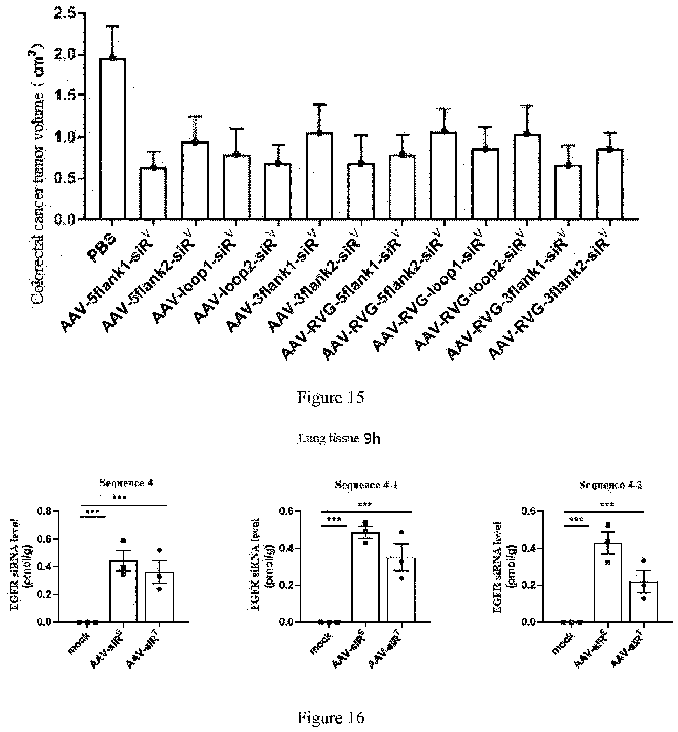

Figure 12 shows mouse HE staining, immunohistochemical staining and staining statistics provided by an embodiment of the present application.

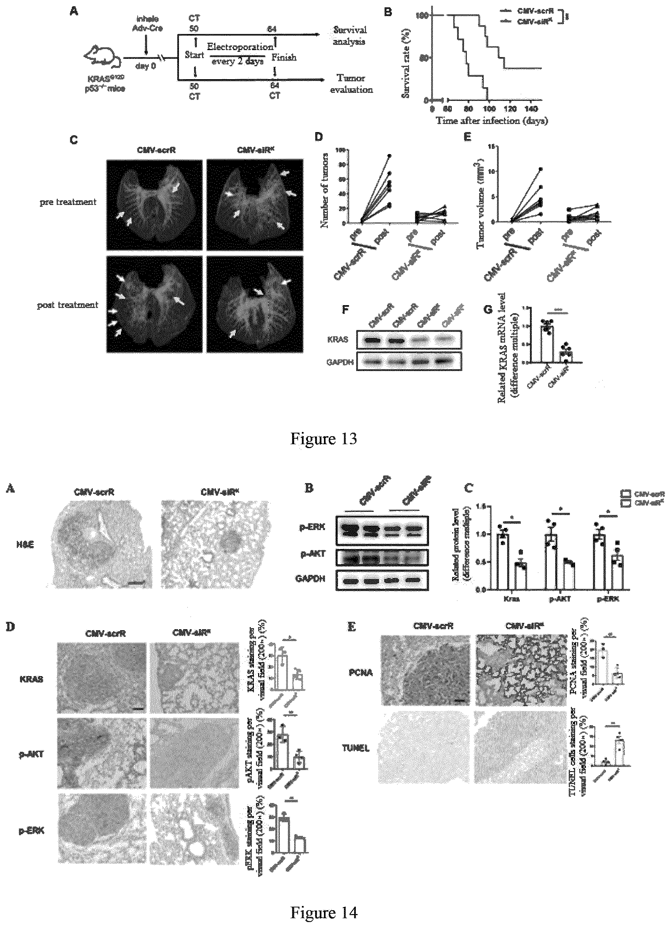

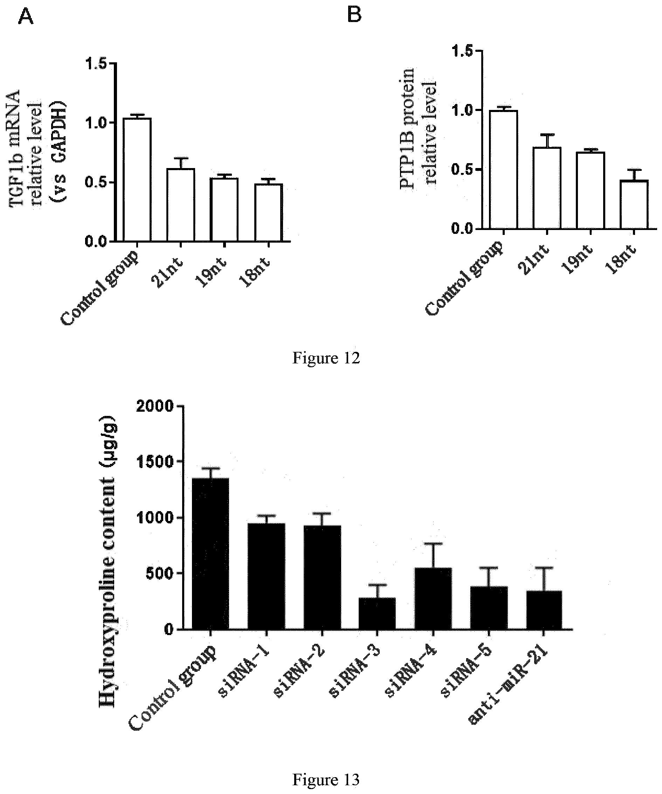

Figure 13 shows the comparison of the therapeutic effects of mouse KRAS mutant lung cancer tumors provided by an embodiment of the present application.

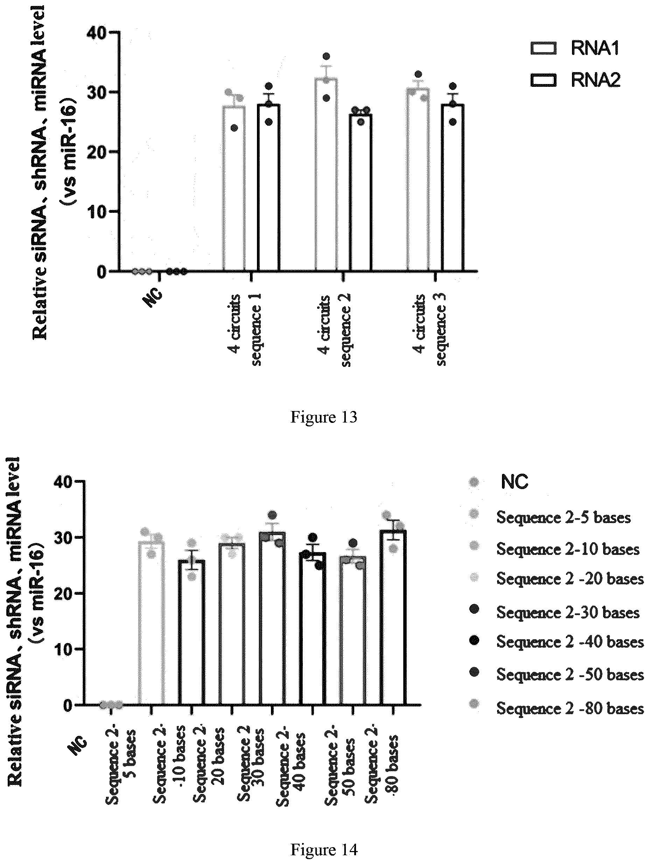

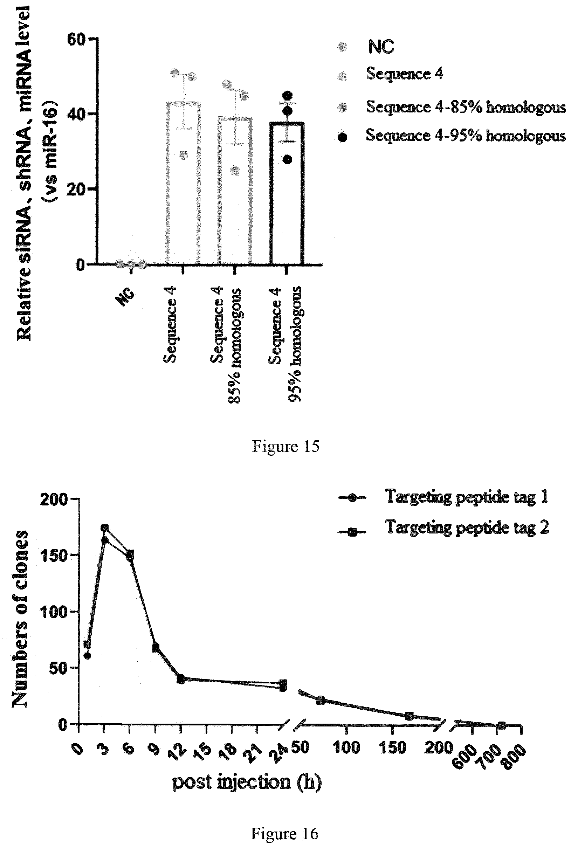

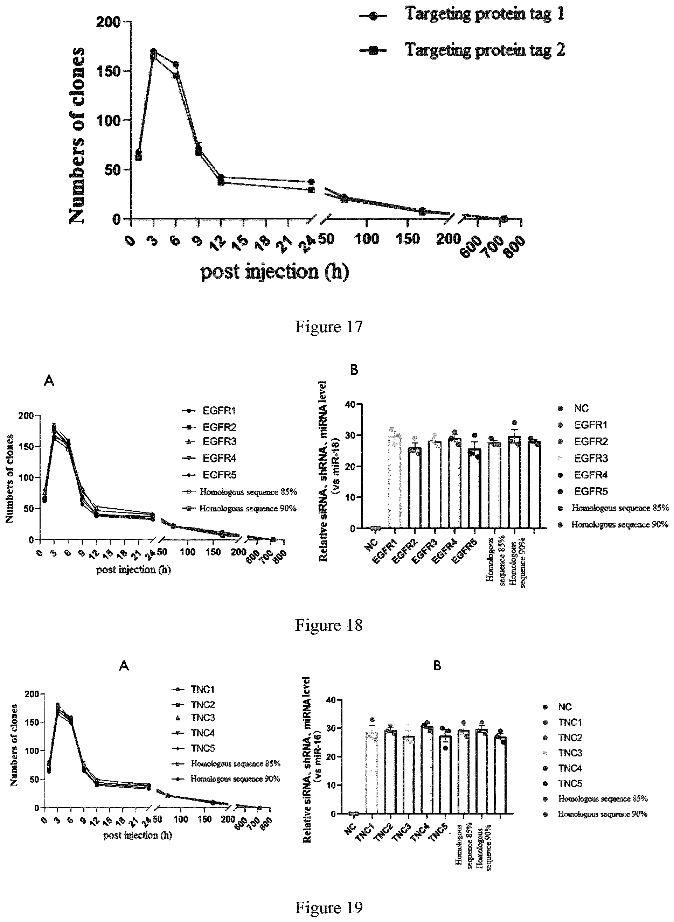

Figure 14 shows mouse HE staining, immunohistochemical staining and staining statistics provided by an embodiment of the present application.

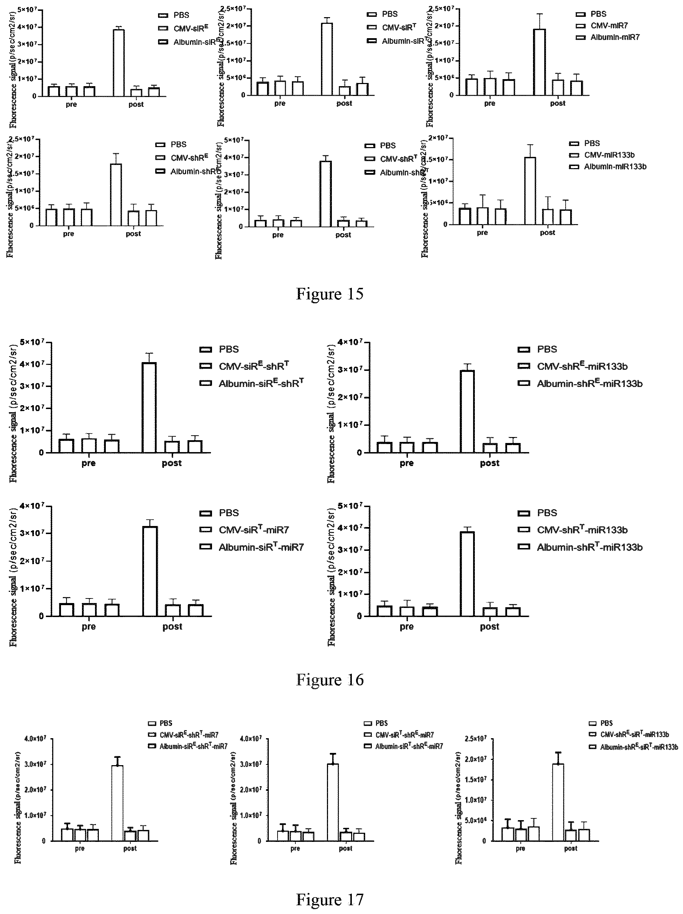

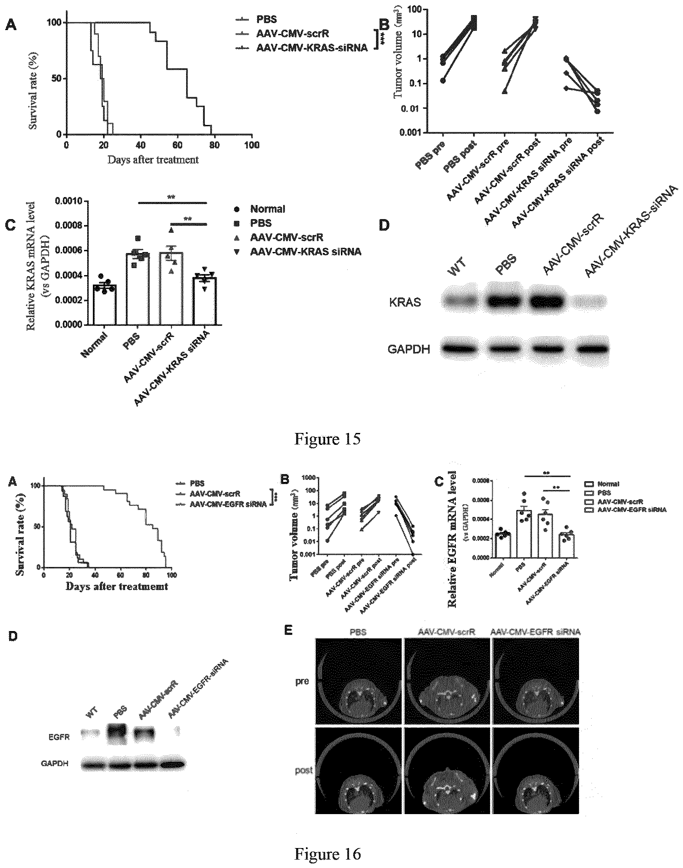

Figure 15 shows the fluorescence signal statistics of six different RNA plasmids after treating lung cancer provided by an embodiment of the present application; where A shows the fluorescence detection results of CMV-siR^{E} and Albumin-siR^{E}, B shows the fluorescence detection result of CMV-siR^{T} and Albumin-siR^{T}, C shows the fluorescence detection results of CMV-miR7 and Albumin-miR7, D shows the fluorescence detection results of CMV-shR^{E} and Albumin-shR^{E}, E shows the fluorescence detection results of CMV-shR^{T} and Albumin-shR^{T}, and F shows the fluorescence detection results of CMV-miR133b and Albumin-miR133b.
Figure 16 shows the fluorescence signal statistics of four plasmids comprising any two RNA sequences of six RNAs with CMV or Albumin linking to the RNA after treating lung cancer provided by an embodiment of the present application; where A shows the fluorescence detection result of siR^{E}+shR^{T}, B shows the fluorescence detection result of shR^{E}+miR133b, C shows the fluorescence detection result of siR^{T}+miR7, and D shows the fluorescence detection result of shR^{T}+miR133b.
Figure 17 shows the fluorescence signal statistics of three plasmids comprising any three RNA sequences of six RNAs with CMV or Albumin linking to the RNA after treating lung cancer provided by an embodiment of the present application; where A shows the fluorescence detection result of siR^{E}+shR^{T}+miR7, B shows the fluorescence detection result of siR^{T}+shR^{E}+miR7, and C shows the fluorescence detection result of shR^{E}+siR^{T}+miR133b.

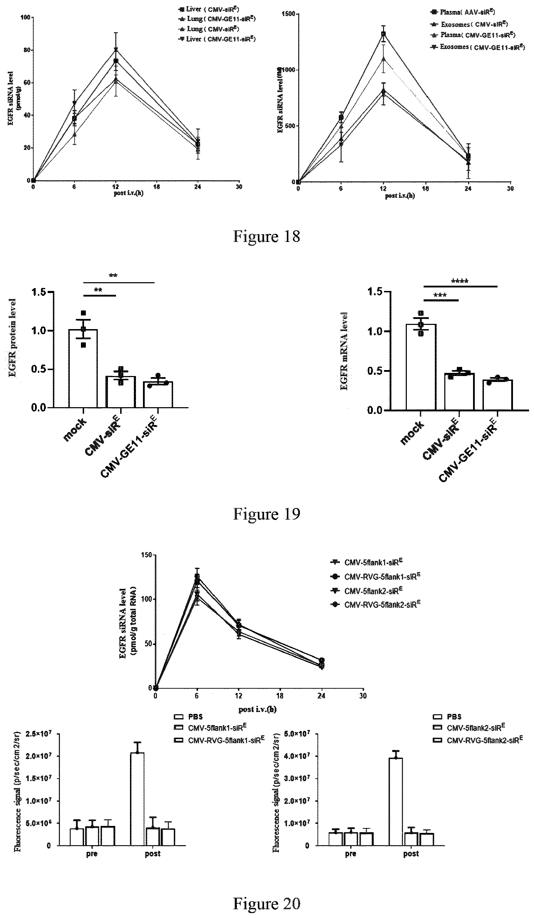

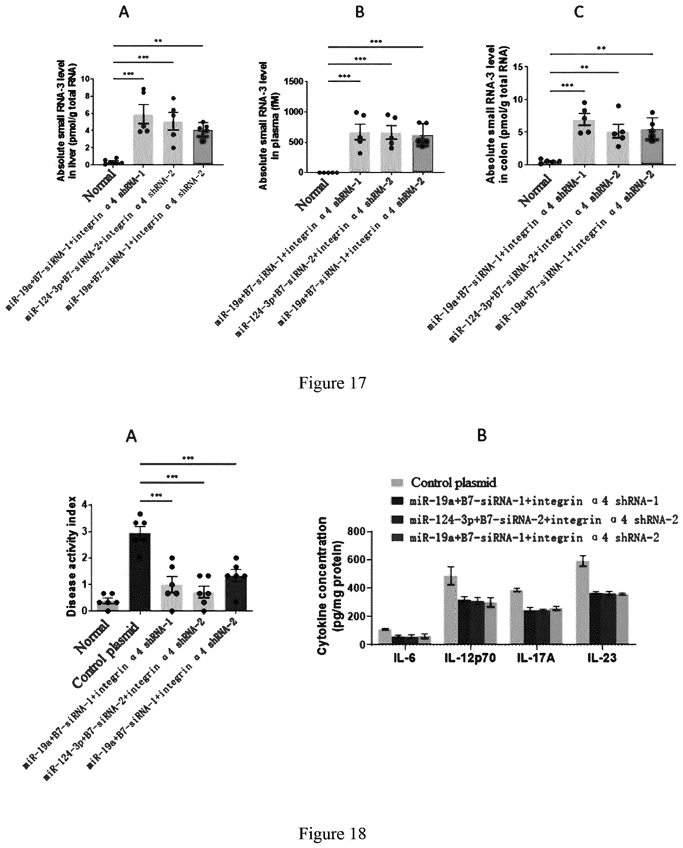

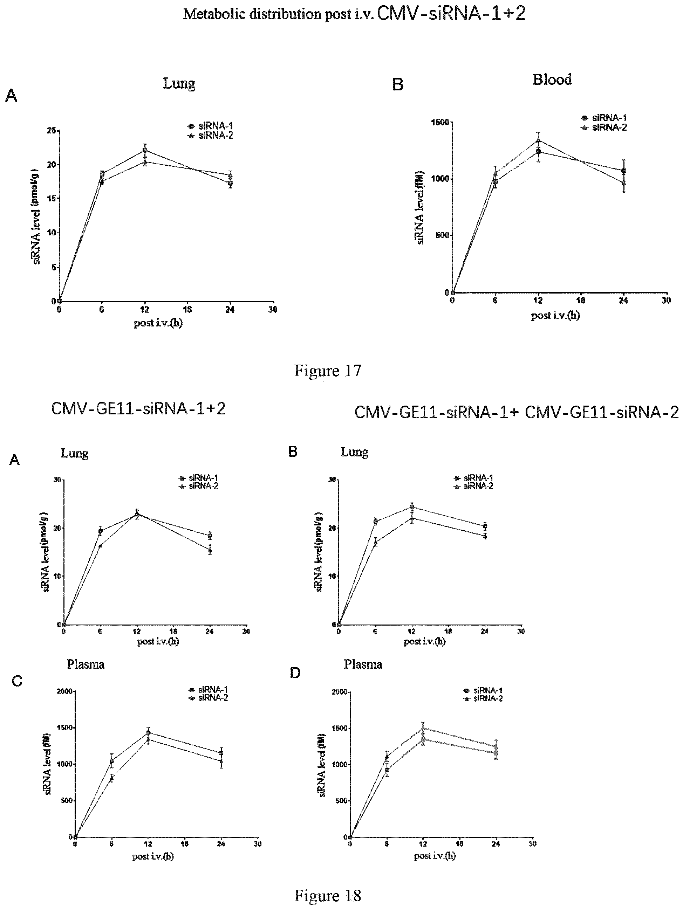

Figure 18 shows the enrichment results of siRNA in the liver, lung, plasma, and exosomes after intravenous injection of plasmid CMV-siR^{E} and plasmid CMV-GE11-siR^{E} respectively provided by an embodiment of the present application; where A shows the EGFR siRNA level in the liver and lung with/without the targeting peptide GE11, and B shows the EGFR siRNA level in plasma and exosomes with/without the targeting peptide GE11.

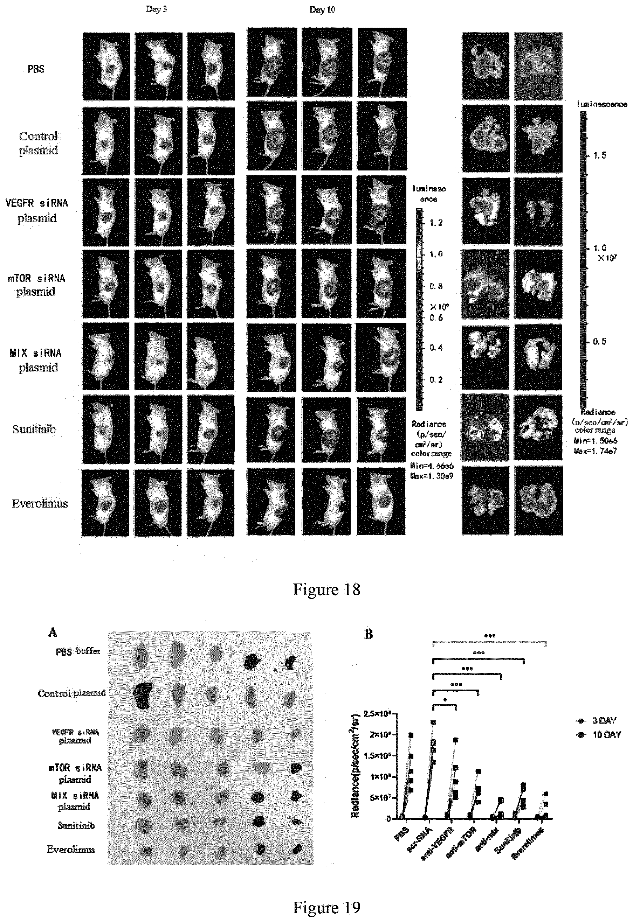

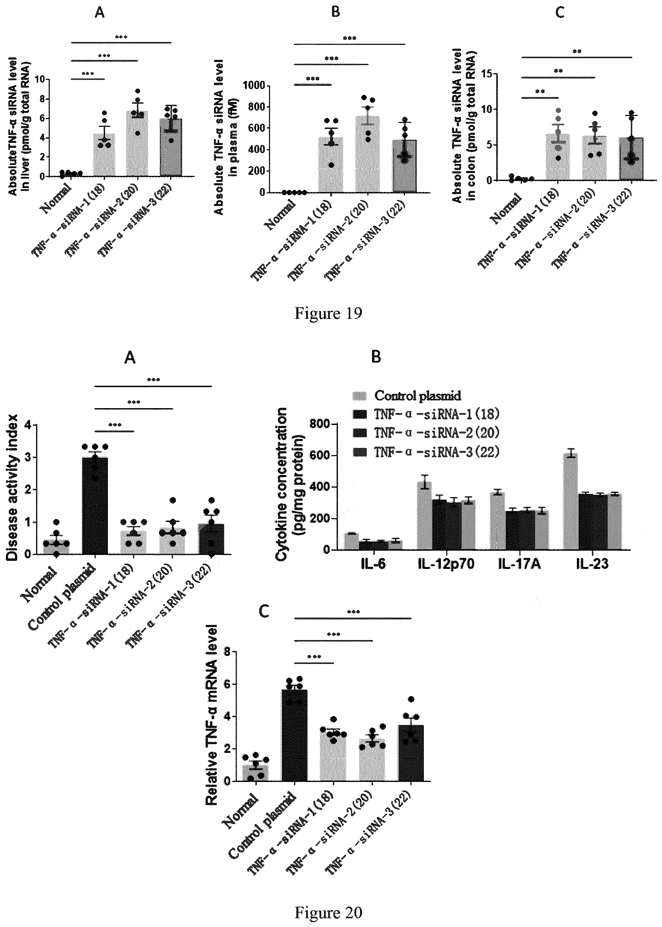

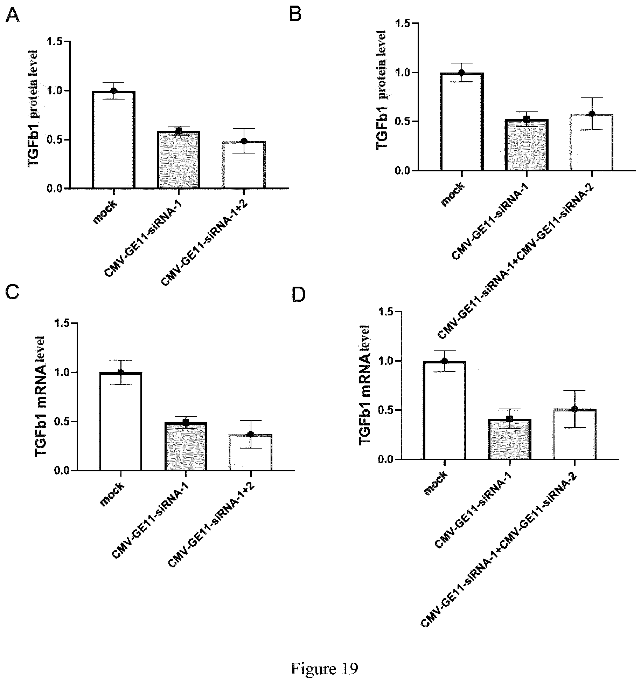

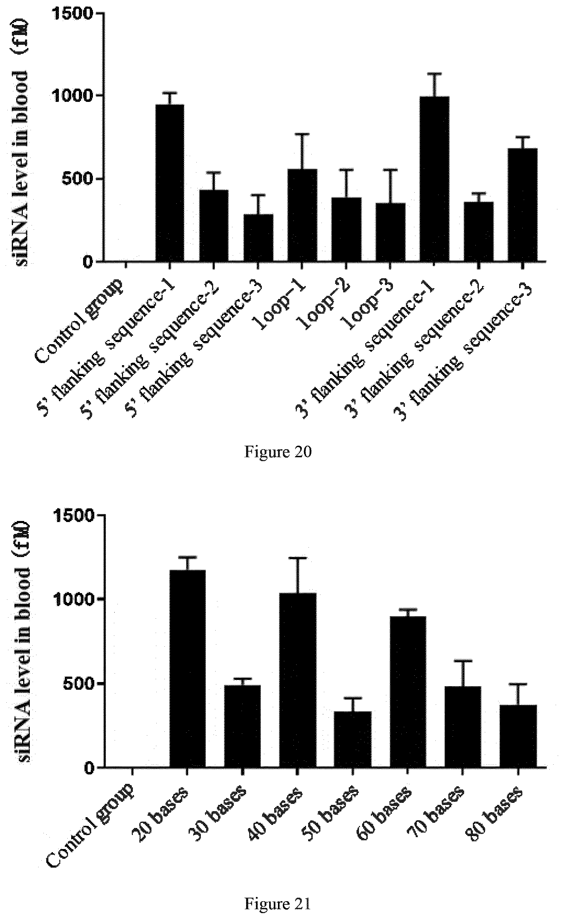

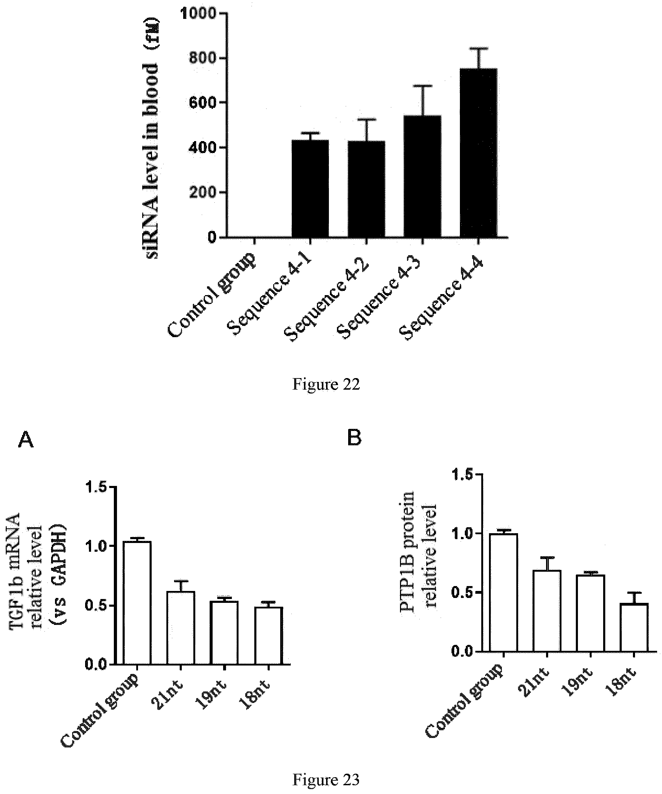

Figure 19 shows the expression levels of EGFR protein and mRNA after intravenous injection of plasmid CMV-siR^{E} and plasmid CMV-GE11-siR^{E} respectively provided by an embodiment of the present application; where A shows the protein level of EGFR with/without the targeting peptide GE11, and B shows the mRNA level of EGFR with/without the targeting peptide GE11.

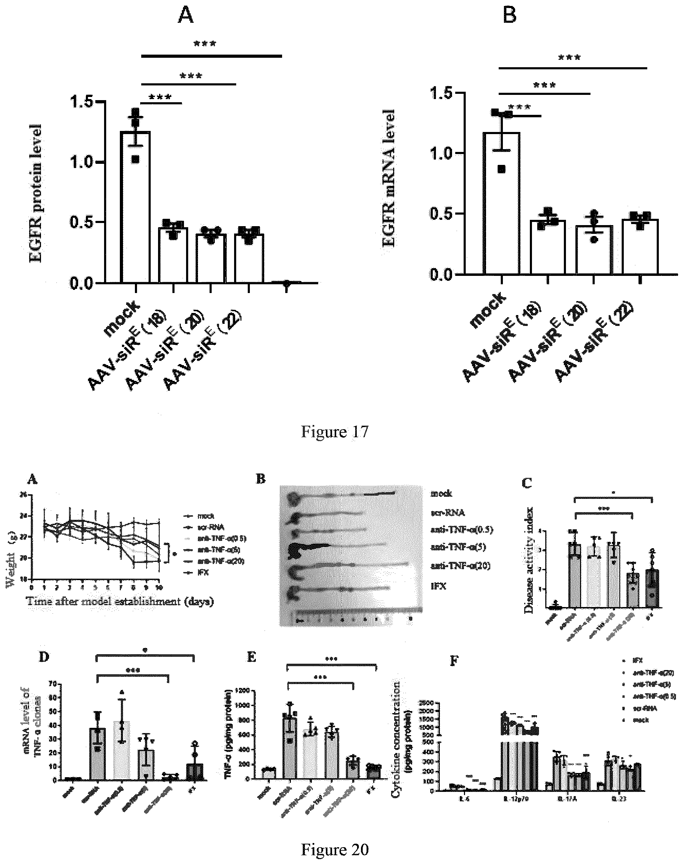

Figure 20 shows enrichment and therapeutic effects of the plasmid containing a more than 80% homologous 5' flanking sequence in the lung provided by an embodiment of the present application; A shows the EGFR siRNA levels of two homologous 5' flanking sequences with/without the targeting tag RVG, B shows the fluorescence signal detection result of one homologous 5' flanking sequence with/without the targeting tag RVG, C shows the fluorescence signal detection result of another homologous 5' flanking sequence with/without the targeting tag RVG.

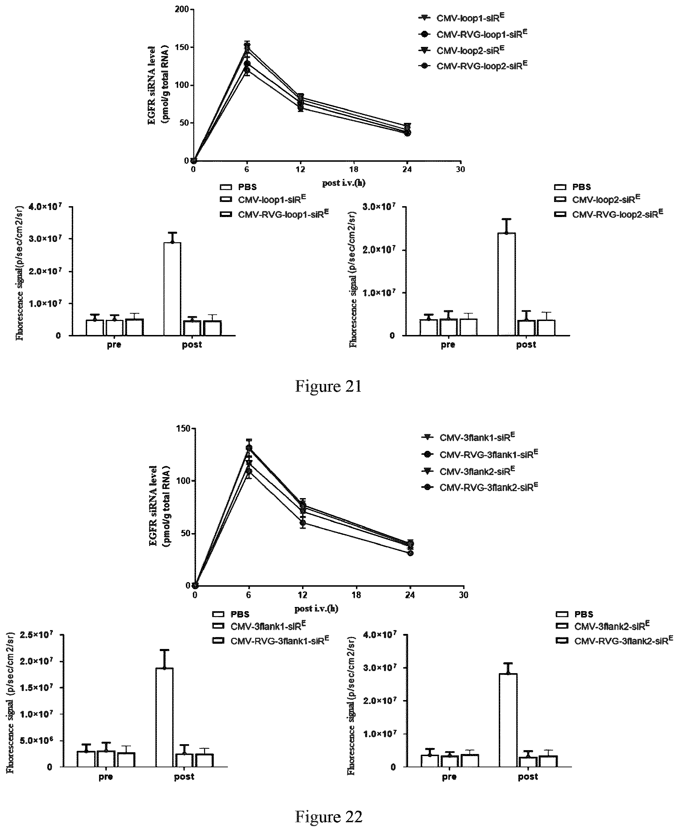

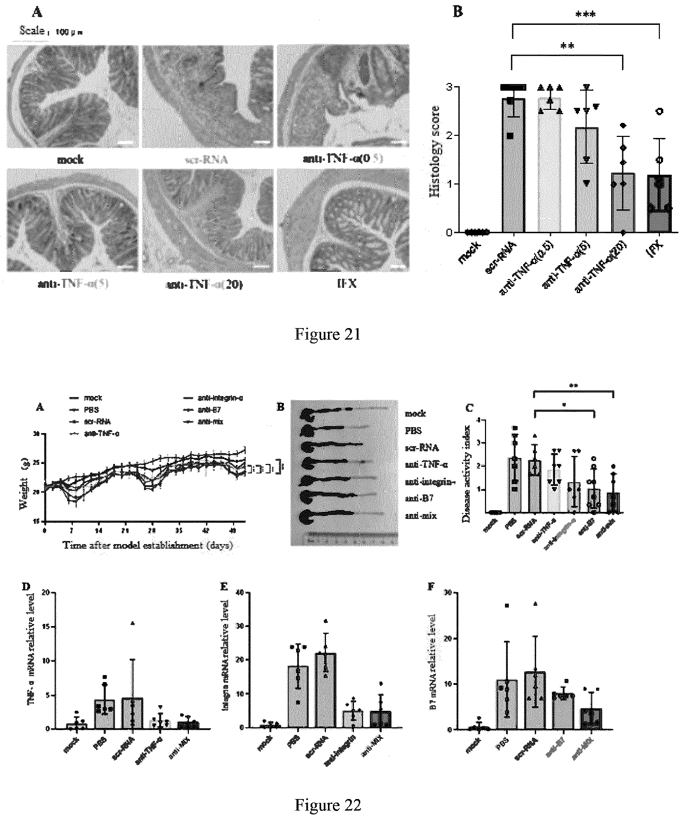

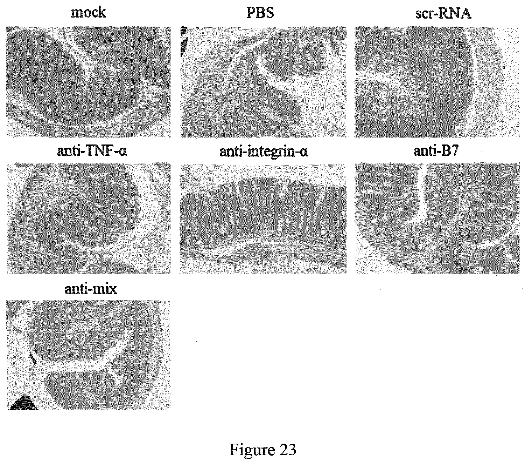

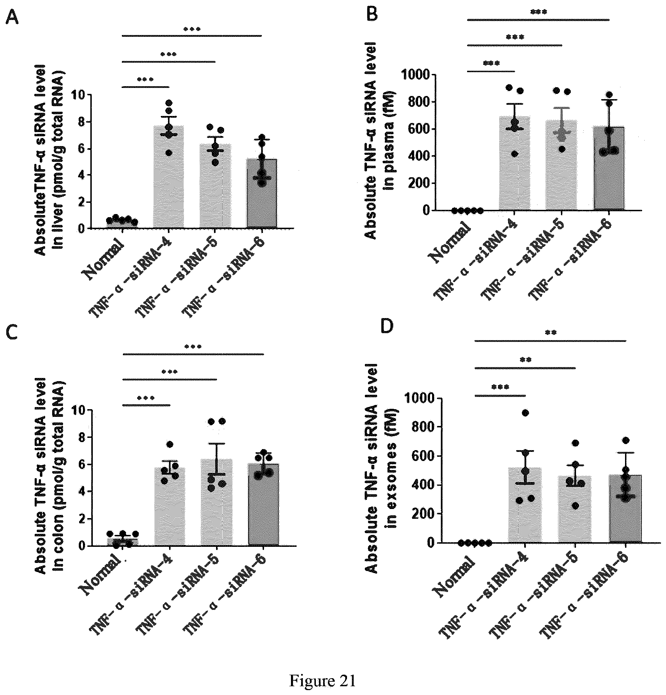

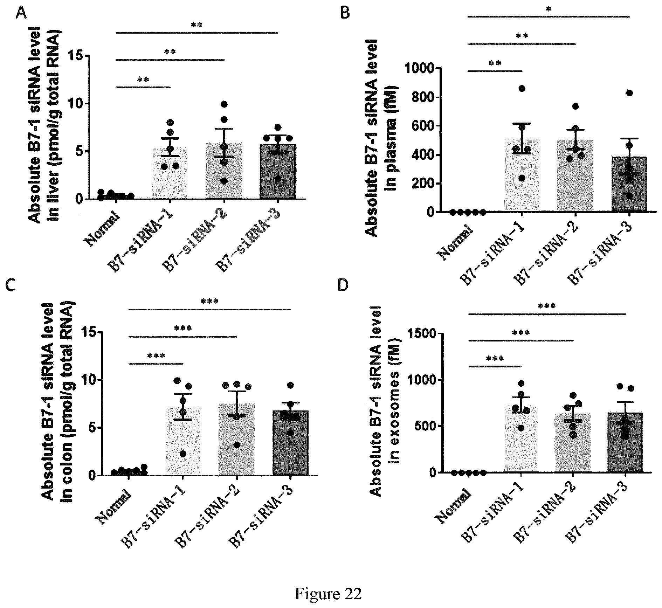

Figure 21 shows enrichment and therapeutic effects of the plasmid containing a more than 80% homologous loop sequence in the lung provided by an embodiment of the present application; A shows the EGFR siRNA levels of two homologous loop sequences with/without the targeting tag RVG, B shows the fluorescence signal detection result of one homologous loop sequence with/without the targeting tag RVG, C shows the fluorescence signal detection result of another homologous loop sequence with/without the targeting tag RVG.
Figure 22 shows enrichment and therapeutic effects of the plasmid containing a more than 80% homologous 3' flanking sequence in the lung provided by an embodiment of the present application; A shows the EGFR siRNA levels of two homologous 3' flanking sequences with/without the targeting tag RVG, B shows the fluorescence signal detection result of one homologous 3' flanking sequence with/without the targeting tag RVG, C shows the fluorescence signal detection result of another homologous 3' flanking sequence with/without the targeting tag RVG.

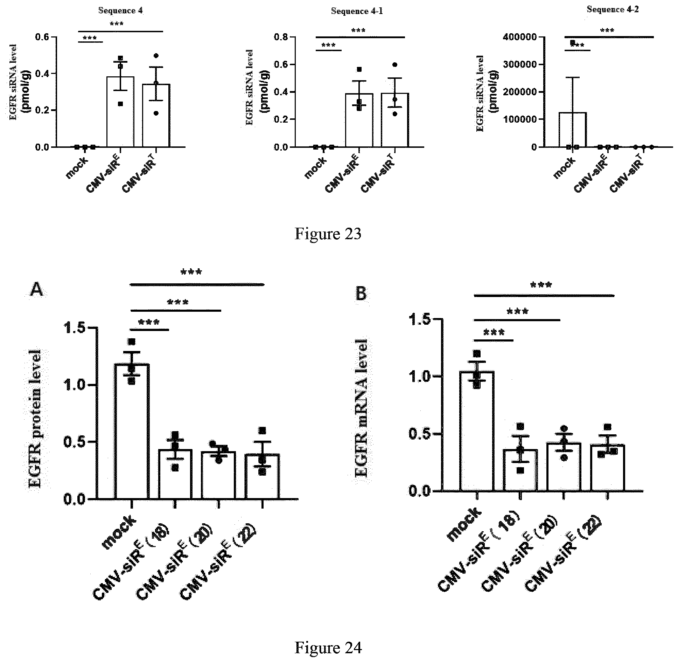

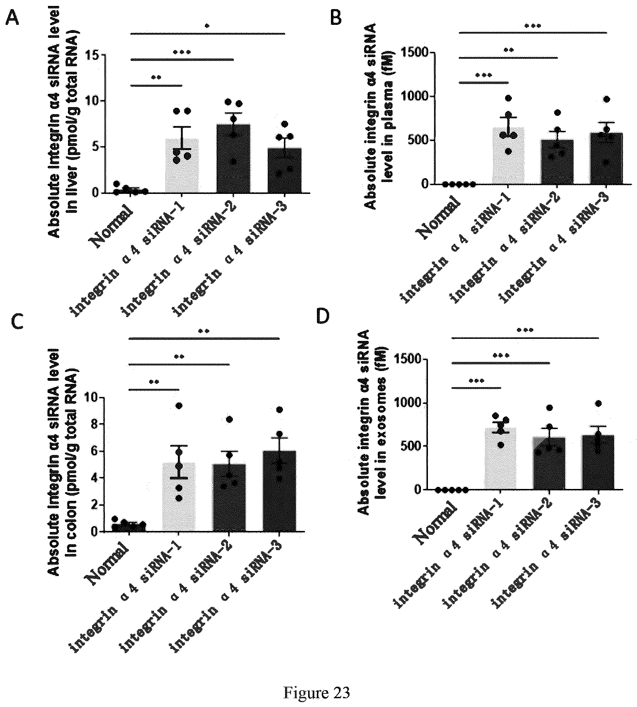

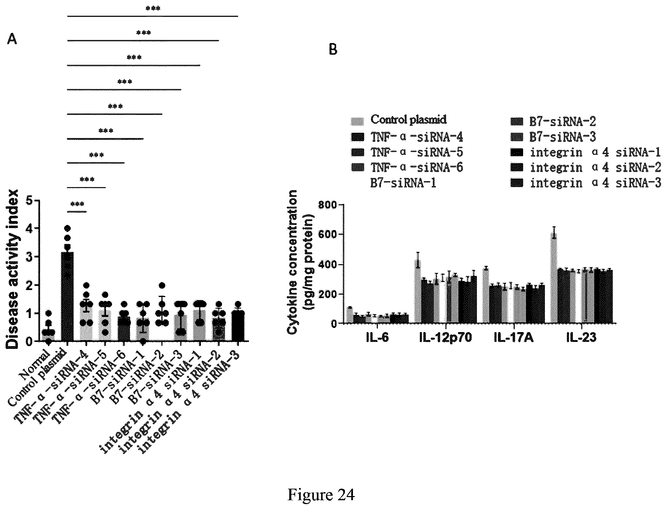

Figure 23 shows the detection results of EGFR siRNA level in the lung tissue after 9 h of intravenous injection of a plasmid containing sequence 4 or two sequences 4-1 and 4-2 with more than 80% homology to sequence 4 provided with siR^{E}/siR^{T} as the RNA by an embodiment of the present application; where A shows the EGFR siRNA level with sequence 4, B shows the EGFR siRNA level with sequence 4-1, and C shows the EGFR siRNA level with sequence 4-2.

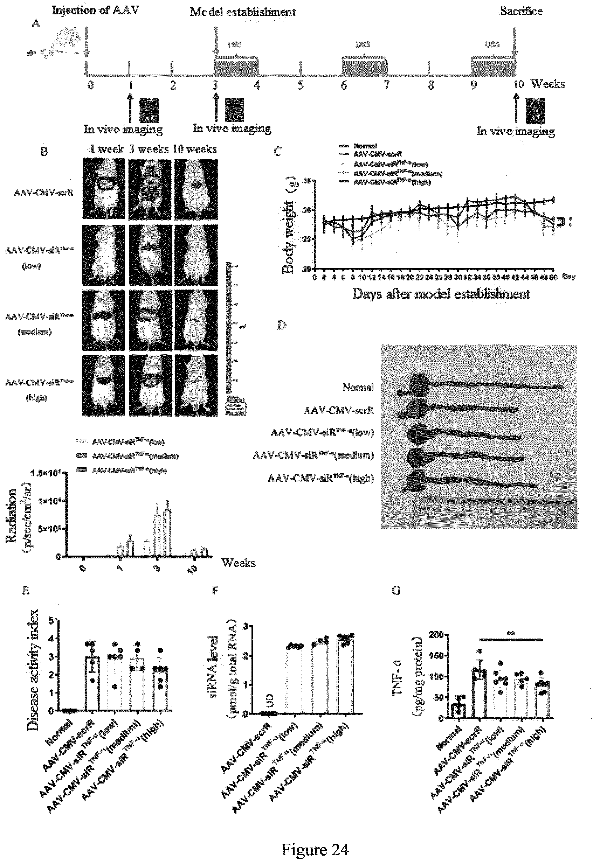

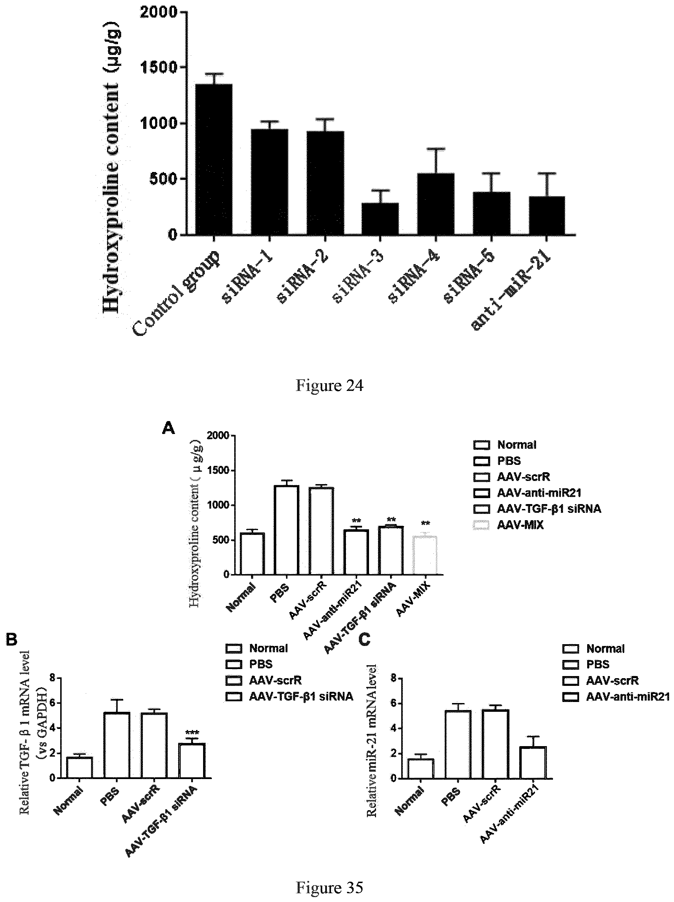

Figure 24 shows the detection results of EGFR expression levels after intravenous injection of three plasmids containing RNA sequences of lengths of 18, 20, and 22 provided by an embodiment of the present application; where A shows the protein level of EGFR, and B shows the mRNA level of EGFR.

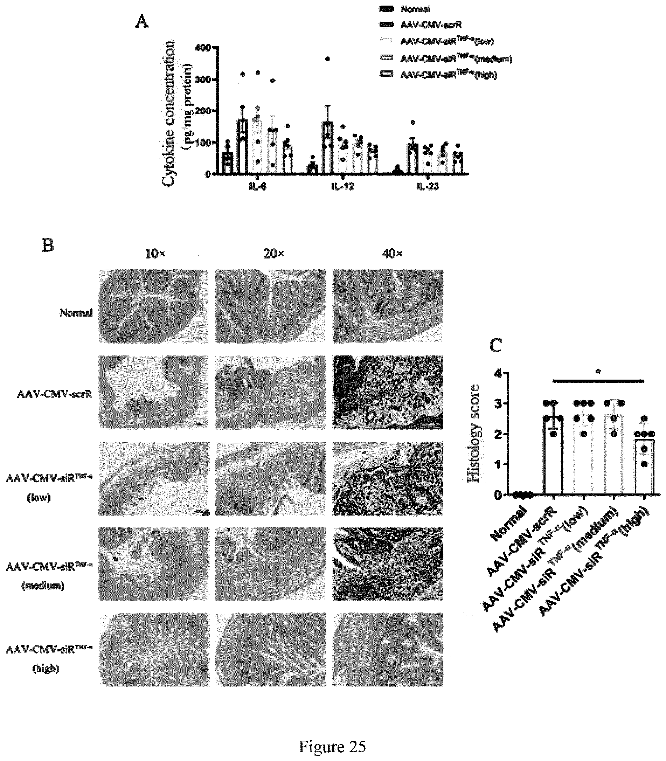

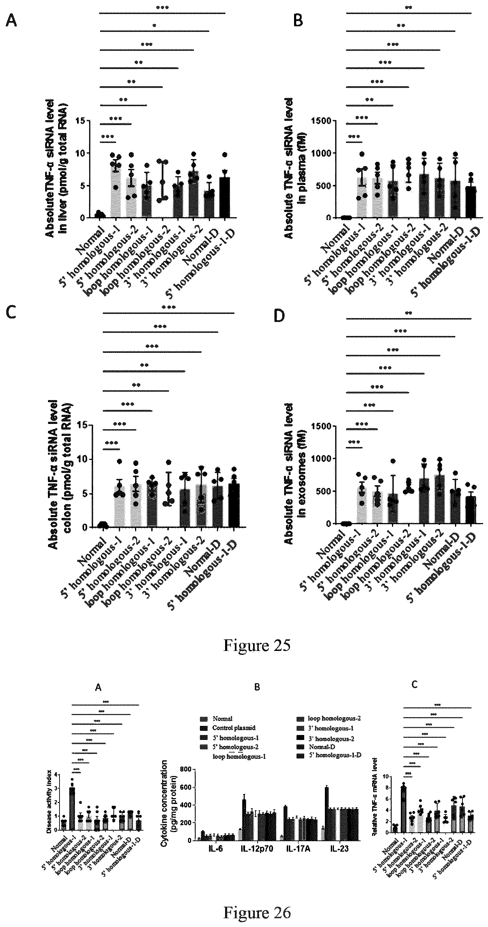

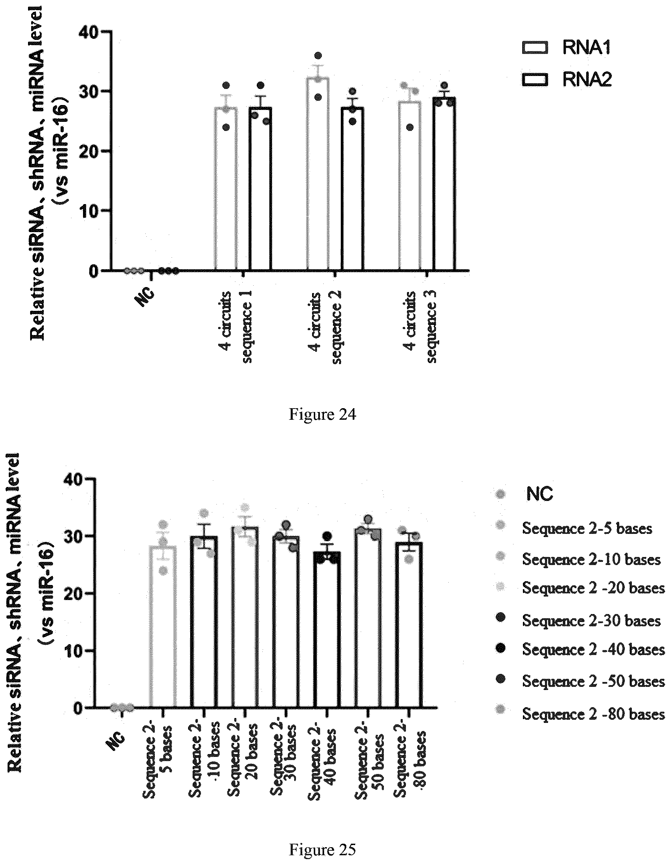

Figure 25 shows the treatment of lung cancer in mice based on KRAS siRNA provided by an embodiment of the present application.

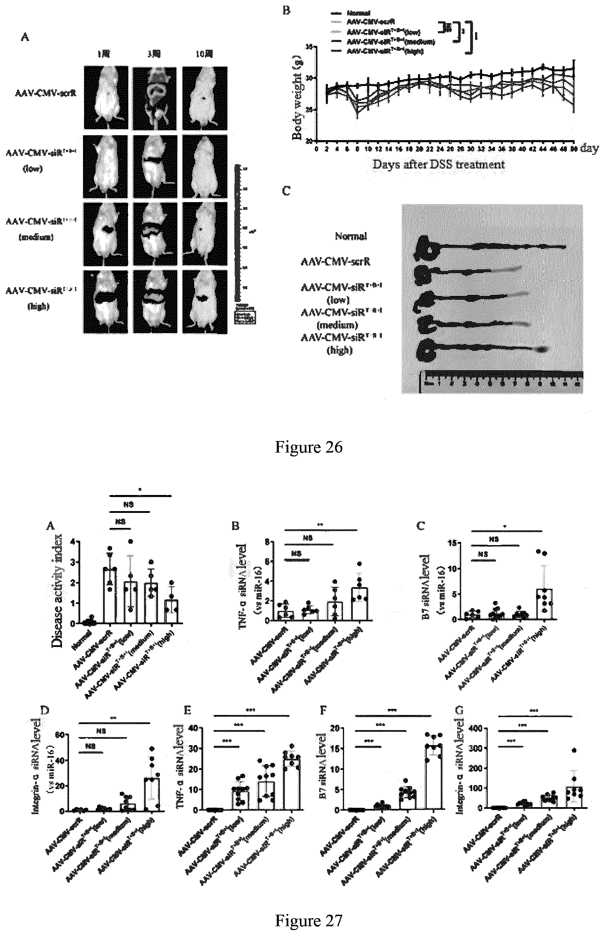

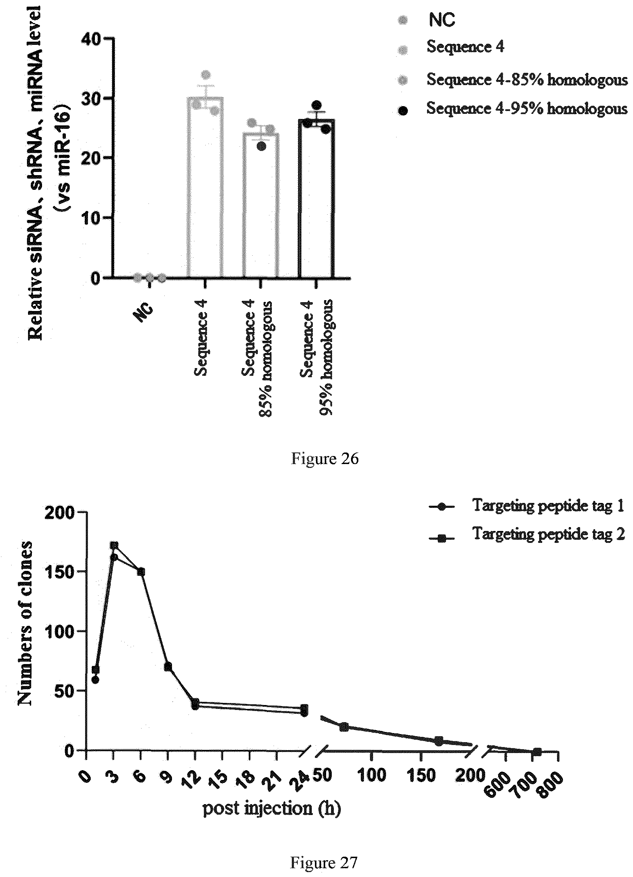

Figure 26 shows the treatment of lung cancer in mice based on EGFR siRNA provided by an embodiment of the present application.
Figure 27 shows the comparison of multiple enzyme contents in mice provided by an embodiment of the present application.

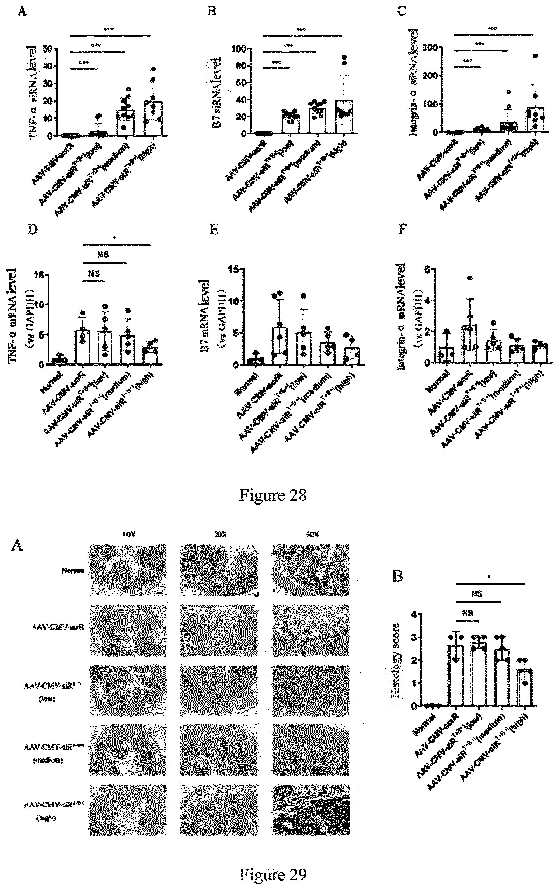

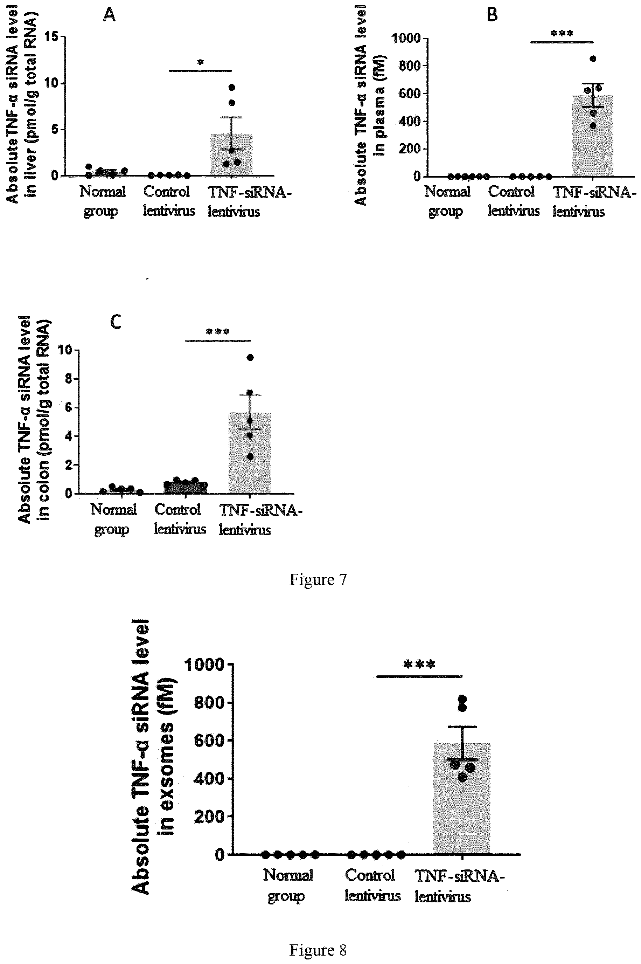

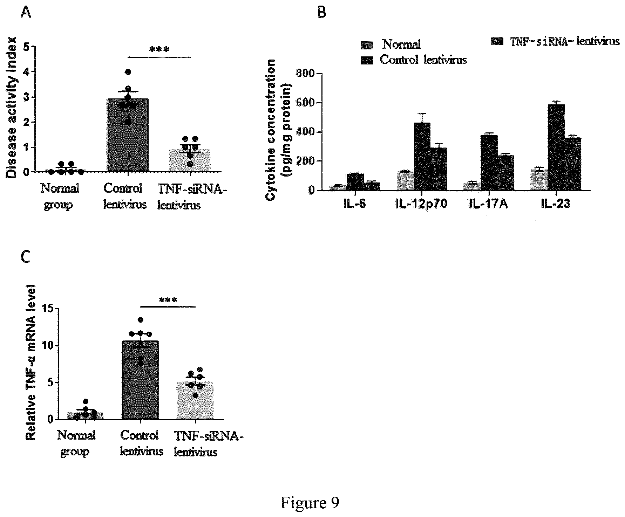

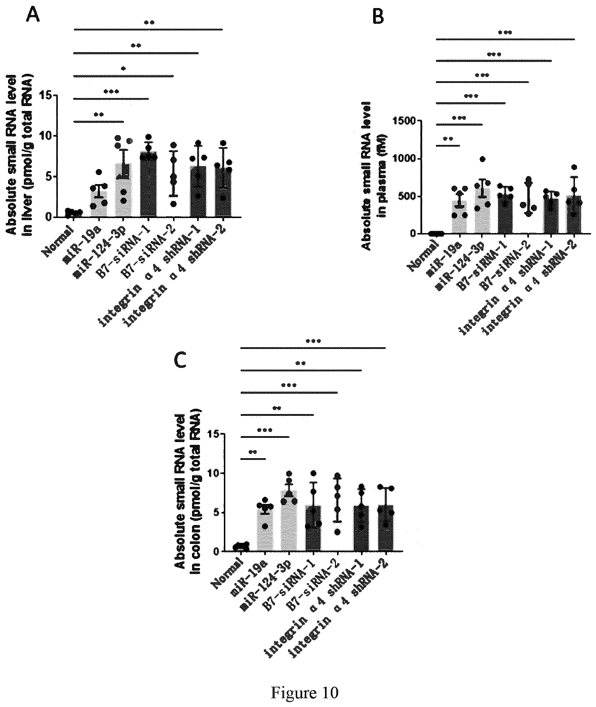

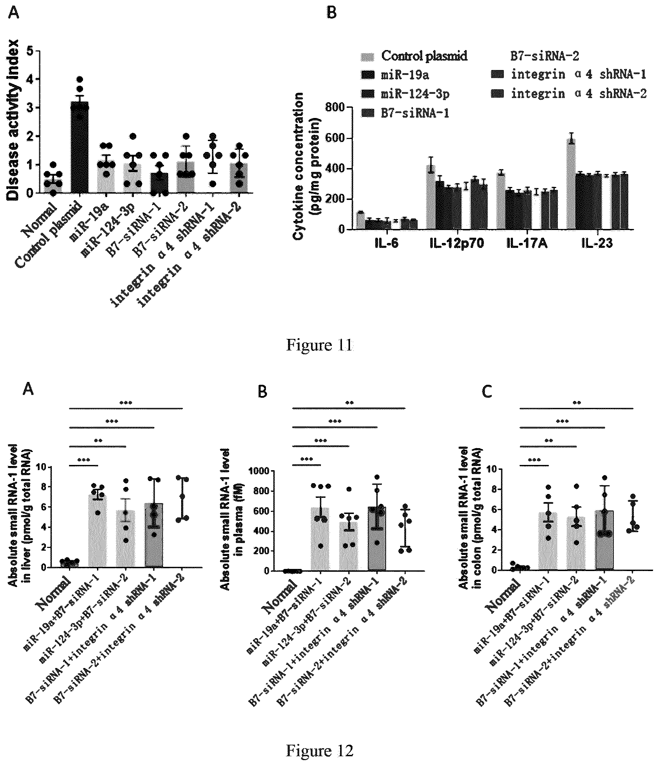

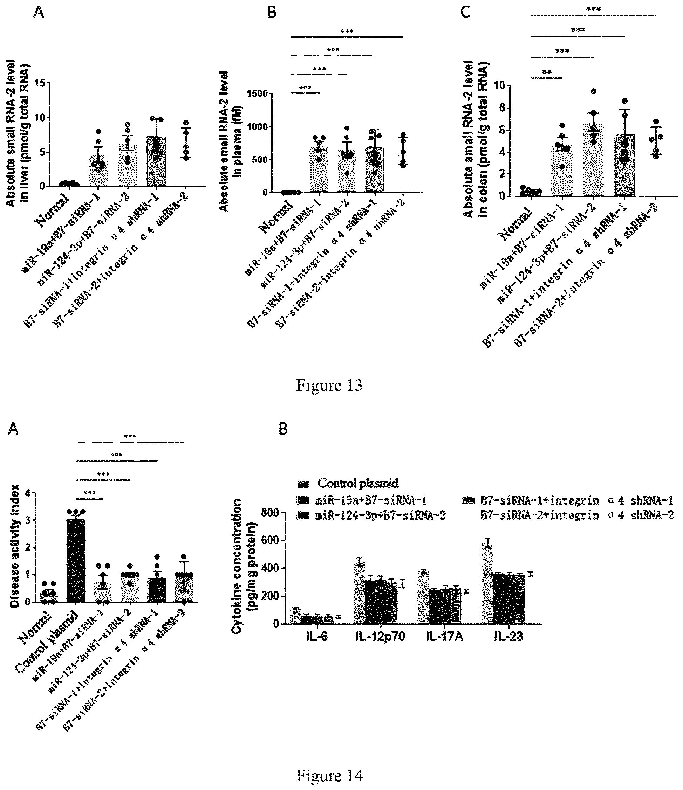

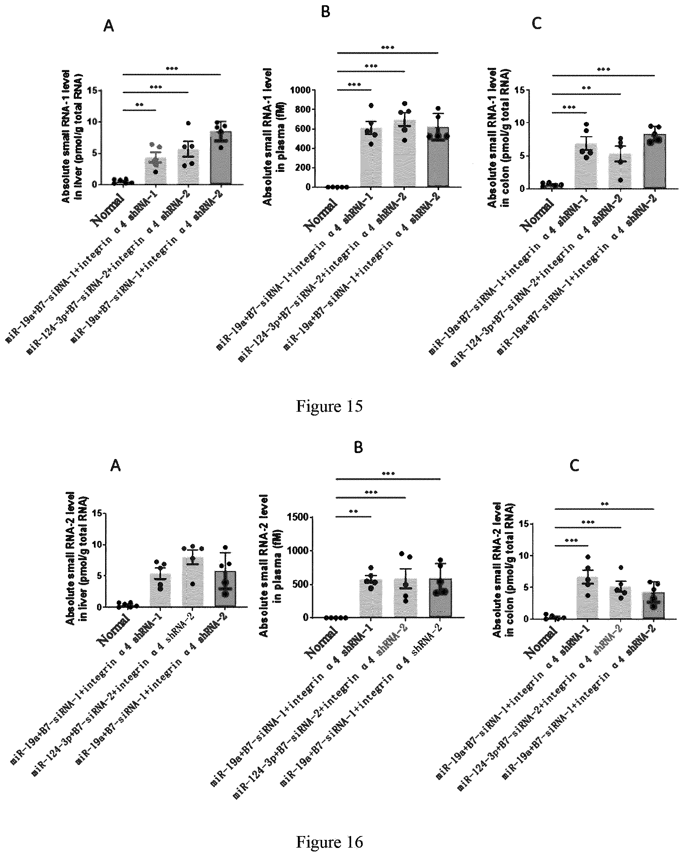

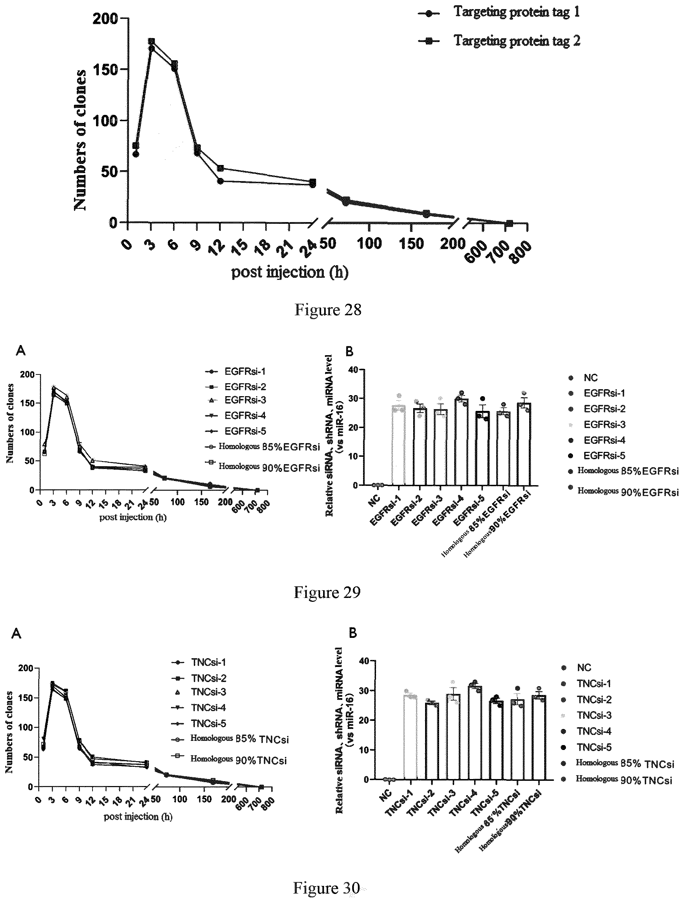

Figure 28 shows the enrichment effect of a lentiviral vector and expression levels of EGFR gene in the liver, lung, plasma and exosomes provided by an embodiment of the present application; where A shows the enrichment effect of EGFR siRNA in the liver and lung after intravenous injection of the lentiviral vector, B shows the enrichment effect of EGFR siRNA in plasma and exosomes after intravenous injection of the lentiviral vector, C shows the protein level of EGFR after intravenous injection of the lentiviral vector, and D shows the mRNA level of EGFR after intravenous injection of the lentiviral vector.

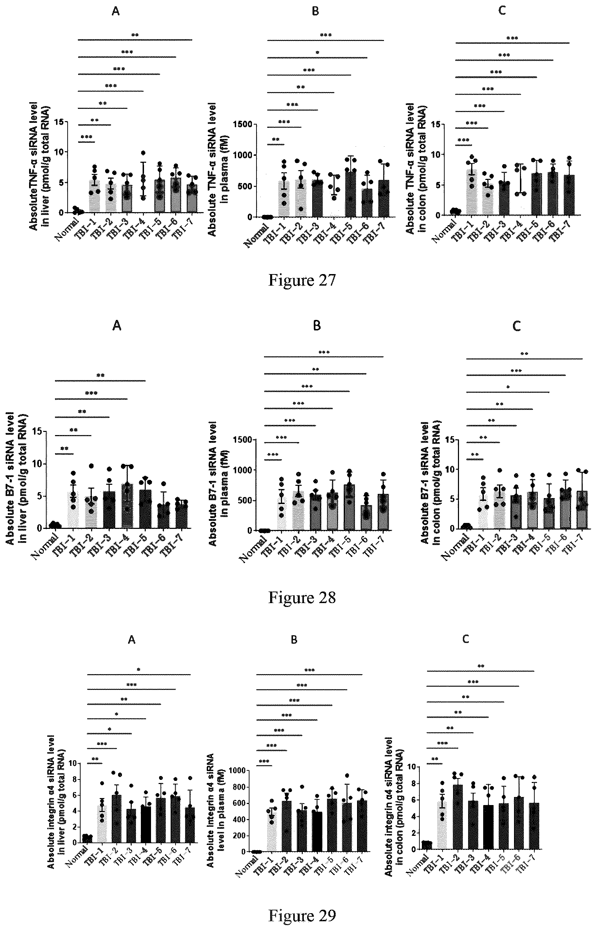

Figure 29 shows the enrichment effect of a adenoviral vector and expression levels of EGFR gene in the liver, lung, plasma and exosomes provided by an embodiment of the present application; where A shows the enrichment effect of EGFR siRNA in the liver and lung after intravenous injection of the adenoviral vector, B shows the enrichment effect of EGFR siRNA in plasma and exosomes after intravenous injection of the adenoviral vector, C shows the protein level of EGFR after intravenous injection of the adenoviral vector, and D shows the mRNA level of EGFR after intravenous injection of the adenoviral vector.

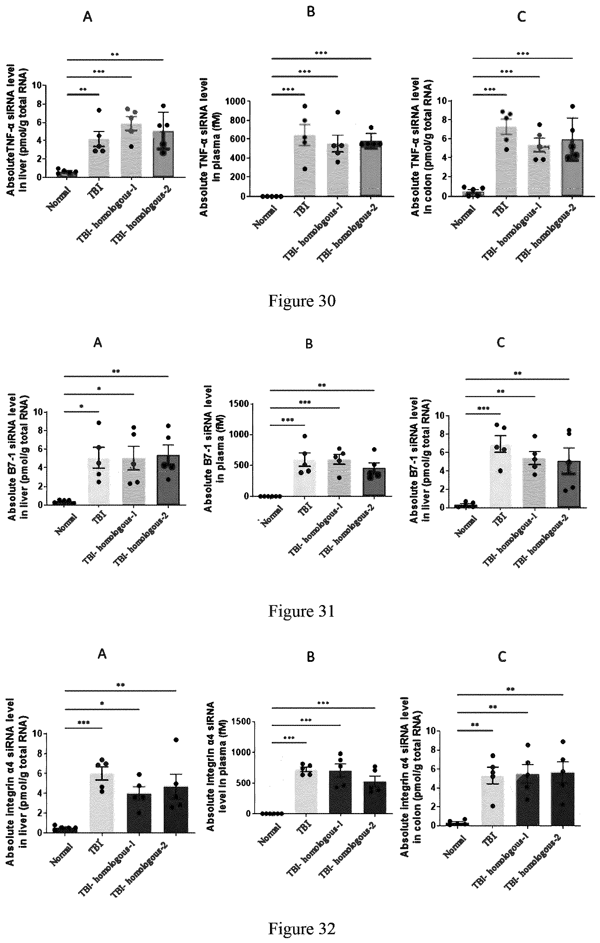

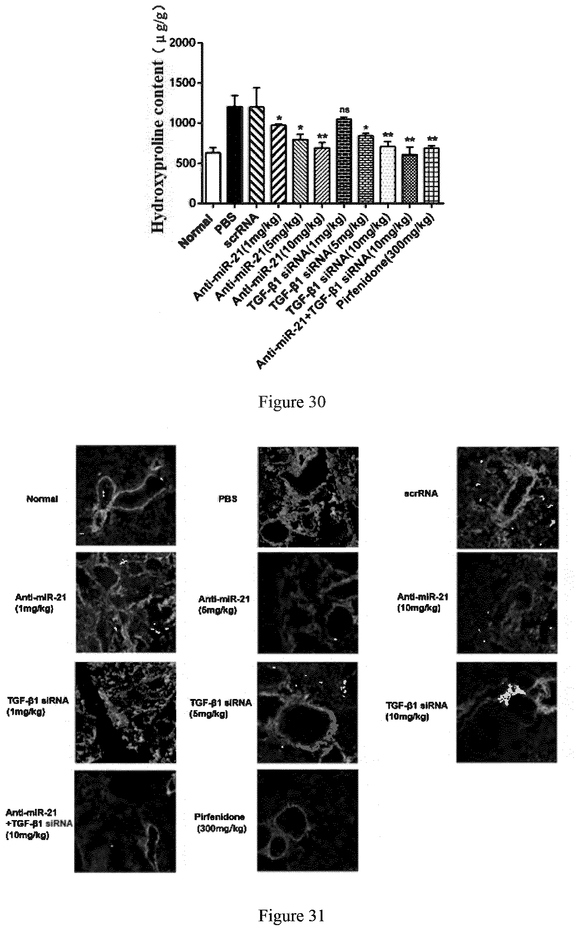

Figure 30 shows the fluorescence signal statistics after constructing 6 different RNAs into an adeno-associated viral vector respectively to treat lung cancer provided by an embodiment of the present application; where A shows the fluorescence signal statistics after constructing siR^{E} into an adeno-associated viral vector to treat lung cancer, B shows the fluorescence signal statistics after constructing siR^{T} into an adeno-associated viral vector to treat lung cancer, C shows the fluorescence signal statistics after constructing miR-7 into an adeno-associated viral vector to treat lung cancer, D shows the fluorescence signal statistics after constructing shR^{E} into an adeno-associated viral vector to treat lung cancer, E shows the fluorescence signal statistics after constructing shR^{T} into an adeno-associated viral vector to treat lung cancer, and F shows the fluorescence signal statistics after constructing miR-133b into an adeno-associated viral vector to treat lung cancer.
Figure 31 shows the fluorescence signal statistics after constructing four RNA fragments comprising any two RNA sequences of six different RNAs into an adeno-associated viral vector respectively to treat lung cancer provided by an embodiment of the present application; where A shows the fluorescence signal statistics after constructing siR^{E}+ shR^{T} into an adeno-associated viral vector to treat lung cancer, B shows the fluorescence signal statistics after constructing siR^{T}+miR-7 into an adeno-associated viral vector to treat lung cancer, C shows the fluorescence signal statistics after constructing shR^{E}+miR-133b into an adeno-associated viral vector to treat lung cancer, and D shows the fluorescence signal statistics after constructing shR^{T}+miR-133b into an adeno-associated viral vector to treat lung cancer.

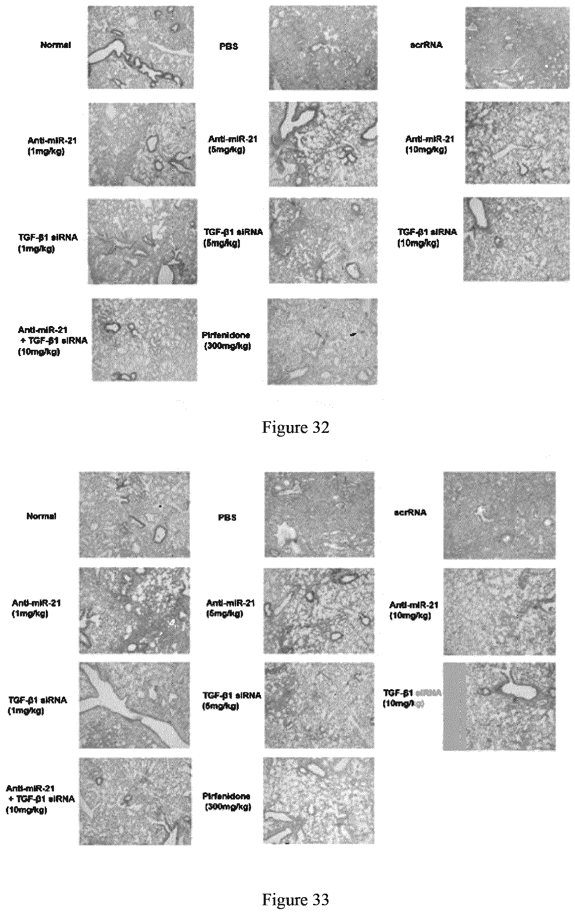

Figure 32 shows the fluorescence signal statistics after constructing three RNA fragments comprising any three RNA sequences of six different RNAs into an adeno-associated viral vector respectively to treat lung cancer provided by an embodiment of the present application; where A shows the fluorescence signal statistics after constructing siR^{E}+shR^{T}+miR-7 into an adeno-associated viral vector to treat lung cancer, B shows the fluorescence signal statistics after constructing siR^{T}+ shR^{E} +miR-7 into an adeno-associated viral vector to treat lung cancer, and C shows the fluorescence signal statistics after constructing shR^{E}+siR^{T}+miR-133b into an adeno-associated viral vector to treat lung cancer.

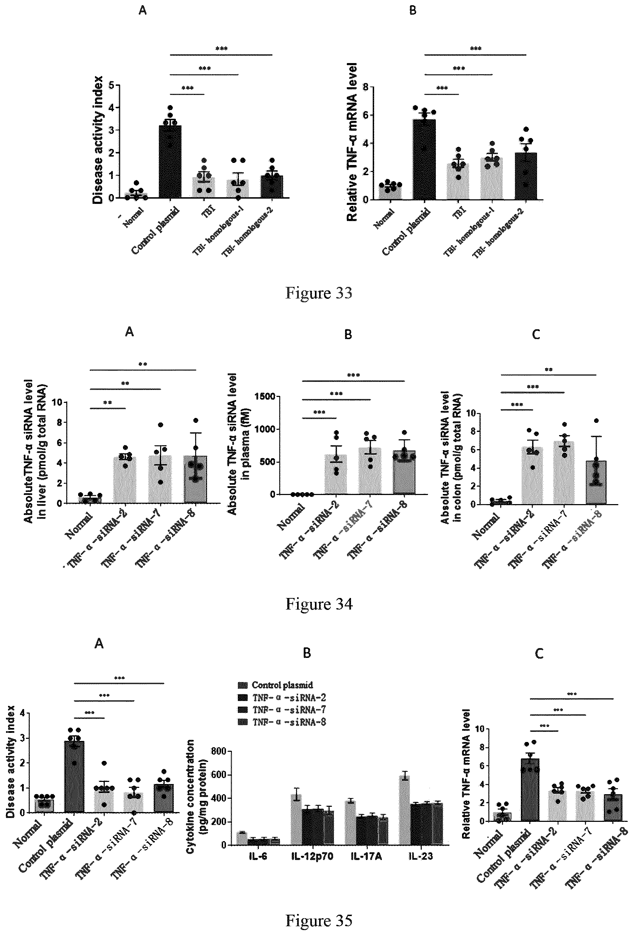

Figure 33 shows the enrichment results of siRNA and the expression levels of EGFR protein and mRNA in the liver, lung, plasma, and exosomes after intravenous injection provided by an embodiment of the present application; where A shows the enrichment results of AAV-siR^{E} and AAV-GE11-siR^{E} in the liver and lung, B shows the enrichment results of AAV-siR^{E} and AAV-GE11-siR^{E} in plasma and exosomes, C shows the protein levels of EGFR with AAV-siR^{E} and AAV GE11-siR^{E}, and D shows the mRNA levels of EGFR with AAV-siR^{E} and AAV-GE11-siR^{E}.

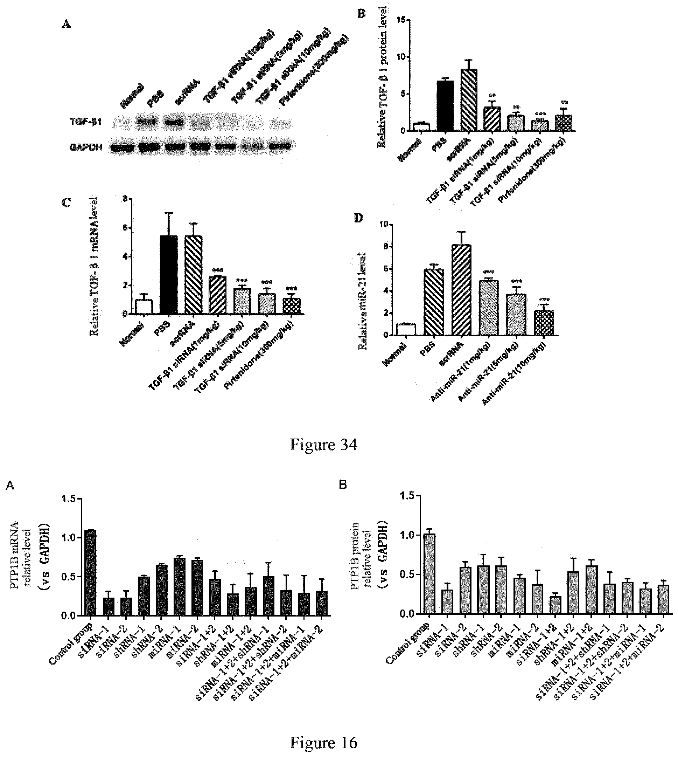

Figure 34 shows the enrichment and therapeutic effects in the lung after constructing two more than 80% homologous 5' flanking sequences into an AAV vector respectively provided by an embodiment of the present application; where A shows the enrichment results in the lung by the EGFR siRNA level, B shows the therapeutic effect of one sequence, and C shows the therapeutic effect of another sequence.
Figure 35 shows the enrichment and therapeutic effects in the lung after constructing more than 80% homologous loop sequences into an AAV vector respectively provided by an embodiment of the present application; where A shows the enrichment results in the lung by the EGFR siRNA level, B shows the therapeutic effect of one sequence, and C shows the therapeutic effect of another sequence.

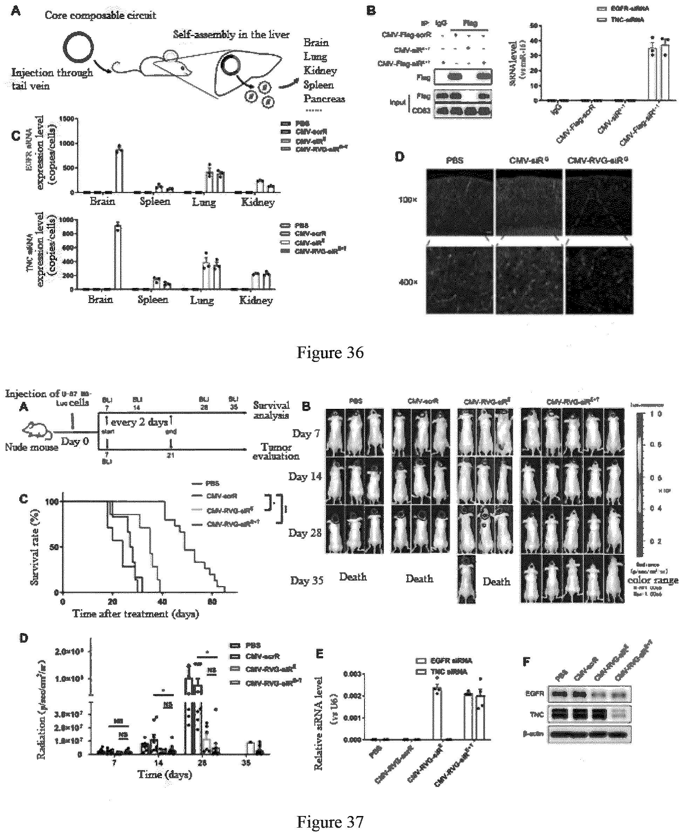

Figure 36 shows the enrichment and therapeutic effects in the lung after constructing more than 80% homologous 3' flanking sequences into an AAV vector respectively provided by an embodiment of the present application; where A shows the enrichment results in the lung by the EGFR siRNA level, B shows the therapeutic effect of one sequence, and C shows the therapeutic effect of another sequence.
Figure 37 shows the detection results of EGFR siRNA levels in the lung after 9 h of intravenous injection of an AAV vector constructed with sequence 4 and two sequences 4-1 and 4-2 with greater than 80% homology to sequence 4 respectively provided by an embodiment of the present application; where A shows the detection results of sequence 4, B shows the detection results of sequence 4-1, and C shows the detection results of sequence 4-2.

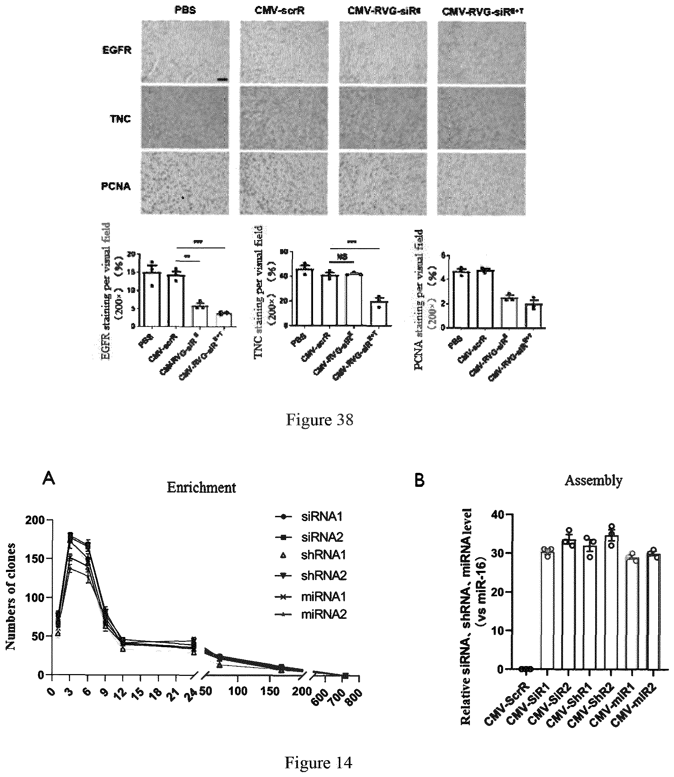

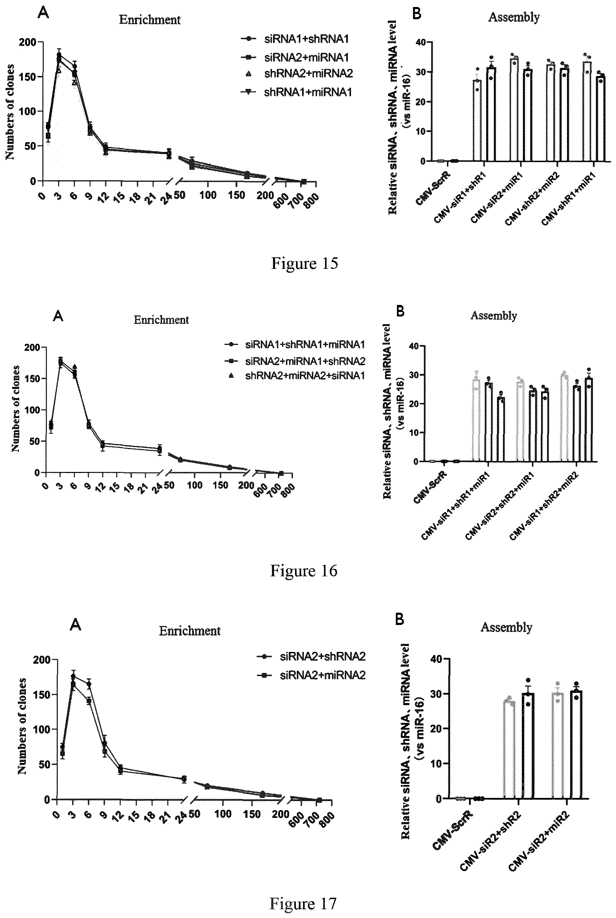

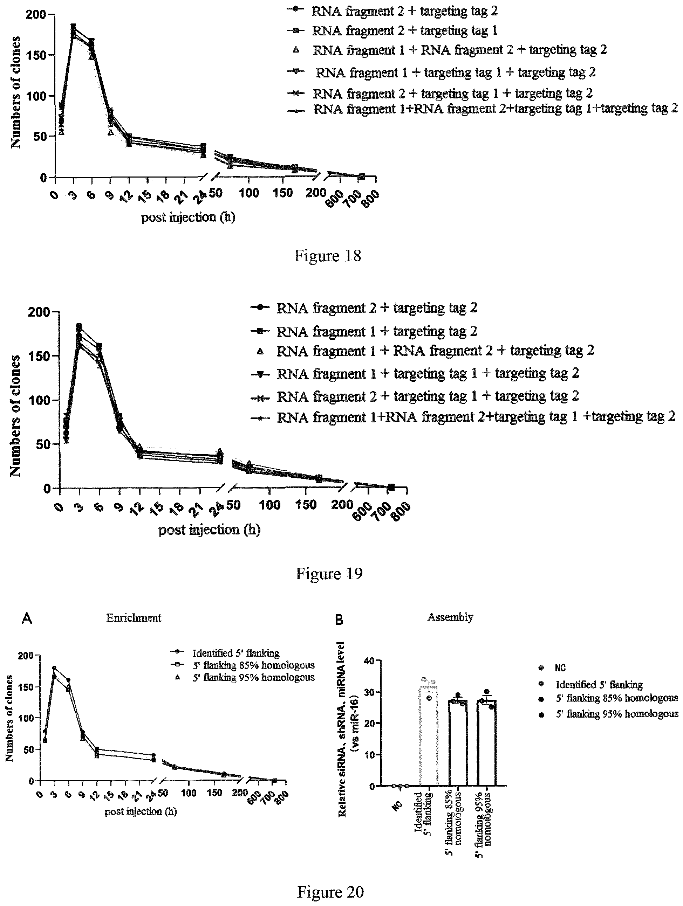

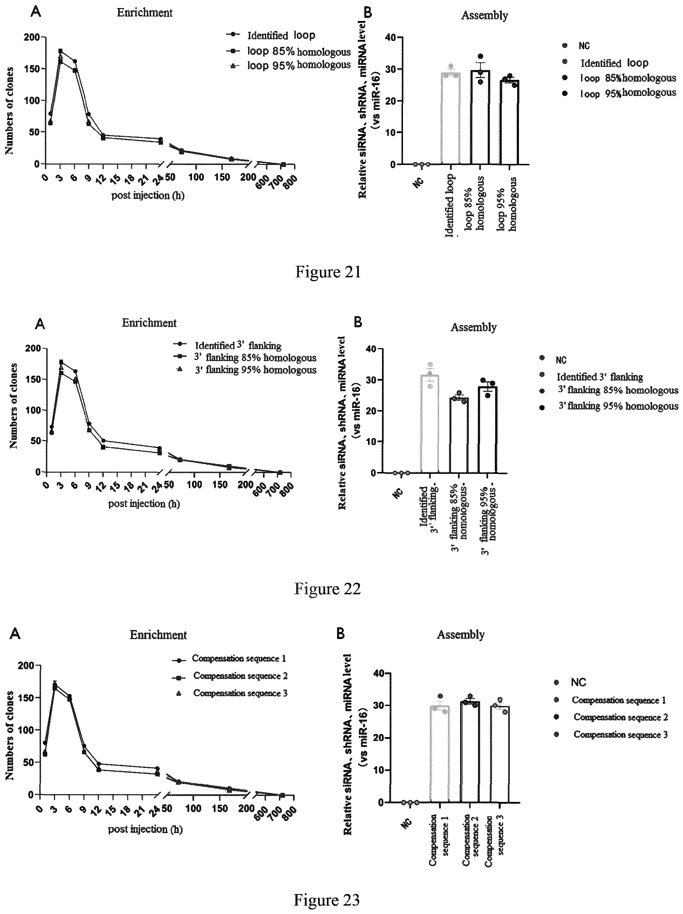

Figure 38 shows the detection of EGFR expression levels after intravenous injection of a gene circuit comprising three RNA sequences of different lengths respectively provided by an embodiment of the present application; where A shows the protein level of EGFR, and B shows the mRNA level of EGFR.

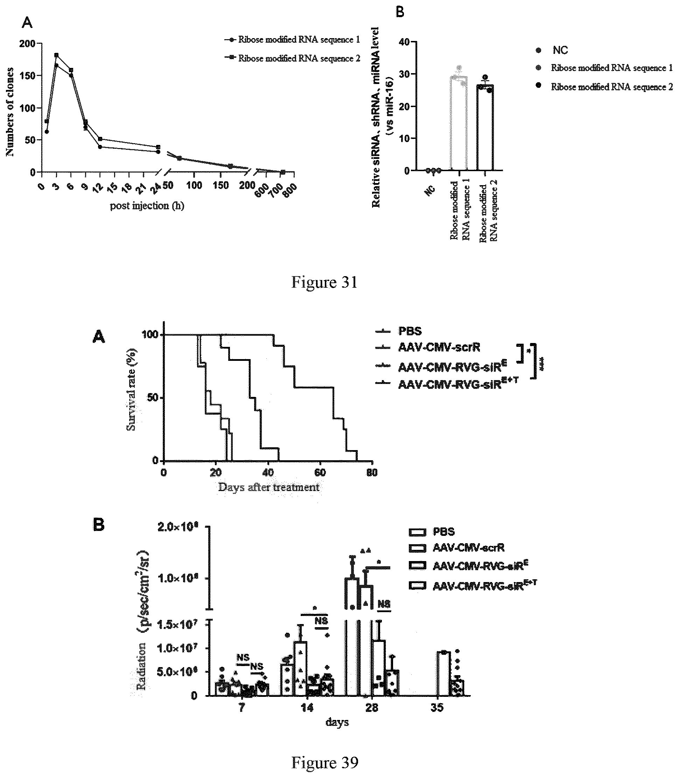

Figure 39 shows the comparison of kidney cancer tumors in mice provided by an embodiment of the present application.

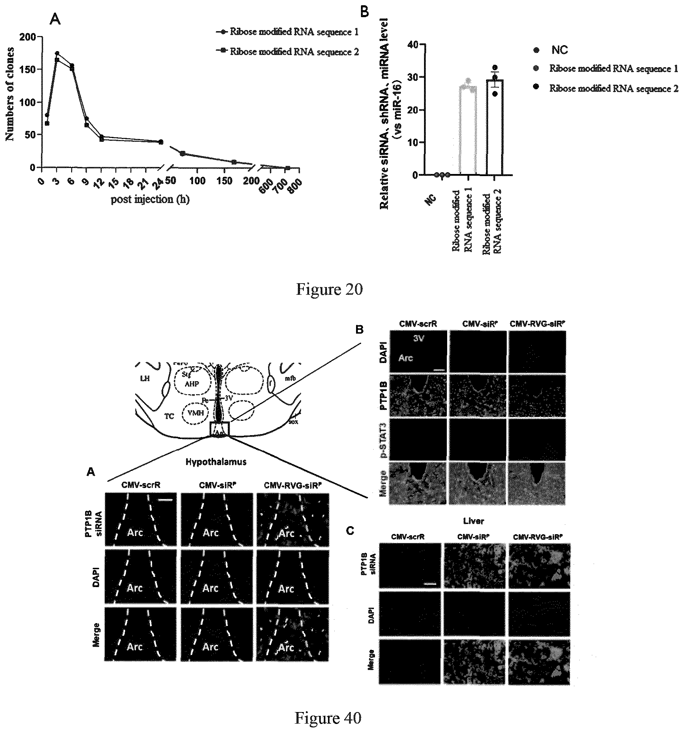

Figure 40 shows the comparison of kidney cancer tumor development in mice provided by an embodiment of the present application.

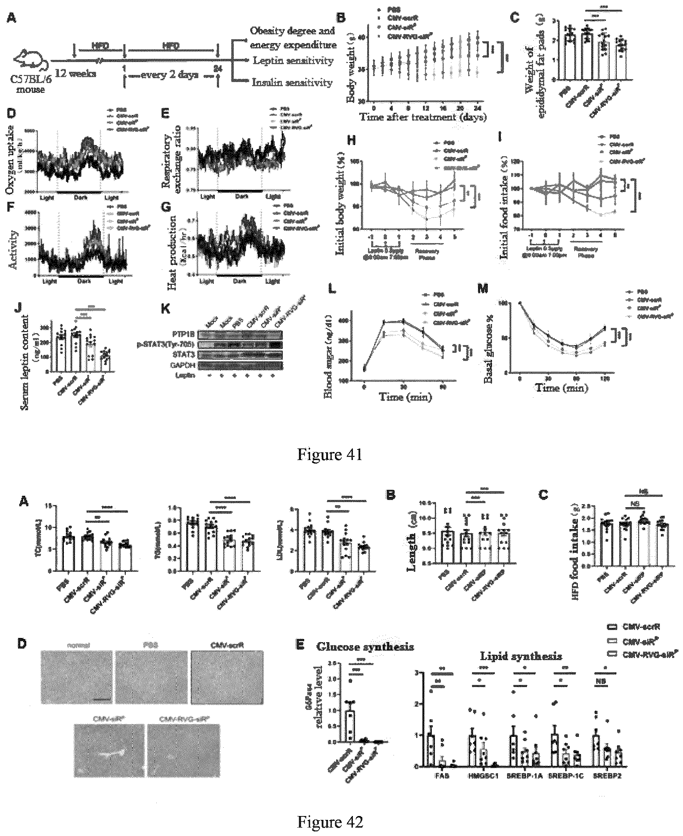

Figure 41 shows the in vivo (plasma, exosomes) enrichment, self-assembly and therapeutic effects (shown by siRNA levels) on colorectal cancer, pancreatic cancer, glioma, lung cancer, and kidney cancer of an adeno-associated virus (AAV) vector containing an RNA fragment provided by an embodiment of the present application.
Figure 42 shows the in vivo enrichment, self-assembly and therapeutic effects on lung cancer, kidney cancer, pancreatic cancer, obesity and glioma of an adeno-associated virus (AAV) vector containing siR^{E}, siR v, siR^{K} and siR^{E+T} respectively provided by an embodiment of the present application; where A-E show the fluorescence signal detection results of lung cancer, kidney cancer, pancreatic cancer, obesity and glioma, respectively.

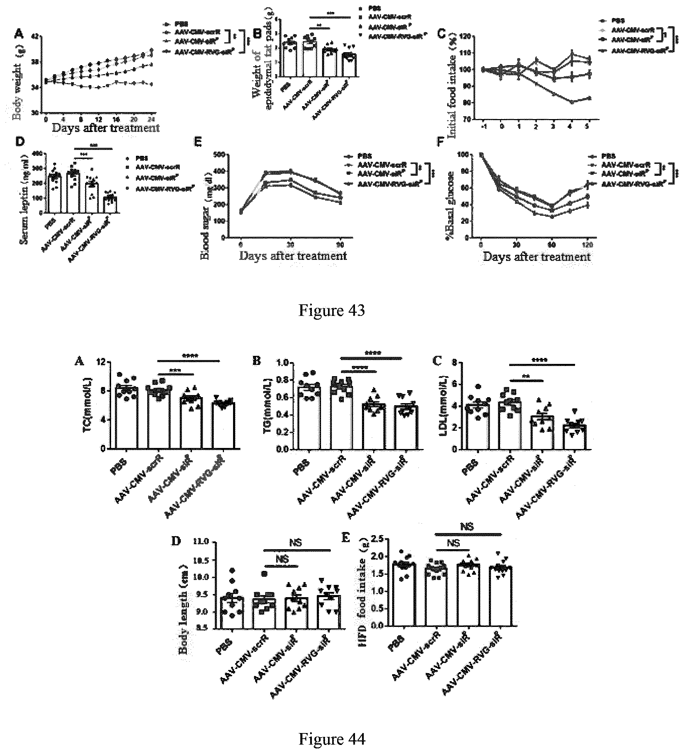

Figure 43 shows the in vivo enrichment, self-assembly and therapeutic effects on lung cancer, kidney cancer, pancreatic cancer, obesity and glioma of a lentivirus (LV) vector containing siR^{E}, siR v, siR^{K} and siR^{E+T} respectively provided by an embodiment of the present application; where A-E show the fluorescence signal detection results of lung cancer, kidney cancer, pancreatic cancer, obesity and glioma, respectively.
Figure 44 shows the in vivo enrichment, self-assembly and therapeutic effects on lung cancer of a viral vector delivery system carrying different RNA fragments provided by an embodiment of the present application; where A shows the tumor volume effect with an RNA sequence acting alone, and B shows the tumor volume effect with an RNA sequence composed of 2-3 RNA fragments.

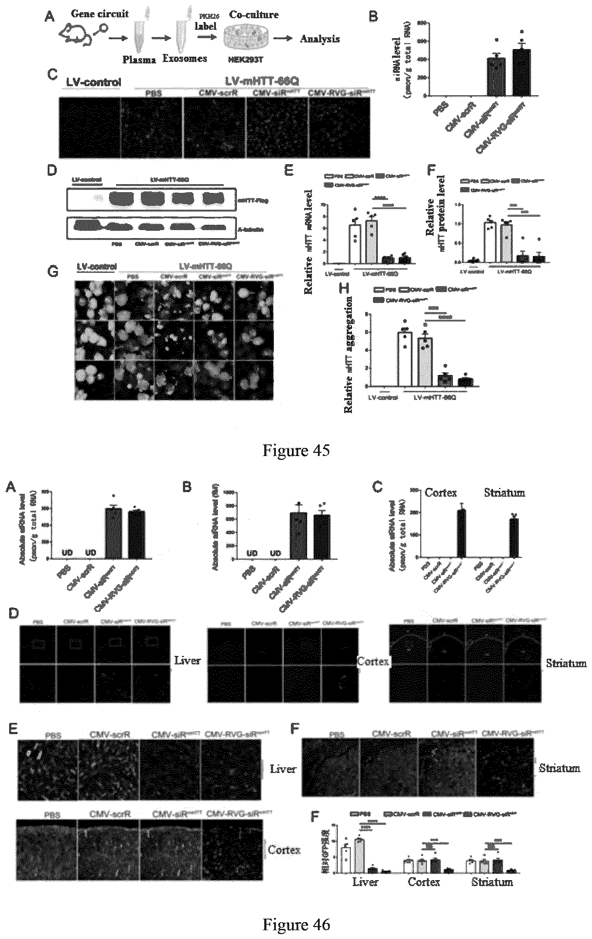

Figure 45 shows the in vivo enrichment, self-assembly and therapeutic effects on kidney cancer of a viral vector delivery system carrying different RNA fragments provided by an embodiment of the present application; where A shows the tumor volume effect with an RNA sequence acting alone, and B shows the tumor volume effect with an RNA sequence composed of 2-3 RNA fragments.
Figure 46 shows the in vivo enrichment, self-assembly and therapeutic effects on colorectal cancer of a viral vector delivery system carrying different RNA fragments provided by an embodiment of the present application; where A shows the tumor volume effect with an RNA sequence acting alone, and B shows the tumor volume effect with an RNA sequence composed of 2-3 RNA fragments.

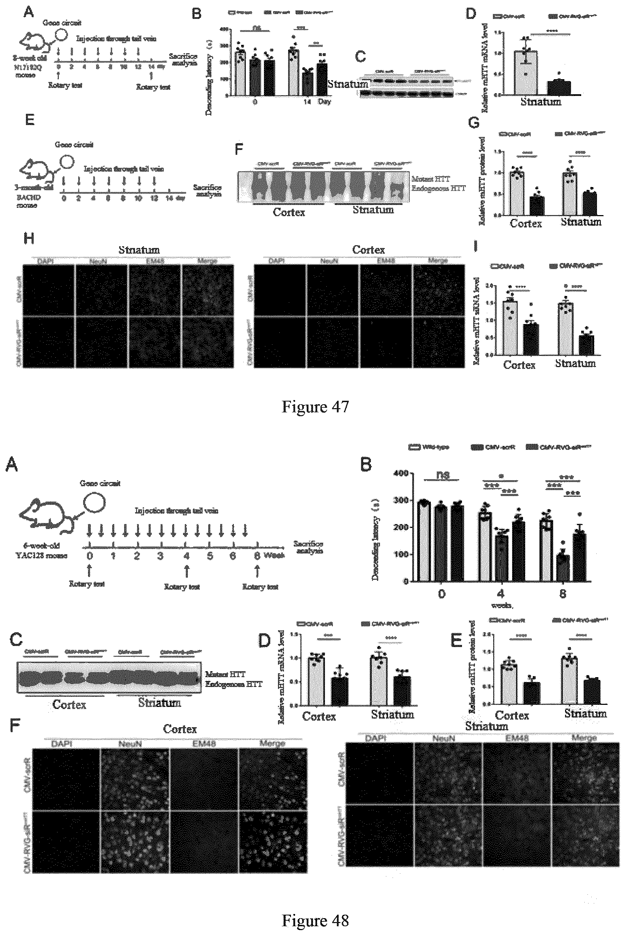

Figure 47 shows the in vivo enrichment, self-assembly and therapeutic effects on pancreatic cancer of a viral vector delivery system carrying different RNA fragments provided by an embodiment of the present application; where A shows the tumor volume effect with an RNA sequence acting alone, and B shows the tumor volume effect with an RNA sequence composed of 2-3 RNA fragments.
Figure 48 shows the in vivo enrichment, self-assembly and therapeutic effects on glioma of a viral vector delivery system carrying different RNA fragments provided by an embodiment of the present application; where A shows the tumor volume effect with an RNA sequence acting alone, and B shows the tumor volume effect with an RNA sequence composed of 2-3 RNA fragments.
Figure 49 shows the in vivo enrichment, self-assembly and therapeutic effects on cancers of an adenoviral vector delivery system comprising 1-2 RNA fragments and 1-2 targeting tags; where A shows the therapeutic effect on pancreatic cancer of the delivery system with AAV as the carrier carrying the circuit siR^{K} or PTP-siR^{K}, B shows the therapeutic effect on glioma of the delivery system with AAV as the carrier carrying the circuit siR^{E+T} or RVG-siR^{E+T}.

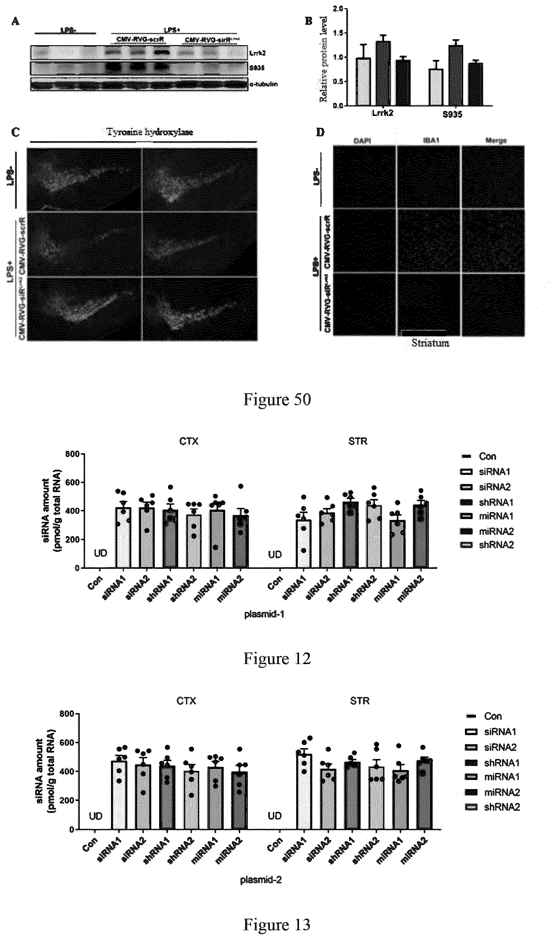

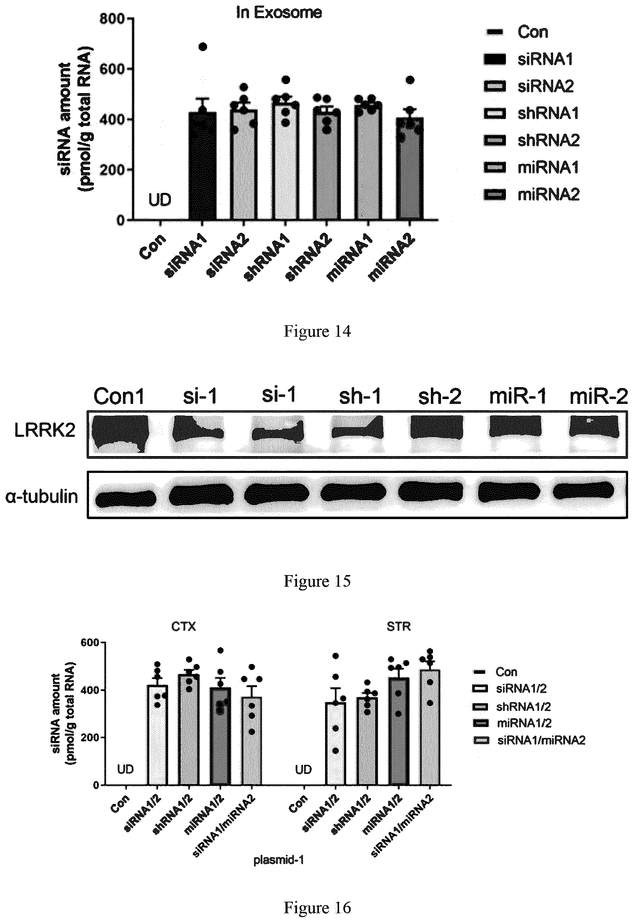

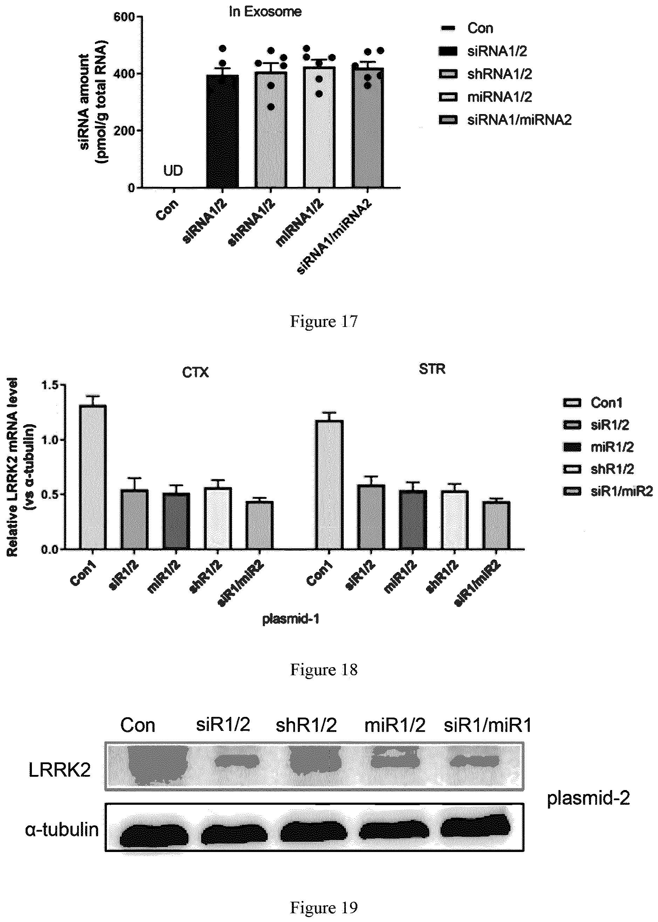

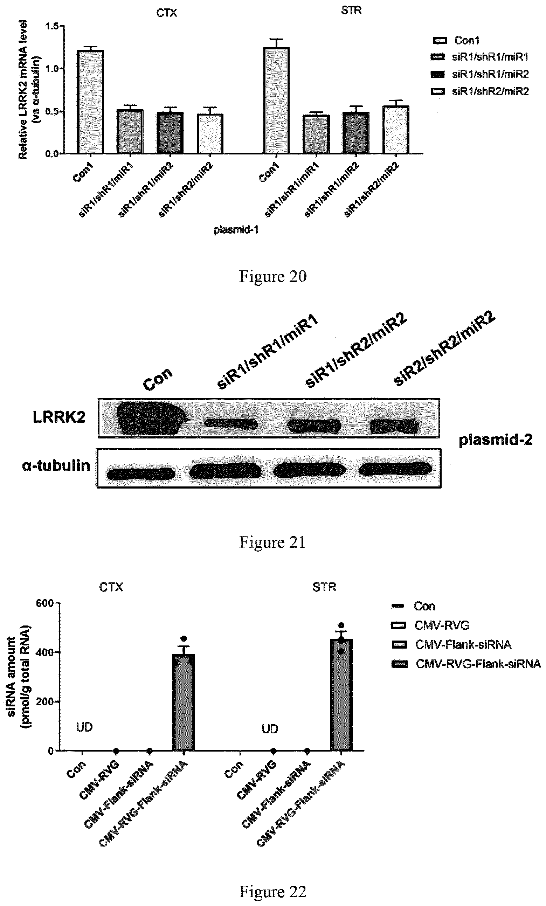

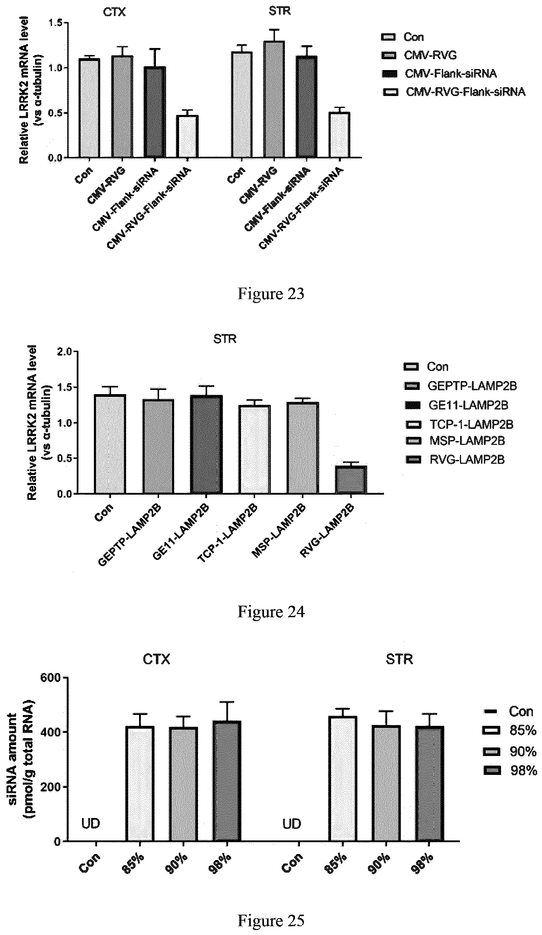

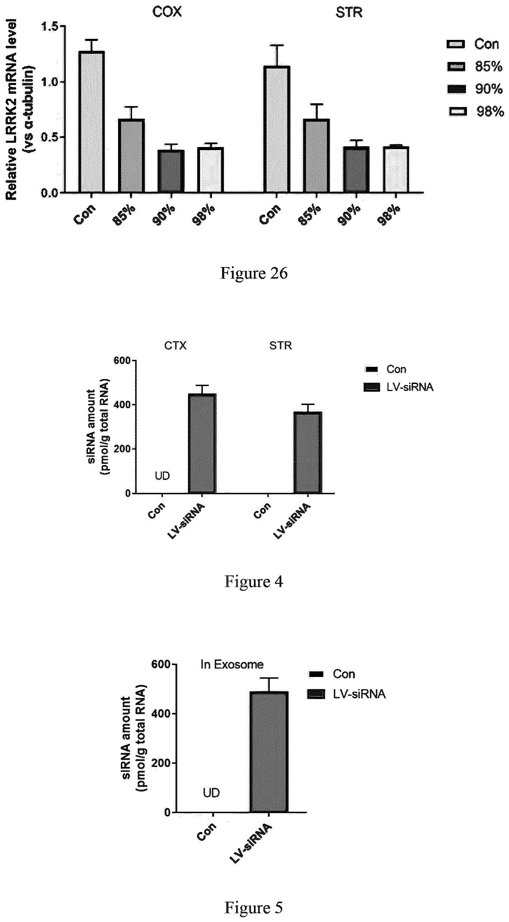

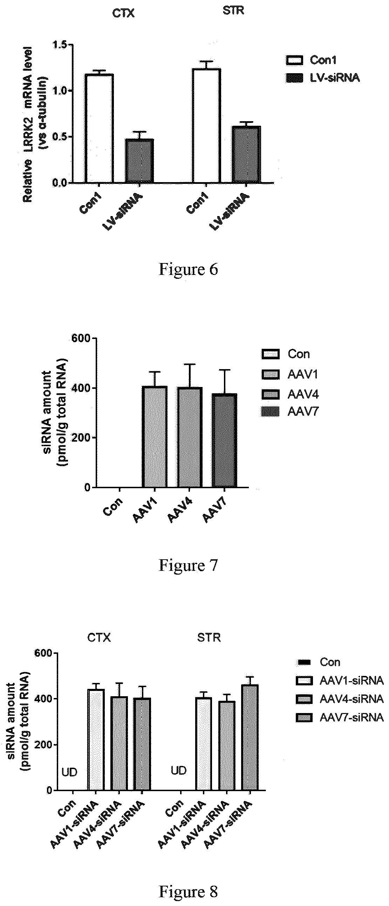

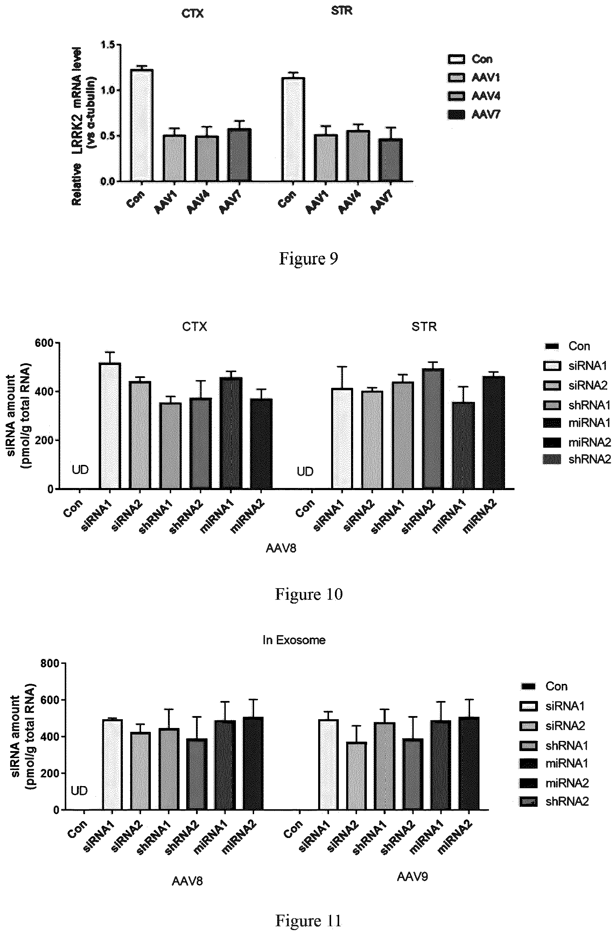

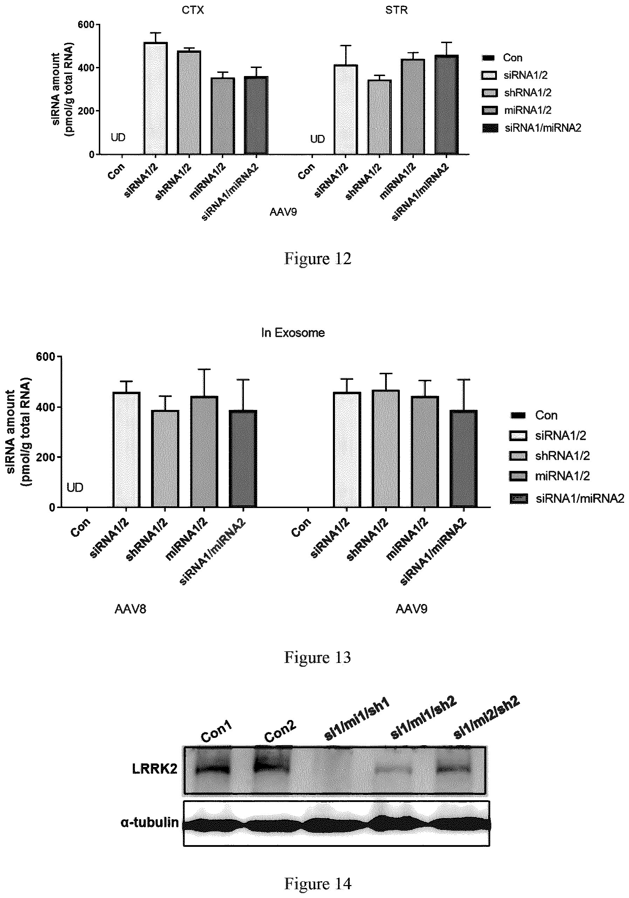

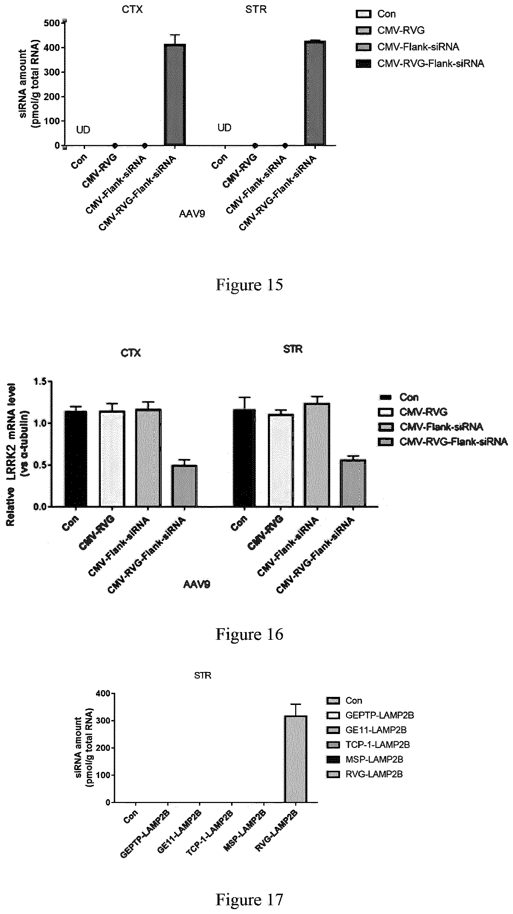

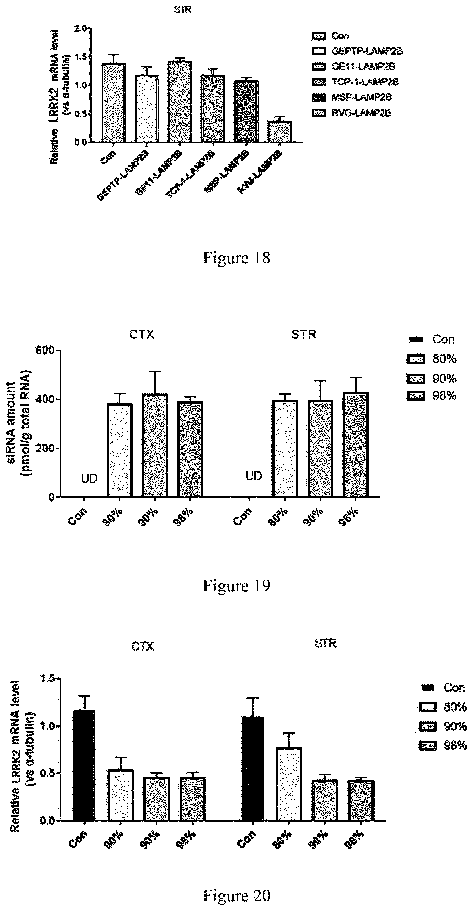

Figure 50 shows the in vivo enrichment, self-assembly and therapeutic effects on lung cancer, kidney cancer, pancreatic cancer and glioma of an adenoviral vector carrying different RNA fragments with 5' flanking sequence/loop sequence/3' flanking sequence provided by an embodiment of the present application; where A shows the effect on lung cancer tumor volume of siR^{E} sequence linking two different 5' flanking sequences/loop sequences/3' flanking sequences with or without RVG, B shows the effect on kidney cancer tumor volume of siR^{V} sequence linking two different 5' flanking sequences/loop sequences/3' flanking sequences with or without RVG, C shows the effect on pancreatic cancer tumor volume of siR^{P} sequence linking two different 5' flanking sequences/loop sequences/3' flanking sequences with or without RVG, and D shows the effect on glioma tumor volume of siR^{E+T} sequence linking two different 5' flanking sequences/loop sequences/3' flanking sequences with or without RVG.

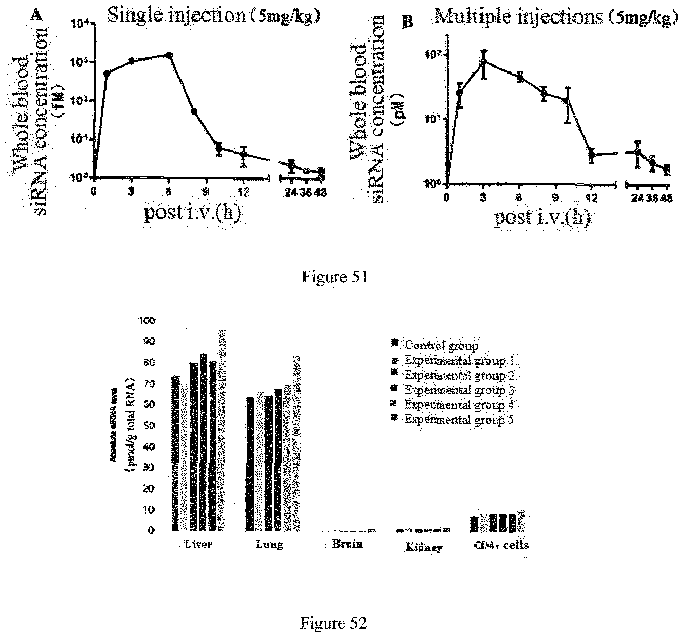

Figure 51 shows the in vivo enrichment, self-assembly and therapeutic effects on colorectal cancer of an adenoviral vector carrying different RNA fragments with 5' flanking sequence/loop sequence/3' flanking sequence provided by an embodiment of the present application; where the figure shows the effect on colorectal cancer tumor volume of siR^{V} sequence linking two different 5' flanking sequences/loop sequences/3' flanking sequences with or without RVG.
Figure 52 shows the enrichment, self-assembly and therapeutic effects on cancers of a delivery system containing a linker of sequence 4 and sequence 4-1 and sequence 4-2 with more than 80% homology with sequence 4 respectively provided by an embodiment of the present application, where the figure shows the EGFR siRNA levels with sequence 4/4-1/4-2 linking siR^{E} and siR^{T} respectively.

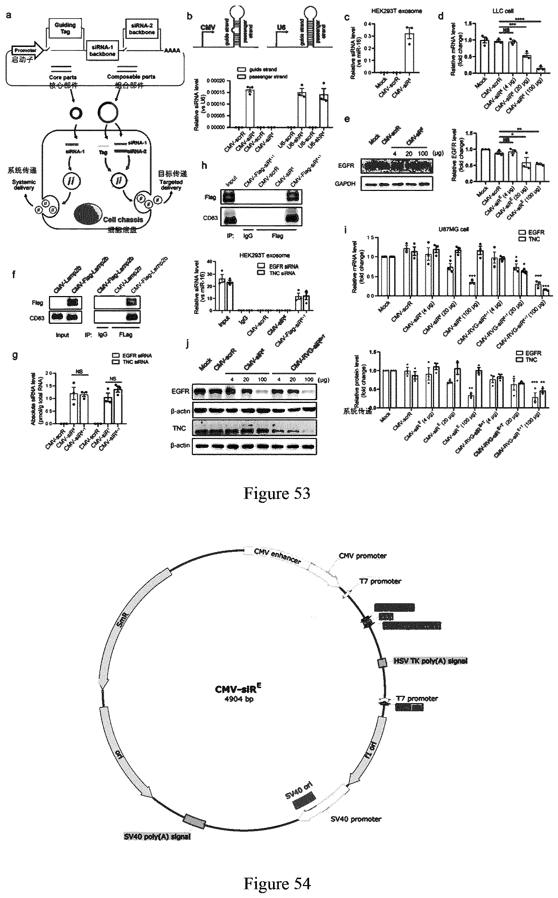

Figure 53 shows the enrichment, self-assembly and therapeutic effects on cancers of a delivery system containing RNA sequences of three lengths of 18, 20, and 22 respectively provided by an embodiment of the present application; where A shows the EGFR protein levels detected after injection of the delivery system constructed by RNA sequences of three lengths, B shows the EGFR mRNA levels detected after injection of the delivery system constructed by RNA sequences of three lengths.
Figure 54 shows the comparison of the development of colitis in mice provided by an embodiment of the present application.

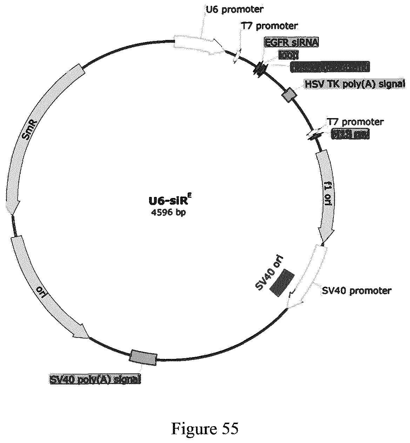

Figure 55 shows the comparison of HE staining of colon in mice provided by an embodiment of the present application.
Figure 56 shows the comparison of the development of colitis in mice provided by an embodiment of the present application.
Figure 57 shows the comparison of HE staining of colon in mice provided by an embodiment of the present application.
Figure 58 shows the comparison of the treatment of colitis in mice and RNA expression levels provided by an embodiment of the present application.
Figure 59 shows the comparison of cytokine concentration and HE staining of colon in mice provided by an embodiment of the present application.
Figure 60 shows the comparison of the treatment of colitis in mice provided by an embodiment of the present application.
Figure 61 shows the comparison of disease activity index and various siRNA levels in mice provided by an embodiment of the present application.
Figure 62 shows the comparison of various siRNA and mRNA levels in mice provided by an embodiment of the present application.
Figure 63 shows the comparison of HE staining of colon in mice provided by an embodiment of the present application.
Figure 64 shows the results of in vivo enrichment of TNF-α siRNA in mice injected with TNF-α siRNA-lentivirus provided by an embodiment of the present application; where A shows enrichment result in the liver, B shows enrichment result in plasma, and C shows enrichment result in the colon.
Figure 65 shows the results of TNF-α siRNA detected in plasma exosomes of mice injected with TNF-α siRNA-lentivirus provided by an embodiment of the present application, indicating the spontaneous formation of a complex.
Figure 66 shows the specific therapeutic effects in mice injected with TNF-α siRNA-lentivirus provided by an embodiment of the present application; where A shows the disease index score, B shows the detection results of inflammatory factors, and C shows the mRNA detection results of target gene.
Figure 67 shows the in vivo enrichment results in mice injected with a viral vector containing 6 different RNAs, miR-19a (target gene TNF-α), miR-124-3p (target gene TNF-α), B7-siRNA-1, B7-siRNA-2, integrin α4 shRNA-1, and integrin α4 shRNA-2, respectively, provided by an embodiment of the present application; where A shows the small RNA expression results detected in the liver, B shows the small RNA expression results detected in plasma, and C shows the small RNA expression results detected in the colon.
Figure 68 shows the specific therapeutic effects in mice injected with a viral vector containing 6 different RNAs, miR-19a (target gene TNF-α), miR-124-3p (target gene TNF-α), B7-siRNA-1, B7-siRNA-2, integrin α4 shRNA-1, and integrin α4 shRNA-2, respectively, provided by an embodiment of the present application; where A shows the disease index score, and B shows the detection results of inflammatory factors.
Figure 69 shows the in vivo enrichment results in mice injected with a viral vector containing four different RNA fragments composed of any two RNA sequences, specifically miR-19a (target gene TNF-α)+B7-siRNA-1, miR-124-3p (target gene TNF-α)+B7-siRNA-2, B7-siRNA-1+integrin α4 shRNA-1, B7-siRNA-2+integrin α4 shRNA-2, respectively, provided by an embodiment of the present application; where A shows the small RNA (sequence 1) expression results detected in the liver, B shows the small RNA (sequence 1) expression results detected in plasma, and C shows the small RNA (sequence 1) expression results detected in the colon.
Figure 70 shows the in vivo enrichment results in mice injected with a viral vector containing four different RNA fragments composed of any two RNA sequences, specifically miR-19a (target gene TNF-α)+B7-siRNA-1, miR-124-3p (target gene TNF-α)+B7-siRNA-2, B7-siRNA-1+integrin α4 shRNA-1, B7-siRNA-2+integrin α4 shRNA-2, respectively, provided by another embodiment of the present application; where A shows the small RNA (sequence 2) expression results detected in the liver, B shows the small RNA (sequence 2) expression results detected in plasma, and C shows the small RNA (sequence 2) expression results detected in the colon.
Figure 71 shows the specific therapeutic effects in mice injected with a viral vector containing four different RNA fragments composed of any two RNA sequences, specifically miR-19a (target gene TNF-α)+B7-siRNA-1, miR-124-3p (target gene TNF-α)+B7-siRNA-2, B7-siRNA-1+integrin α4 shRNA-1, B7-siRNA-2+integrin α4 shRNA-2, respectively, provided by an embodiment of the present application; where A shows the disease index score, and B shows the detection results of inflammatory factors.
Figure 72 shows the in vivo enrichment results in mice injected with a viral vector containing three different RNA fragments composed of any three RNA sequences, specifically miR-19a (target gene TNF-α)+B7-siRNA-1+integrin α4 shRNA-1, miR-124-3p (target gene TNF-α)+B7-siRNA-2+integrin α4 shRNA-2, and miR-19a (target gene TNF-α)+B7-siRNA-1+integrin α4 shRNA-2, respectively, provided by an embodiment of the present application; where A shows the small RNA (sequence 1) expression results detected in the liver, B shows the small RNA (sequence 1) expression results detected in plasma, and C shows the small RNA (sequence 1) expression results detected in the colon.
Figure 73 shows the in vivo enrichment results in mice injected with a viral vector containing three different RNA fragments composed of any three RNA sequences, specifically miR-19a (target gene TNF-α)+B7-siRNA-1+integrin α4 shRNA-1, miR-124-3p (target gene TNF-α)+B7-siRNA-2+integrin α4 shRNA-2, and miR-19a (target gene TNF-α)+B7-siRNA-1+integrin α4 shRNA-2, respectively, provided by an embodiment of the present application; where A shows the small RNA (sequence 2) expression results detected in the liver, B shows the small RNA (sequence 2) expression results detected in plasma, and C shows the small RNA (sequence 2) expression results detected in the colon.
Figure 74 shows the in vivo enrichment results in mice injected with a viral vector containing three different RNA fragments composed of any three RNA sequences, specifically miR-19a (target gene TNF-α)+B7-siRNA-1+integrin α4 shRNA-1, miR-124-3p (target gene TNF-α)+B7-siRNA-2+integrin α4 shRNA-2, and miR-19a (target gene TNF-α)+B7-siRNA-1+integrin α4 shRNA-2, respectively, provided by an embodiment of the present application; where A shows the small RNA (sequence 3) expression results detected in the liver, B shows the small RNA (sequence 3) expression results detected in plasma, and C shows the small RNA (sequence 3) expression results detected in the colon.
Figure 75 shows the specific therapeutic effects in mice injected with a viral vector containing three different RNA fragments composed of any three RNA sequences, specifically miR-19a (target gene TNF-α)+B7-siRNA-1+integrin α4 shRNA-1, miR-124-3p (target gene TNF-α)+B7-siRNA-2+integrin α4 shRNA-2, and miR-19a (target gene TNF-α)+B7-siRNA-1+integrin α4 shRNA-2, respectively, provided by an embodiment of the present application; where A shows the disease index score, and B shows the detection results of inflammatory factors.
Figure 76 shows the in vivo enrichment results in mice injected with a viral vector containing RNA sequences of different lengths, specifically TNF-α-siRNA-1 (siRNA length 18 bp), TNF-α-siRNA-2 (siRNA length 20 bp), and TNF-α-siRNA-3 (siRNA length 22 bp), respectively, provided by an embodiment of the present application; where A shows the siRNA expression results detected in the liver, B shows the siRNA expression results detected in plasma, and C shows the siRNA expression results detected in the colon.
Figure 77 shows the specific therapeutic results in mice injected with a viral vector containing RNA sequences of different lengths, specifically TNF-α-siRNA-1 (siRNA length 18 bp), TNF-α-siRNA-2 (siRNA length 20 bp), and TNF-α-siRNA-3 (siRNA length 22 bp), respectively, provided by an embodiment of the present application; where A shows the disease index score, and B shows the detection results of inflammatory factors.
Figure 78 shows the in vivo enrichment results in mice injected with a viral vector containing three homologous TNF-α-siRNA sequences, specifically TNF-α-siRNA-4, TNF-α-siRNA-5, and TNF-α-siRNA-6, respectively, provided by an embodiment of the present application; where A shows the TNF-α-siRNA expression results detected in the liver, B shows the TNF-α-siRNA expression results detected in plasma, C shows the TNF-α-siRNA expression results detected in the colon, and D shows the TNF-α-siRNA expression results detected in plasma exosomes.
Figure 79 shows the in vivo enrichment results in mice injected with a viral vector containing three homologous B7-siRNA sequences, specifically B7-siRNA-1, B7-siRNA-2, and B7-siRNA-3, respectively, provided by another embodiment of the present application; where A shows the B7-siRNA expression results detected in the liver, B shows the B7-siRNA expression results detected in plasma, C shows the B7-siRNA expression results detected in the colon, and D shows the B7-siRNA expression results detected in plasma exosomes.
Figure 80 shows the in vivo enrichment results in mice injected with a viral vector containing three homologous integrin α4 siRNA sequences, specifically integrin α4 siRNA-1, integrin α4 siRNA-2, and integrin α4 siRNA-3, respectively, provided by another embodiment of the present application; where A shows the integrin α4 siRNA expression results detected in the liver, B shows the integrin α4 siRNA expression results detected in plasma, C shows the integrin α4 siRNA expression results detected in the colon, and D shows the integrin α4 siRNA expression results detected in plasma exosomes.
Figure 81 shows the specific therapeutic results in mice injected with a viral vector containing nine homologous siRNA sequences, specifically TNF-α-siRNA-4, TNF-α-siRNA-5, TNF-α-siRNA-6, B7-siRNA-1, B7-siRNA-2, B7-siRNA-3, integrin α4 siRNA-1, integrin α4 siRNA-2, and integrin α4 siRNA-3, respectively, provided by another embodiment of the present application; where A shows the disease index score, and B shows the detection results of inflammatory factors.
Figure 82 shows the in vivo enrichment results in mice injected with a viral vector containing an RNA fragment with different flanking sequences, loop sequences and reverse complementary sequences, specifically two identified sequences with more than 80% homology to an identified 5' flanking sequences, two identified sequences with more than 80% homology to an identified loop sequences, two identified sequences with more than 80% homology to an identified 3' flanking sequences, one reverse complement sequence of a normal sequence, and one reverse complement sequence of an identified sequence with more than 80% homology to an identified 5' flanking sequence, respectively, provided by an embodiment of the present application; where A shows the TNF-α-siRNA expression results detected in the liver, B shows the TNF-α-siRNA expression results detected in plasma, C shows the TNF-α-siRNA expression results detected in the colon, and D shows the TNF-α-siRNA expression results detected in plasma exosomes.
Figure 83 shows the specific therapeutic results in mice injected with a viral vector containing an RNA fragment with different flanking sequences, loop sequences and reverse complementary sequences, specifically two identified sequences with more than 80% homology to an identified 5' flanking sequences, two identified sequences with more than 80% homology to an identified loop sequences, two identified sequences with more than 80% homology to an identified 3' flanking sequences, one reverse complement sequence of a normal sequence, and one reverse complement sequence of an identified sequence with more than 80% homology to an identified 5' flanking sequence, respectively, provided by an embodiment of the present application; A shows the disease index score, B shows the detection results of inflammatory factors, and C shows the mRNA detection results of target gene.
Figure 84 shows the in vivo enrichment results in mice injected with an adenoviral vector carrying multiple circuits in which adjacent circuits are linked via sequence 1-sequence 2-sequence 3 with sequence 2 of 5 bases, 10 bases, 20 bases, 30 bases, 40 bases, 50 bases or 80 bases provided by an embodiment of the present application; where A shows the TNF-α-siRNA expression results detected in the liver, B shows the TNF-α-siRNA expression results detected in plasma, and C shows the TNF-α-siRNA expression results detected in the colon.
Figure 85 shows the in vivo enrichment results in mice injected with an adenoviral vector carrying multiple circuits in which adjacent circuits are linked via sequence 1-sequence 2-sequence 3 with sequence 2 of 5 bases, 10 bases, 20 bases, 30 bases, 40 bases, 50 bases or 80 bases provided by another embodiment of the present application; where A shows the B7-1-siRNA expression results detected in the liver, B shows the B7-1-siRNA expression results detected in plasma, and C shows the B7-1-siRNA expression results detected in the colon.
Figure 86 shows the in vivo enrichment results in mice injected with an adenoviral vector carrying multiple circuits in which adjacent circuits are linked via sequence 1-sequence 2-sequence 3 with sequence 2 of 5 bases, 10 bases, 20 bases, 30 bases, 40 bases, 50 bases or 80 bases provided by another embodiment of the present application; where A shows the integrin a4-siRNA expression results detected in the liver, B shows the integrin a4-siRNA expression results detected in plasma, and C shows the integrin a4-siRNA expression results detected in the colon.
Figure 87 shows the in vivo enrichment results in mice injected with an adenoviral vector carrying multiple circuits with a linker of sequence 4 and two sequences with more than 80% homology to sequence 4 respectively provided by an embodiment of the present application; where A shows the TNF-α-siRNA expression results detected in the liver, B shows the TNF-α-siRNA expression results detected in plasma, and C shows the TNF-α-siRNA expression results detected in the colon.
Figure 88 shows the in vivo enrichment results in mice injected with an adenoviral vector carrying multiple circuits with a linker of sequence 4 and two sequences with more than 80% homology to sequence 4 respectively provided by another embodiment of the present application; where A shows the B7-1-siRNA expression results detected in the liver, B shows the B7-1-siRNA expression results detected in plasma, and C shows the B7-1-siRNA expression results detected in the colon.
Figure 89 shows the in vivo enrichment results in mice injected with an adenoviral vector carrying multiple circuits with a linker of sequence 4 and two sequences with more than 80% homology to sequence 4 respectively provided by another embodiment of the present application; where A shows the integrin a4-siRNA expression results detected in the liver, B shows the integrin a4-siRNA expression results detected in plasma, and C shows the integrin a4-siRNA expression results detected in the colon.
Figure 90 shows the specific therapeutic effects in mice injected with an adenoviral vector carrying multiple circuits with a linker of sequence 4 and two sequences with more than 80% homology to sequence 4 respectively provided by another embodiment of the present application; where A shows the disease index score, and B shows the mRNA detection results of target gene.
Figure 91 shows the enrichment results of TNF-α-siRNA loaded in mice injected with a viral vector with adenovirus-associated virus types 2, 7 and 8 as a carrier provided by an embodiment of the present application; where A shows the TNF-α-siRNA expression results detected in the liver, B shows the TNF-α-siRNA expression results detected in plasma, and C shows the TNF-α-siRNA expression results detected in the colon.
Figure 92 shows the specific therapeutic results after TNF-α-siRNA loaded is expressed in mice injected with a viral vector with adenovirus-associated virus types 2, 7 and 8 as a carrier provided by an embodiment of the present application; where A shows the disease index score, B shows the detection results of inflammatory factors, and C shows the mRNA detection results of target gene.
Figure 93 shows the comparison of hydroxyproline content in mice provided by an embodiment of the present application.
Figure 94 shows fluorescence staining images of mouse lung provided by an embodiment of the present application.
Figure 95 shows Masson's trichrome staining images of mouse lung provided by an embodiment of the present application.
Figure 96 shows HE staining images of mouse lung provided by an embodiment of the present application.
Figure 97 shows the comparison of some protein and mRNA levels in mice provided by an embodiment of the present application.
Figure 98 shows the therapeutic effects on pulmonary fibrosis of a plasmid delivery system containing an RNA fragment provided by an embodiment of the present application; where A shows the detection results of the relative levels of PTP1B mRNA after injection of a plasmid delivery system containing six RNA sequences, any two of the six RNA sequences, and any three of the six RNA sequences respectively, and B shows the detection results of the relative levels of PTP1B protein after injection of a plasmid delivery system containing six RNA sequences, any two of the six RNA sequences, and any three of the six RNA sequences respectively.
Figure 99 shows the metabolic distribution results after intravenous injection of CMV-siRNA-1+2 provided by an embodiment of the present application; where A shows the enrichment effect in the lung, and B shows the enrichment effect in the blood.
Figure 100 shows the metabolic distribution results after intravenous injection of CMV GE11-siRNA-1+2 and CMV-GE11-siRNA-1+CMV-GE11-siRNA-2 with targeting tag GE11 provided by an embodiment of the present application; where A and C show the enrichment effects of CMVGE11-siRNA-1+2 in the lung and plasma respectively, and B and D show the enrichment effects of CMVGE11-siRNA-1+ CMV-GE 11-siRNA-2 in the lung and plasma respectively.
Figure 101 shows the therapeutic effects on pulmonary fibrosis after intravenous injection of CMV-GE11-siRNA-1, CMV-GE11-siRNA-1+2 and CMV-GE11-siRNA-1+ CMV-GE11-siRNA-2 with targeting tag GE11 provided by an embodiment of the present application; where A and C show the protein and mRNA levels of TGFb1 with CMV-GE11-siRNA-1 and CMV-GE11-siRNA-1+2 respectively, and B and D show the protein and mRNA levels of TGFb1 with CMV GE11-siRNA-1 and CMVGE11-siRNA-1+ CMVGE11-siRNA-2 respectively.
Figure 102 shows the enrichment effects (represented as siRNA level) in blood after injection of a plasmid containing a sequence fragment of three different 5' flanking sequences, loop sequences and 3' flanking sequences respectively provided by an embodiment of the present application.
Figure 103 shows the enrichment effects (represented as siRNA level) in blood after injection of a plasmid delivery system containing a linker (sequence 2) with different numbers of bases provided by an embodiment of the present application.
Figure 104 shows the enrichment effects (represented as siRNA level) in blood after injection of a plasmid delivery system containing a linker (sequence 4) of more than 80% homologous sequences provided by an embodiment of the present application, where the abscissa sequence 4-1 is the basic sequence 4, and the sequences 4-2/4-3/4-4 are homologous sequences with more than 80% homology to sequence 4-1 (sequence 4).
Figure 105 shows the therapeutic effect on pulmonary fibrosis in the case of a plasmid delivery system containing RNA sequences of lengths of 18, 19, and 21 respectively provided by an embodiment of the present application; where A shows the mRNA level of TGFb1, and B shows the protein level of TGFb1.
Figure 106 shows the results of the hydroxyproline content detected in the case of a gene circuit comprising the antisense strand of miRNA-21 and five siRNAs of TGF-β1 gene provided by an embodiment of the present application.
Figure 107 shows the comparison of hydroxyproline content and mRNA levels in mice provided by an embodiment of the present application.
Figure 108 shows the detection results of the in vivo enrichment (represented by siRNA level), self-assembly and therapeutic effects on pulmonary fibrosis of a viral vector using adenovirus/lentivirus and loading an RNA fragment provided by an embodiment of the present application; where A shows the detection results of enrichment (siRNA-1) in the lung after injection of the delivery system, B shows the detection results of enrichment (siRNA-2) in the lung after injection of the delivery system, C shows the detection results of enrichment (siRNA-1) in the blood after injection of the delivery system, and D shows the detection results of enrichment (siRNA-2) in the blood after injection of the delivery system.
Figure 109 shows the detection results of the in vivo enrichment (represented by siRNA level), self-assembly and therapeutic effects on pulmonary fibrosis of a viral vector using adenovirus/lentivirus and loading an RNA fragment provided by another embodiment of the present application; where A shows the detection results of enrichment in the lung after injection of the delivery system without a targeting peptide (GE11), B shows the detection results of enrichment in the lung after injection of the delivery system with a targeting peptide (GE11), C shows the detection results of enrichment (siRNA-1) in the blood after injection of the delivery system without a targeting peptide (GE11), and D shows the detection results of enrichment (siRNA-2) in the blood after injection of the delivery system with a targeting peptide (GE11),
Figure 110 shows the detection results of the in vivo enrichment, self-assembly and therapeutic effects on pulmonary fibrosis of a viral vector system containing different RNA fragments provided by an embodiment of the present application; where A shows the detection results of mRNA levels of PTP1B, and B shows the detection results of protein levels of PTP1B.
Figure 111 shows the detection results of the in vivo enrichment, self-assembly and therapeutic effects on pulmonary fibrosis after intravenous injection of a viral vector delivery system containing multiple RNA fragments and multiple targeting tags (CMV-siRNA-1+2) provided by an embodiment of the present application; where A shows the enrichment effect (represented as siRNA level) in the lung, and B shows the enrichment effect (represented as siRNA level) in the blood.
Figure 112 shows the detection results of the in vivo enrichment, self-assembly and therapeutic effects on pulmonary fibrosis after intravenous injection of a viral vector delivery system containing multiple RNA fragments and multiple targeting tags (CMV-GE11-siRNA-1+2, CMV-GE11-siRNA-1+CMV-GE11-siRNA-2) provided by another embodiment of the present application; where A and B show the enrichment effect (represented as siRNA level) in the lung, and C and D show the enrichment effect (represented as siRNA level) in plasma.
Figure 113 shows the detection results of the in vivo enrichment, self-assembly and therapeutic effects on pulmonary fibrosis after intravenous injection of a viral vector delivery system containing multiple RNA fragments and multiple targeting tags (CMV-GE11-siRNA-1+2, CMV-GE11-siRNA-1+CMV-GE11-siRNA-2) provided by another embodiment of the present application; where A and B show the detection results of protein levels of TGFb1, and C and D show the detection results of mRNA levels of TGFb1.
Figure 114 shows the detection results of the in vivo enrichment (represented as siRNA level in blood) in the case of an adenoviral vector delivery system containing three more than 80% homologous 5' flanking sequences/loop sequences/3' flanking sequences provided by an embodiment of the present application.
Figure 115 shows the detection results of the in vivo enrichment (represented as siRNA level in blood) in the case of a delivery system constructed with an adenoviral vector carrying multiple circuits in which adjacent circuits are linked via sequence 1-sequence 2-sequence 3 with sequence 2 containing multiple bases provided by an embodiment of the present application.
Figure 116 shows the detection results of the in vivo enrichment (represented as siRNA level in blood) in the case of a delivery system with a linker of sequence 4 and sequences with more than 80% homology to sequence 4 respectively provided by another embodiment of the present application.
Figure 117 shows the detection results of the therapeutic effect on pulmonary fibrosis of a delivery system constructed with RNA sequences of lengths of 18, 20, and 21 respectively provided by an embodiment of the present application; where A shows the relative levels of PTP1B mRNA with different lengths of RNA sequences, and B shows the relative levels of PTP1B protein with different lengths of RNA sequences.
Figure 118 shows the results of the hydroxyproline content detected in the case of a gene circuit comprising the antisense strand of miRNA-21 and five siRNAs of TGF-β1 gene provided by an embodiment of the present application.
Figure 119 shows the comparison of siRNA-related expression in mice provided by an embodiment of the present application.
Figure 120 shows the comparison of the treatment of glioblastoma in mice provided by an embodiment of the present application.
Figure 121 shows the comparison of immunohistochemical staining of mouse brain provided by an embodiment of the present application.
Figure 122 shows the verification of effects of in vivo enrichment and spontaneous formation of a complex of a plasmid delivery system carrying a single RNA fragment provided by an embodiment of the present application; where A shows the in vivo enrichment of plasmids containing different RNA fragments, and B shows the in vivo self-assembly effect shown by expression levels of different RNA fragment.
Figure 123 shows the verification of effects of in vivo enrichment and spontaneous formation of a complex of a plasmid delivery system carrying any two RNA fragments provided by an embodiment of the present application; where A shows the in vivo enrichment of plasmids containing different combinations of RNA fragments, and B shows the in vivo self-assembly effect shown by expression levels of different combinations of RNA fragment.
Figure 124 shows the verification of effects of in vivo enrichment and spontaneous formation of a complex of a plasmid delivery system carrying any three RNA fragments provided by an embodiment of the present application; where A shows the in vivo enrichment of plasmids containing different combinations of RNA fragments, and B shows the in vivo self-assembly effect shown by expression levels of different combinations of RNA fragment.
Figure 125 shows the verification of effects of in vivo enrichment and spontaneous formation of a complex of a plasmid delivery system carrying any two RNA fragments provided by another embodiment of the present application; where A shows the in vivo enrichment of plasmids containing different combinations of RNA fragments, and B shows the in vivo self-assembly effect shown by expression levels of different combinations of RNA fragment.
Figure 126 shows the verification of the in vivo enrichment effect of a plasmid delivery system carrying any 1-2 RNA fragments and 1-2 targeting tags in the same circuit provided by an embodiment of the present application. Figure 19 shows the verification of the in vivo enrichment effect of a plasmid delivery system carrying any 1-2 RNA fragments and 1-2 targeting tags in different circuits provided by another embodiment of the present application.

Figure 20 shows the verification of effects of in vivo enrichment and spontaneous formation of a complex of a plasmid delivery system carrying an identified 5' flanking sequence and at least 2 identified sequences with more than 80% homology thereto respectively provided by another embodiment of the present application; where A shows the in vivo enrichment effect of plasmids containing different 5' flanking sequences, and B shows the in vivo self-assembly effect shown by the expression levels of RNA fragments with different 5' flanking sequences.

Figure 21 shows the verification of effects of in vivo enrichment and spontaneous formation of a complex of a plasmid delivery system carrying an identified loop sequence and at least 2 identified sequences with more than 80% homology thereto respectively provided by another embodiment of the present application; where A shows the in vivo enrichment effect of plasmids containing different loop sequences, and B shows the in vivo self-assembly effect shown by the expression levels of RNA fragments with different loop sequences.

Figure 22 shows the verification of effects of in vivo enrichment and spontaneous formation of a complex of a plasmid delivery system carrying an identified 3' flanking sequence and at least 2 identified sequences with more than 80% homology thereto respectively provided by another embodiment of the present application; where A shows the in vivo enrichment effect of plasmids containing different 3' flanking sequences, and B shows the in vivo self-assembly effect shown by the expression levels of RNA fragments with different 3' flanking sequences.

Figure 23 shows the verification of effects of in vivo enrichment and spontaneous formation of a complex of a plasmid delivery system carrying a reverse complementary sequence of the RNA sequence with any 1, 2, 3, 4, or 5 bases deleted provided by another embodiment of the present application; where A shows the in vivo enrichment effect of plasmids containing different compensation sequences, and B shows the in vivo self-assembly effect shown by the expression levels of RNA fragments with different compensation sequences.

Figure 24 shows the verification of effects of spontaneous formation of a complex of a plasmid delivery system carrying four of the circuits in which the adjacent circuits are linked via sequence 1-sequence 2-sequence 3 provided by an embodiment of the present application.

Figure 25 shows the verification of effects of spontaneous formation of a complex of a plasmid delivery system carrying four of the circuits in which the adjacent circuits are linked via sequence 1-sequence 2-sequence 3 with sequence 2 of 5 bases, 10 bases, 20 bases, 30 bases, 40 bases, 50 bases or 80 bases provided by another embodiment of the present application.

Figure 26 shows the verification of effects of spontaneous formation of a complex of a plasmid delivery system with a linker of sequence 4 and at least two sequences with more than 80% homology to sequence 4 respectively provided by an embodiment of the present application.

Figure 27 shows the verification of the in vivo enrichment effect of a plasmid delivery system only containing a targeting peptide tag provided by an embodiment of the present application.

Figure 28 shows the verification of the in vivo enrichment effect of a plasmid delivery system only containing a targeting protein tag provided by an embodiment of the present application

Figure 29 shows the verification of effects of in vivo enrichment and spontaneous formation of a complex of a gene circuit containing siRNA of EGFR gene provided by an embodiment of the present application; where A shows the in vivo enrichment effects of different gene circuits containing siRNA of EGFR gene, and B shows the in vivo self-assembly effect shown by expression levels of different gene circuits containing siRNA of EGFR gene.

Figure 30 shows the verification of effects of in vivo enrichment and spontaneous formation of a complex of a gene circuit containing siRNA of TNC gene provided by an embodiment of the present application; where A shows the in vivo enrichment effects of different gene circuits containing siRNA of TNC gene, and B shows the in vivo self-assembly effect shown by expression levels of different gene circuits containing siRNA of TNC gene.

Figure 31 shows the verification of effects of in vivo enrichment and spontaneous formation of a complex of a delivery system containing two different ribose-modified RNA sequences provided by an embodiment of the present application; where A shows the in vivo enrichment effects of a delivery system containing different ribose-modified RNAs, and B shows the in vivo self-assembly effect shown by expression levels of different ribose-modified RNAs.

Figure 39 shows the comparison of mouse survival and tumor evaluation provided by an embodiment of the present application.

Figure 2 shows the verification of the in vivo enrichment and self-assembly effects of three other viral vectors provided by an embodiment of the present application; where A shows the in vivo enrichment result of other viral vector 1, B shows the in vivo enrichment result of other viral vector 2, C shows the in vivo enrichment result of other viral vector 3, and D shows the in vivo self-assembly result of three other viral vectors.

Figure 3 shows the verification of the in vivo enrichment and self-assembly effects of a viral vector carrying one of six RNA fragments respectively provided by an embodiment of the present application; where A shows the in vivo enrichment result of a vector containing different RNA fragments, and B shows the in vivo self-assembly effect shown by the expression levels of different RNA fragments.

Figure 4 shows the verification of the in vivo enrichment and self-assembly effects of a viral vector carrying four RNA fragments composed of any two RNA sequences respectively provided by an embodiment of the present application; where A shows the in vivo enrichment result of a vector containing different RNA fragments, and B shows the in vivo self-assembly effect shown by the expression levels of different RNA fragments.

Figure 5 shows the verification of the in vivo enrichment and self-assembly effects of a viral vector carrying three RNA fragments composed of any three RNA sequences respectively provided by an embodiment of the present application; where A shows the in vivo enrichment result of a vector containing different RNA fragments, and B shows the in vivo self-assembly effect shown by the expression levels of different RNA fragments.

Figure 6 shows the verification of the in vivo enrichment and self-assembly effects of a viral vector carrying two RNA fragments composed of any two other RNA sequences respectively provided by another embodiment of the present application; where A shows the in vivo enrichment result of a vector containing different RNA fragments, and B shows the in vivo self-assembly effect shown by the expression levels of different RNA fragments.

Figure 7 shows the verification of the in vivo enrichment and self-assembly effects of a viral vector carrying any 1-2 RNA fragments and 1-2 targeting tags in the same circuit provided by an embodiment of the present application; where A shows the in vivo enrichment result of a vector containing different RNA fragments and targeting tags, and B shows the in vivo self-assembly effect shown by the expression levels of different RNA fragments.

Figure 8 shows the verification of the in vivo enrichment and self-assembly effects of a viral vector carrying any 1-2 RNA fragments and 1-2 targeting tags in the different circuits provided by another embodiment of the present application; where A shows the in vivo enrichment result of a vector containing different RNA fragments and targeting tags, and B shows the in vivo self-assembly effect shown by the expression levels of different RNA fragments.

Figure 9 shows the verification of the in vivo enrichment and self-assembly effects of a viral vector carrying an identified 5' flanking sequence and at least 2 identified sequences with more than 80% homology thereto respectively provided by another embodiment of the present application; where A shows the in vivo enrichment effect of a vector containing different 5' flanking sequences, and B shows the in vivo self-assembly effect shown by the expression levels of RNA fragments with different 5' flanking sequences.

Figure 10 shows the verification of the in vivo enrichment and self-assembly effects of a viral vector carrying an identified loop sequence and at least 2 identified sequences with more than 80% homology thereto respectively provided by another embodiment of the present application; where A shows the in vivo enrichment effect of a vector containing different loop sequences, and B shows the in vivo self-assembly effect shown by the expression levels of RNA fragments with different loop sequences.

Figure 11 shows the verification of the in vivo enrichment and self-assembly effects of a viral vector carrying an identified 3' flanking sequence and at least 2 identified sequences with more than 80% homology thereto respectively provided by another embodiment of the present application; where A shows the in vivo enrichment effect of a vector containing different 3' flanking sequences, and B shows the in vivo self-assembly effect shown by the expression levels of RNA fragments with different 3' flanking sequences.

Figure 12 shows the verification of the in vivo enrichment and self-assembly effects of a viral vector carrying a reverse complementary sequence of the RNA sequence with any 1, 2, 3, 4, or 5 bases deleted provided by another embodiment of the present application; where A shows the in vivo enrichment effect of a vector containing different compensation sequences, and B shows the in vivo self-assembly effect shown by the expression levels of RNA fragments with different compensation sequences.

Figure 13 shows the verification of the self-assembly effect of a viral vector carrying four of the circuits in which the adjacent circuits are linked via sequence 1-sequence 2-sequence 3 provided by an embodiment of the present application.

Figure 14 shows the verification of the self-assembly effect of a viral vector carrying four of the circuits in which the adjacent circuits are linked via sequence 1-sequence 2-sequence 3 with sequence 2 of 5 bases, 10 bases, 20 bases, 30 bases, 40 bases, 50 bases or 80 bases provided by an embodiment of the present application.

Figure 15 shows the verification of the self-assembly effect of a viral vector with a linker of sequence 4 and at least two sequences with more than 80% homology to sequence 4 respectively provided by an embodiment of the present application.

Figure 16 shows the verification of the in vivo enrichment effect of a viral vector containing different targeting peptide tags provided by an embodiment of the present application.

Figure 17 shows the verification of the in vivo enrichment effect of a viral vector containing different targeting protein tags provided by an embodiment of the present application.

Figure 18 shows the verification of the in vivo enrichment and self-assembly effects of a gene circuit containing siRNA of EGFR gene provided by an embodiment of the present application; where A shows the in vivo enrichment effects of different gene circuits containing siRNA of EGFR gene, and B shows the in vivo self-assembly effect shown by expression levels of different gene circuits containing siRNA of EGFR gene.

Figure 19 shows the verification of the in vivo enrichment and self-assembly effects of a gene circuit containing siRNA of TNC gene provided by an embodiment of the present application; where A shows the in vivo enrichment effects of different gene circuits containing siRNA of TNC gene, and B shows the in vivo self-assembly effect shown by expression levels of different gene circuits containing siRNA of TNC gene.

Figure 20 shows the verification of the in vivo enrichment and self-assembly effects of a viral vector delivery system containing two different ribose-modified RNA sequences provided by an embodiment of the present application; where A shows the in vivo enrichment effects of a viral vector delivery system containing different ribose-modified RNAs, and B shows the in vivo self-assembly effect shown by expression levels of different ribose-modified RNAs.

Figure 40 shows fluorescence microscope images of mouse hypothalamus and liver provided by an embodiment of the present application.

Figure 41 shows the comparison of the treatment of obesity in mice provided by an embodiment of the present application.

Figure 42 shows the comparison of the treatment of fatty liver in mice with obesity provided by an embodiment of the present application.

Figure 43 shows the comparison of the treatment of obesity in mice provided by an embodiment of the present application.

Figure 44 shows the comparison of various obesity indicators in mice provided by an embodiment of the present application.

Figure 3 shows the detection results of the in vivo enrichment effect of an RNA delivery system constructed with adenovirus or lentivirus as a viral vector provided by an embodiment of the present application; where A shows the detection results of siRNA levels in the blood after injection of the delivery system, and B shows the detection results of siRNA levels in the hypothalamus after injection of the delivery system.

Figure 4 shows the detection results of the in vivo enrichment effect of an RNA delivery system constructed with adenovirus or lentivirus as a viral vector provided by another embodiment of the present application, where the figure shows the detection results of siRNA levels in plasma exosomes after injection of the delivery system.

Figure 5 shows the detection results of the in vivo self-assembly effect and therapeutic effect on obesity of an RNA delivery system constructed with adenovirus or lentivirus as a viral vector provided by an embodiment of the present application; where A shows the detection results of mRNA level of PTP1B, B shows the detection results of protein level of PTP1B, and C shows the change value of body weight with increasing days.

Figure 6 shows the detection results of the in vivo enrichment, self-assembly and therapeutic effects on obesity of a viral vector system carrying different RNA fragments provided by an embodiment of the present application; where A shows the detection results of the relative level of PTP1B mRNA, and B shows the detection results of the relative level of PTP1B protein.

Figure 7 shows the detection results of the in vivo enrichment, self-assembly and therapeutic effects on obesity of an adenoviral vector delivery system comprising different RNA fragments siRNA-1, siRNA-2, and siRNA-1+siRNA-2 and different targeting tags RVG provided by an embodiment of the present application; where A shows the detection results of the relative level of PTP1B mRNA, B shows the detection results of the relative level of PTP1B protein, and C shows the change value of body weight with increasing days.

Figure 8 shows the detection results of the in vivo enrichment (represented as siRNA level in blood) in the case of an adenoviral vector delivery system containing three more than 80% homologous 5' flanking sequences/loop sequences/3' flanking sequences provided by an embodiment of the present application.

Figure 9 shows the detection results of the in vivo enrichment (represented as siRNA level in blood) in the case of a delivery system constructed with an adenoviral vector carrying multiple circuits in which adjacent circuits are linked via sequence 1-sequence 2-sequence 3 with sequence 2 containing multiple bases provided by an embodiment of the present application.

Figure 10 shows the detection results of the in vivo enrichment (represented as siRNA level in blood) in the case of a delivery system with a linker of sequence 4 and sequences with more than 80% homology to sequence 4 respectively provided by another embodiment of the present application.

Figure 11 shows the detection results of the therapeutic effect on obesity of a delivery system constructed with RNA sequences of lengths of 18, 20, and 21 respectively provided by an embodiment of the present application; where A shows the relative levels of PTP1B mRNA with different lengths of RNA sequences, and B shows the relative levels of PTP1B protein with different lengths of RNA sequences.

Figure 45 shows the comparison of the treatment of Huntington's disease in mice provided by an embodiment of the present application.

Figure 46 shows the comparison of siRNA and protein levels in mouse liver, cortex, and striatum provided by an embodiment of the present application.

Figure 47 shows the comparison of the treatment of Huntington's disease in mice provided by an embodiment of the present application.

Figure 48 shows the comparison of mHTT protein levels and toxic aggregates in the mouse striatum and cortex provided by an embodiment of the present application.

Figure 49 shows the comparison of the treatment of Huntington's disease in mice provided by an embodiment of the present application.

Figure 3 shows the in vivo enrichment effect of a lentiviral vector loaded with siRNA provided by an embodiment of the present application.

Figure 4 shows the in vivo self-assembly effect of a lentiviral vector loaded with siRNA provided by an embodiment of the present application.

Figure 5 shows the electrophoresis results of the therapeutic effect on Huntington's disease of a lentiviral vector loaded with siRNA provided by an embodiment of the present application.

Figure 6 shows the in vivo enrichment of adenovirus-associated virus type 1, 4, and 7 vectors loaded with siRNA provided by an embodiment of the present application.

Figure 7 shows the electrophoresis results of the therapeutic effect on Huntington's disease of adeno-associated virus type 1, 2, and 7 vectors loaded with siRNA provided by an embodiment of the present application.

Figure 8 shows the therapeutic effect on Huntington's disease of adeno-associated virus type 1, 2, and 7 vectors loaded with siRNA provided by an embodiment of the present application, where HTT mRNA levels are used for comparison.

Figure 9 shows the in vivo enrichment of adenovirus-associated virus type 8 vector loaded with 6 respective RNA sequences provided by an embodiment of the present application.

Figure 10 shows the in vivo enrichment of adenovirus-associated virus type 9 vector loaded with 6 respective RNA sequences provided by an embodiment of the present application.

Figure 11 shows the electrophoresis results of the therapeutic effect on Huntington's disease of adenovirus-associated virus type 9 vector loaded with 6 respective RNA sequences provided by an embodiment of the present application.

Figure 12 shows the therapeutic effect on Huntington's disease of adeno-associated virus type 8 vector loaded with a RNA fragment of any two RNA sequences provided by an embodiment of the present application, where HTT mRNA levels are used for comparison.

Figure 13 shows the electrophoresis results of the therapeutic effect on Huntington's disease of adeno-associated virus type 9 vector loaded with a RNA fragment of any two RNA sequences provided by an embodiment of the present application.

Figure 14 shows the therapeutic effect on Huntington's disease of adeno-associated virus type 8 vector loaded with a RNA fragment of any three RNA sequences provided by an embodiment of the present application, where HTT mRNA levels are used for comparison.

Figure 15 shows the electrophoresis results of the therapeutic effect on Huntington's disease of adeno-associated virus type 9 vector loaded with a RNA fragment of any three RNA sequences provided by an embodiment of the present application.

Figure 16 shows the in vivo enrichment of adeno-associated virus type 9 vector containing siRNA and RVG provided by an embodiment of the present application.

Figure 17 shows the therapeutic effect on Huntington's disease of adeno-associated virus type 9 vector containing siRNA and RVG provided by an embodiment of the present application, where HTT mRNA levels are used for comparison.

Figure 18 shows the therapeutic effect on Huntington's disease of an adenoviral vector with RVG-LAMP2B fusion protein and other fusion proteins provided by an embodiment of the present application, where HTT mRNA relative levels are used for comparison.

Figure 19 shows the in vivo enrichment of an adenoviral vector system comprising three RNA sequences with more than 80% homology to the siRNA sequence of HTT gene provided by an embodiment of the present application.

Figure 20 shows the therapeutic effect on Huntington's disease of an adenoviral vector system comprising three RNA sequences with more than 80% homology to the siRNA sequence of HTT gene provided by an embodiment of the present application, where HTT mRNA relative levels are used for comparison.

Figure 50 shows the comparison of the treatment of Parkinson's disease in transgenic mice provided by an embodiment of the present application.

Figure 12 shows the in vivo enrichment data of plasmid 1 containing six RNAs respectively provided in an embodiment of the present application.

Figure 13 shows the in vivo enrichment data of plasmid 2 containing six RNAs respectively provided in an embodiment of the present application.

Figure 14 shows the in vivo self-assembly data of exosomes containing six RNAs respectively provided by an embodiment of the present application.

Figure 15 shows the therapeutic effect of a plasmid containing six RNAs respectively on Parkinson's disease represented by the expression results of LRRK2 gene provided by an embodiment of the present application.

Figure 16 shows the in vivo enrichment data of plasmid 1 containing any two RNA sequences provided in an embodiment of the present application.

Figure 17 shows in vivo self-assembly data of exosomes containing any two RNA sequences provided in an embodiment of the present application.

Figure 18 shows the therapeutic effect of plasmid 1 containing any two RNA sequences on Parkinson's disease represented by the expression results of LRRK2 mRNA provided by an embodiment of the present application.

Figure 19 shows the therapeutic effect of plasmid 2 containing any two RNA sequences on Parkinson's disease represented by the expression results of LRRK2 gene provided by another embodiment of the present application.

Figure 20 shows the therapeutic effect of plasmid 1 containing any three RNA sequences on Parkinson's disease represented by the expression results of LRRK2 mRNA provided by an embodiment of the present application.

Figure 21 shows the therapeutic effect of plasmid 2 containing any three RNA sequences on Parkinson's disease represented by the expression results of LRRK2 gene provided by another embodiment of the present application.

Figure 22 shows the in vivo enrichment data of a plasmid containing siRNA and RVG provided by an embodiment of the present application.

Figure 23 shows the therapeutic effect of a plasmid containing siRNA and RVG on Parkinson's disease represented by the expression results of LRRK2 mRNA provided by another embodiment of the present application.

Figure 24 shows the therapeutic effect of a plasmid vector with RVG-LAMP2B fusion protein or other fusion proteins on Parkinson's disease represented by the expression results of LRRK2 mRNA provided by another embodiment of the present application.

Figure 25 shows the in vivo enrichment data of a gene circuit comprising three RNA sequences with more than 80% homology to the siRNA sequence of LRRK2 gene provided by an embodiment of the present application.

Figure 26 shows the therapeutic effect of a gene circuit comprising three RNA sequences with more than 80% homology to the siRNA sequence of LRRK2 gene on Parkinson's disease represented by the relative levels of LRRK2 mRNA provided by another embodiment of the present application.

Figure 4 shows the in vivo enrichment effect of a lentiviral vector loaded with siRNA provided by an embodiment of the present application.

Figure 5 shows the in vivo self-assembly effect of a lentiviral vector loaded with siRNA provided by an embodiment of the present application.

Figure 6 shows the therapeutic effect on Parkinson's disease of a lentiviral vector loaded with siRNA provided by an embodiment of the present application, where LRRK2 mRNA levels are used for comparison.

Figure 7 shows the in vivo enrichment effect of adenovirus-associated virus type 1, 4, and 7 vectors loaded with siRNA provided by an embodiment of the present application

Figure 8 shows the in vivo self-assembly effect of adenovirus-associated virus type 1, 4, and 7 vectors loaded with siRNA provided by an embodiment of the present application.

Figure 9 shows the therapeutic effect data on Parkinson's disease of adeno-associated virus type 1, 4, and 7 vectors loaded with siRNA provided by an embodiment of the present application, where LRRK2 mRNA levels are used for comparison.

Figure 10 shows the in vivo enrichment effect of adenovirus-associated virus type 8 vector loaded with 6 respective RNA sequences provided by an embodiment of the present application.

Figure 11 shows the in vivo self-assembly effect of adenovirus-associated virus type 8 and type 9 vectors loaded with 6 respective RNA sequences provided by an embodiment of the present application.

Figure 12 shows the in vivo enrichment effect of adeno-associated virus type 9 vector loaded with a RNA fragment of any two RNA sequences provided by an embodiment of the present application.

Figure 13 shows the in vivo self-assembly effect of adeno-associated virus type 8 and type 9 vectors loaded with a RNA fragment of any two RNA sequences provided by an embodiment of the present application.

Figure 14 shows the electrophoresis results of the therapeutic effect on Huntington's disease of an adenovirus vector loaded with a RNA fragment of any three RNA sequences provided by an embodiment of the present application.

Figure 15 shows the in vivo enrichment data of adeno-associated virus type 9 vector containing siRNA and RVG provided by an embodiment of the present application.

Figure 16 shows the therapeutic effect of adeno-associated virus type 9 vector containing siRNA and RVG on Parkinson's disease represented by the expression results of LRRK2 mRNA provided by another embodiment of the present application.

Figure 17 shows the in vivo enrichment date of an adenoviral vector with RVG-LAMP2B fusion protein and other fusion proteins provided by an embodiment of the present application.

Figure 18 shows the therapeutic effect of an adenoviral vector with RVG-LAMP2B fusion protein and other fusion proteins on Parkinson's disease represented by the relative levels of LRRK2 mRNA provided by another embodiment of the present application.

Figure 19 shows the in vivo enrichment of an adenoviral vector system comprising three RNA sequences with more than 80% homology to the siRNA sequence of LRRK2 gene provided by an embodiment of the present application.

Figure 20 shows the therapeutic effect of an adenoviral vector system comprising three RNA sequences with more than 80% homology to the siRNA sequence of LRRK2 gene on Parkinson's disease represented by the relative levels of LRRK2 mRNA provided by another embodiment of the present application.

Figure 51 shows siRNA concentration changes in whole blood of Macaca fascicularis provided by an embodiment of the present application.

Figure 52 shows the comparison of the effects of chemical modifications on siRNA levels in mice provided by an embodiment of the present application.

Figure 53 shows a schematic diagram of a system for delivering RNA that inhibits gene expression provided by the present invention and verification experiment results of one of the embodiments.

Figure 54 shows a schematic structural diagram of an exemplary plasmid CMV-siR^{E} provided by the present invention.

Figure 55 shows a schematic structural diagram of an exemplary plasmid U6-siR^{E} provided by the present invention.

### DETAILED DESCRIPTION

The essence and beneficial effects of the present invention will be further described below in conjunction with examples. The examples are only used to illustrate the present invention but not to limit the present invention.

### Example 1 Materials and Methods

Hematoxylin-eosin staining is referred to as HE staining. Hematoxylin stain is alkaline and can stain the basophilic structures of tissues (such as ribosomes, nuclei and ribonucleic acid in the cytoplasm, etc.) into blue-purple, and eosin is an acidic dye that can stain eosinophilic structures of tissues (such as intracellular and intercellular proteins, Lewy bodies, Mallory bodies, and most of the cytoplasm) into pink, making the morphology of the entire cell tissue clearly visible.

The specific steps of HE staining include: sample tissue fixation and sectioning; tissue sample deparaffinizing; tissue sample hydration; hematoxylin staining of tissue sections, differentiation and anti-blue; eosin staining of tissue sections and dehydration; air-drying and sealing tissue sample sections; and finally, observation and taking pictures under a microscope.

Masson's staining makes collagen fibers blue (stained by aniline blue) or green (stained by bright green), and muscle fibers red (stained by acid fuchsin and ponceau). The fixated tissue is stained sequentially or mixed with a series of anionic water-soluble dyes. It can be found that red blood cells are stained by the smallest molecular anionic dyes, muscle fibers and cytoplasm are stained by medium-sized anionic dyes, and collagen fibers are stained by macromolecular anionic dyes. This shows that red blood cells are the least permeable to anionic dyes, followed by muscle fibers and cytoplasm, while collagen fibers have the greatest permeability. Type I and type III collagen are green (GBM, TBM, mesangial matrix and renal interstitium are green), and eosinophilic proteins, renal tubular cytoplasm and red blood cells are red.

The specific steps of Masson's staining include: fixing the tissue in Bouin's solution, rinsing with running water overnight, and routinely dehydrating and embedding; deparaffinizing and dehydrating sections (deparaffinizing in xylene for 10 min× 3 times, absorbing the liquid with absorbent paper; 100% ethanol for 5 min × 2 times, absorbing the liquid with absorbent paper; 95% ethanol for 5 min × 2 times, absorbing the liquid with absorbent paper; rinsing with running water for 2 min, absorbing the liquid with absorbent paper); staining with Weigert's iron hematoxylin for 5-10 min; washing slightly with running water; differentiating with 0.5% hydrochloric acid and alcohol for 15 seconds; rinsing with running water for 3 min; staining with ponceau-fuchsin solution for 8 min; rinsing slightly with distilled water; treating with 1% phosphomolybdic acid aqueous solution for about 5 min; directly counterstaining with aniline blue solution or bright green solution for 5 min without washing with water; treating with 1% glacial acetic acid for 1 min; dehydrating in 95% ethanol for 5 min × 2 times, absorbing the liquid with absorbent paper; 100% ethanol for 5 min × 2 times, absorbing the liquid with absorbent paper; clearing in xylene for 5 min × 2 times, absorbing the liquid with absorbent paper; and sealing with neutral gum.

Western Blot transfers proteins to a membrane and then uses antibodies for detection. For known expressed proteins, the corresponding antibodies can be used as primary antibodies for detection. For the expression products of new genes, the antibodies against the fusion part can be used for detection.

In Western Blot, polyacrylamide gel electrophoresis is employed, where the detected object is protein, the "probe" is an antibody, and the "color development" uses a labeled secondary antibody. The protein sample separated by PAGE is transferred to a solid-phase carrier (such as nitrocellulose film), where it is absorbed through non-covalent bonds without changing the type or biological activity of the polypeptide separated by electrophoresis. The protein or peptide on the solid-phase carrier acts as an antigen and reacts immunologically with the corresponding antibody. Then, it reacts with the second antibody labeled with either enzyme or isotope. The protein component expressed by the specific target gene is separated through electrophoresis and detected through substrate color development or autoradiography. The process primarily comprises of extracting proteins, quantifying proteins, preparing gels, running electrophoresis, transferring membranes, performing immunolabeling, and developing.

Immunohistochemistry (also known as immunocytochemistry), based on the antigen-antibody reaction, is the process of identifying antigens (polypeptides or proteins) in tissue cells, determining their localization, and studying them qualitatively and relative quantitatively by chemical reaction of the chromogenic agent (fluorescein, enzyme, metal ion, or isotope) of the labeled antibody to develop color.

The main steps of immunohistochemistry include soaking sections, airing overnight, deparaffinizing in xylene followed by alcohol of gradient concentrations (100%, 95%, 90%, 80%, 75%, 70%, 50%, 3 min each time), using double distilled water, adding dropwise 3% hydrogen peroxide solution to remove catalase, washing with water, repairing antigen, adding dropwise 5% BSA, blocking for 1h, diluting primary antibody, washing with PBS buffer, incubating with secondary antibody, washing with PBS buffer, developing color with chromogenic solution, washing with water, staining with hematoxylin, dehydrating with ethanol of gradient concentrations, and sealing with neutral gum.

### Example 2

The inventor designed a system that delivers RNA that inhibits gene expression to an organ or tissu as needed, and the RNA is enriched and assembled in cells to form cellular microvesicles including exosomes, and then delivered to a target tissue for disease treatment after being released by the cells. The system comprises one or more RNAs that inhibit gene expression and a protein that targets a target tissue.

Figure 53a is a schematic diagram of an exemplary system for delivering RNA that inhibits gene expression provided by the present invention. The system is a vector (in this example, a plasmid) that comprises a nucleic acid encoding RNA that inhibits gene expression and/or a protein that targets a target tissue, which allows free combination of different functional modules. In the system, the core part, consisting of a promoter part and an siRNA-expressing part (e.g., siRNA-1 backbone in Figure 53a), was designed to produce and organize siRNAs as the payload for exosomes. Other composable parts (plug-ins) could then be integrated into the system framework comprising the core gene circuit to achieve plug-and-play functionality. Examples of composable parts include two types of siRNA that can be optimized: one modifying the membrane-anchored proteins of exosomes to enable tissue selectivity (e.g., Guiding Tag in Figure 53a); the other enabling the co-expression of a second siRNA (e.g., siRNA-2 backbone in Figure 53a) for the simultaneous inhibition of two molecular targets.

For the core gene circuit construct, the inventors optimized the siRNA expression backbone part encoded under the control of a promoter part to maximize guide strand expression while minimizing undesired passenger strand expression.

In one embodiment, epidermal growth factor receptor (EGFR) is used as the siRNA target of the core circuit. EGFR is one of the most potent oncogenes that is frequently mutated and highly expressed in a variety of human cancers (e.g., lung cancer and glioblastoma). In addition, human embryonic kidney 293T cells (HEK293T) and mouse hepatoma cells (Hepa 1-6) were chosen as the cell chassis for in vitro assembly of siRNA. To optimize the siRNA production efficiency, two design strategies were compared: one is to drive the expression of an miRNA precursor (pre-miRNA) by the CMV promoter and substitute the miRNA sequence with siRNA, thus constructing the plasmid EGFR siRNA (CMV-siR^{E}) (the structure of the plasmid is shown in Figure 54, synthesized and provided by Realgene Biotech Company, Nanjing, China), in which the siRNA sequence (the nucleotide sequence shown in SEQ ID NO.: 1: UGUGGCUUCUCUUAACUCCU) inhibiting EGFR is operably linked after the CMV promoter, with ggatcctggaggcttgctgaaggctgtatgctgaattc (SEQ ID NO.: 2) as the 5' flanking sequence, gttttggccactgactgac (SEQ ID NO.: 4) as the stem-loop sequence, and accggtcaggacacaaggcctgttactagcactcacatggaacaaatggcccagatctggccgcactcgag (SEQ ID NO.: 3) as the 3' flanking sequence, and the reverse complement sequence of the RNA fragment with base at position 9 and/or 10 of the RNA fragment deleted.

The other is to use the U6 promoter to drive the expression of a short hairpin RNA (shRNA), thus constructing the plasmid EGFR shRNA (U6-siR^{E}) (the structure of the plasmid is shown in Figure 55, synthesized and provided by Realgene Biotech Company, Nanjing, China).

The siRNA production efficiency of a CMV-directed pre-miRNA encoding an EGFR siRNA (CMV-siR^{E}) and a U6-directed EGFR shRNA (U6-shR^{E}) was compared, see Figure 53b. Both strategies had similar efficiencies in driving the transcription of the guide strand of EGFR siRNA; compared with the shRNA approach, the pre-miRNA approach produced less or no passenger strand (the passenger strand was degraded during the biogenesis of mature guide strand), see Figure 53b. Therefore, the CMV-driven pre-miRNA design was chosen to avoid off-target effects in subsequent experiments.

Whether the core gene circuit directed the loading of siRNA into exosomes was examined.

CMV-scr^{R} was constructed. CMV-scr^{R} has the same nucleotide type and number as the siRNA encoding sequence in CMV-siR^{E} but a different nucleotide arrangement, serving as a blank control for encoding CMV-siR^{E}.

HEK293T cells were transfected with CMV-scr^{R} or CMV-siR^{E}, and the exosomes in cell culture medium were characterized. Nanoparticle tracking analysis (NTA) revealed that similar amounts of exosomes were secreted in each group with similar size and distribution, peaking at 128-131 nm. Transmission electron microscopy (TEM) confirmed that the purified exosomes exhibited a typical round-shaped vesicular morphology and had correct size. Moreover, enrichment of specific exosome markers (CD63, TSG101 and CD9) was only detected in purified exosomes but not in cell culture medium. These results demonstrate that transfection with gene circuits did not affect the size, structure or number of exosomes generated by HEK293T cells. Finally, a significant amount of EGFR siRNA was detected in exosomes derived from HEK293T and Hepa 1-6 cells transfected with the CMV-siR^{E} gene circuit, see Figure 53c. When these exosomes were incubated with mouse Lewis lung carcinoma (LLC) cells, a dose-dependent knockdown of EGFR expression was achieved, see Figure 53d and Figure 53e, suggesting that the exosomal siRNAs were biologically functional.

In one embodiment, exosomes are introduced into the brain using the RVG-Lamp2b fusion protein (the RVG targeting peptide linked to the N-terminus of the Lamp2b protein) as the anchoring protein. Rabies virus glycoprotein (RVG) is a neurotropic protein that can bind to acetylcholine receptors expressed in nerve cells. RVG has been shown to help exosomes cross the blood-brain barrier and enter nerve cells. In CMV-RVG-siR^{E}, the sequence encoding the RVG-Lamp2b fusion protein is inserted downstream of the CMV promoter and upstream of the siRNA. Wherein, the amino acid sequence of RVG is shown in SEQ ID NO.: 17. The amino acid sequence of the entire RVG-Lamp2b fusion protein is shown in SEQ ID NO.: 18.

The efficiency of the promoters in initiating the expression of the RVG-Lamp2b fusion protein was assessed. The CMV promoter generates RVG-Lamp2b mRNA and marker protein eGFP in HEK293T cells. The correct display of the targeting tag on the exosome surface was then verified using immunoprecipitation. A Flag tag was used to temporarily replace RVG in the experiment. After transfection of HEK293T and Hepa 1-6 cells with CMV-directed Flag-Lamp2b, intact exosomes were successfully immunoprecipitated with anti-Flag beads, see Figure 53f, demonstrating the precise localization of the targeting tag.

In one embodiment, tenascin-C (TNC), a critical oncogene associated with many cancers, especially glioblastoma, was selected as the second siRNA target. TNC-siRNA comprises siRNA sequence that inhibits TNC and is expressibly linked after the CMV promoter, and is also embedded in the pre-miR-155 backbone to obtain plasmid CMV-siR^{T}. Wherein, the siRNA of the TNC gene has the nucleotide sequence of UAUGAAAUGUAAAAAAAGGGA (SEQ ID NO. 5).

In addition, the siRNA inhibiting EGFR and the siRNA inhibiting TNC were constructed in tandem on the same plasmid. TNC-siRNA was inserted downstream of EGFR-siRNA, and cagatctggccgcactcgaggtagtgagtcgaccagtggatc (SEQ ID NO.: 6) was used as the linker between the sequences encoding EGFR-siRNA and TNC-siRNA, to obtain plasmid CMV siR^{E+T}. In the experiment, it was found that regardless of the individual (CMV siR^{E} or CMV siR^{T}) or tandem (CMV siR^{E+T}) transcription, equal amounts of EGFR and TNC siRNA were detected, see Figure 53g.

Another immunoprecipitation experiment was performed to assess the association of AGO2 with siRNAs in exosomes. Experiments demonstrated that EGFR and TNC siRNAs were readily detected in the exosomes precipitated with anti-AGO2 antibody, suggesting that the loading of siRNA into the RNA-induced silencing complex (RISC) can be guaranteed and facilitates the efficient transport of AGO2-bound siRNA into exosomes. Finally, to investigate whether the in vitro assembled siRNAs are functional, exosomes derived from HEK293T cells transfected with the CMV-RVG-siR^{E+T} gene circuit were incubated with the U87MG glioblastoma cells. A dose-dependent downregulation of EGFR and TNC expression in U87MG cells was achieved, see Figure 53i and Figure 53j. Moreover, the RVG tag on exosome surface did not affect the silencing efficacy of EGFR and TNC siRNAs on their targets.

Herein, unless otherwise specified, CMV-siR gene abbreviation or initials represents a plasmid, which has the construction structure of CMV-siR^{E} as described above but differs only in the RNA sequence of the encoded gene to be inhibited. For example, "plasmid CMV-siR^{T}" or "CMV-siR^{T}" refers to a plasmid in which an siRNA sequence that inhibits TNC is expressibly linked after a CMV promoter as described above, with ggatcctggaggcttgctgaaggctgtatgctgaattc (SEQ ID NO.: 2) as 5' flanking sequence, gttttggccactgactgac (SEQ ID NO.: 4) as the stem loop sequence, accggtcaggacacaaggcctgttactagcactcacatggaacaaatggcccagatctggccgcactcgag (SEQ ID NO.: 3) as the 3' flanking sequence, and the reverse complement sequence of the RNA fragment with base at position 9 and/or 10 of the RNA fragment deleted. In addition, CMV-siR ^{the first letter of the first gene + the first letter of the second gene} represents a plasmid carrying both the siRNA sequence that inhibits the first gene and the siRNA that inhibits the second gene, which has a construction structure as CMV-siR^{E+T} but differs only in the RNA sequence of the encoded gene to be inhibited, and the siRNA sequence inhibiting the second gene is inserted downstream of the siRNA sequence inhibiting the first gene, with cagatctggccgcactcgaggtagtgagtcgaccagtggatc (SEQ ID NO.: 6) as the linker between the sequences encoding TNC-siRNA and EGFR-siRNA. By analogy, it can represent a plasmid carrying siRNA sequences that inhibit multiple genes at the same time. In addition, CMV-abbreviation or initials of targeting peptide - siR ^{gene abbreviation or initials} represents that a targeting peptide, such as RVG, is present downstream of the CMV promoter and upstream of the siRNA.

### Example 3

As shown in Figure 1A, in order to understand the distribution of the plasmid in the body, a plate test was conducted on mice. After the plasmid CMV-siR^{E} was injected into the mice, samples were taken at various time points (1h, 3h, 6h, 9h, 12h, 24h, 72h, 168h, 720h). The plasmid extracted with spectinomycin was used for transformation, and the number of clones in the liver, plasma, lung, brain, kidney, and spleen was observed. The results are shown in Figure 1B, Figure 1C, and Figure 1D. It can be seen that the plasmid was mostly distributed in the mouse liver, reached its peak about 3 h after injection, and was basically metabolized 12 h after injection.

CMV eGFP siR^{E} co-expressing eGFP protein and EGFR siRNA was injected intravenously into C57BL/6J mice. The results are shown in Figure 2 that the eGFP fluorescence in the mouse liver gradually increased over time, reached a peak at about 12 h, and decreased to the background level at 48 h. No obvious eGFP signal was seen in other tissues.

The control plasmid (CMV-scrR) and the plasmid expressing EGFR siRNA (CMV-siR^{E}) were injected into the mice respectively, and an in vitro model of mouse hepatocytes was established. The related siRNA levels in the hepatocyte exosomes of mice injected with CMV-scrR and CMV-siR^{E} were respectively detected. The results are shown in Figure 3A. It can be seen that siRNA expression was present in the hepatocyte exosomes of mice injected with CMV-siR^{E}.

Ago2 immunoprecipitation experiments were performed, and the results are shown in Figure 3B and Figure 3C. Wherein, Input represents a sample in which exosomes were directly lysed and detected without immunoprecipitation, serving as a positive control.

After intravenous injection of the plasmid into mice, the distribution of mature siRNA in different tissues is shown in Figure 4. It can be seen from Figure 4A that the level of EGFR-siRNA in plasma, exosomes, and plasma without exosomes varied in a time-dependent manner; as can be seen from Figure 4B, the accumulation of mouse EGFR-siRNA in the liver, lung, pancreas, spleen, and kidney was time-dependent.

Mice were injected with control plasmid (CMV-scrR), 0.05 mg/kg CMV-siR^{E} plasmid, 0.5 mg/kg CMV-siR^{E} plasmid, and 5 mg/kg CMV-siR^{E} plasmid, respectively, and the level of absolute siRNA (EGFR siRNA) was detected in the mouse liver, spleen, heart, lung, kidney, pancreas, brain, skeletal muscle, and CD4⁺ cells. The results are shown in Figure 5A. It can be seen that there was no expression of siRNA in the tissues of mice injected with control plasmid, and in the tissues of mice injected with CMV-siR^{E} plasmid, the level of siRNA expression was positively correlated with the concentration of CMV-siR^{E} plasmid. As shown in Figure 5B, fluorescence in situ hybridization assay (FISH) also confirmed that the level of siRNA expression was positively correlated with the concentration of CMV-siR^{E} plasmid, indicating that the tissue distribution of EGFR siRNA was dose-dependent.

After the plasmid enters the body, it will express the precursor and then process it into a mature body (siRNA). Therefore, the metabolism of the precursor and mature body (siRNA) were detected in the liver of mice injected with the plasmid, and the result is shown in Figure 6. It can be seen that the expression levels of precursor and mature body (siRNA) in the mouse liver reached the peak at 6 h after the injection of the plasmid; 36 h after plasmid injection, the metabolism of the mature body (siRNA) was completed in the mouse liver; and 48 h after the plasmid injection, the metabolism of the precursor was completed in the mouse liver.

After injecting exogenous siRNA into the common bile duct of mice, the levels of absolute siRNA in exosome-free plasma, exosomes, and plasma were detected respectively. The results are shown in Figure 7A. After injecting exogenous siRNA into the common bile duct of mice, the levels of siRNA in the spleen, heart, lung, kidney, pancreas, brain, skeletal muscle, and CD4⁺ cells of mice were detected respectively. The results are shown in Figure 7B. These two figures reflect that the kinetics of siRNA in different tissues was almost the same, and the distribution of siRNA in different tissues was significantly different.

Mice were intravenously injected with siRNA with albumin ALB as promoter, siRNA with CMV as promoter, and siRNA without any promoter respectively. The absolute siRNA levels in mice were detected at 0h, 3h, 6h, 9h, 12h, 24h, 36h, and 48h after injection, and the results are shown in Figure 8. It can be seen that the level of siRNA using CMV as the promoter was the highest in mice, that is, using CMV as the promoter had the best effect.

Fluorescence experiments were performed to observe the inhibition of eGFP levels in mice by self-assembled eGFP siRNA. The process was as follows: eGFP transgenic mice were intravenously injected with PBS, or 5 mg/kg CMV-siR^{G} or CMV-RVG-siR^{G} plasmid, and treated for 24 h. The mice were then sacrificed, and their eGFP fluorescence levels were detected in frozen sections. Figure 9A shows a representative fluorescence microscope image, in which green indicates positive eGFP signals and blue indicates DAPI-stained nuclei, with a scale bar of 100 µm. It can be seen that the inhibitory effect of CMV-RVG-siR^{G} plasmid on mouse eGFP was more obvious. eGFP transgenic mice were intravenously injected with PBS, or CMV-scrR or CMV-siR^{E} plasmid, and treated for 24 h. The mice were then sacrificed, and their eGFP fluorescence levels were detected in frozen sections. Figure 9B is a column comparison chart of the fluorescence intensity of the heart, lung, kidney, pancreas, brain, and skeletal muscle of mice injected with PBS, CMV-siR^{E}, and CMV-RVG-siR^{E}. It can be seen that the fluorescence intensity was more obvious in the liver, spleen, lung, and kidney of mice.

The alanine aminotransferase (ALT), aspartate aminotransferase (AST), total bilirubin (TBIL), blood urea nitrogen (BUN), serum alkaline phosphatase (ALP), creatinine (CREA) contents, thymus weight, spleen weight, and percentage in peripheral blood cells were detected in mice injected with PBS, CMV-scrR, and CMV-siR^{E} respectively. The results are shown in Figure 10. Figures 10A-F show the comparison of alanine aminotransferase, aspartate aminotransferase, total bilirubin, blood urea nitrogen, serum alkaline phosphatase, and creatinine contents in mice injected with PBS, CMV-scrR, and CMV-siR^{E}; Figure 10G show the comparison of mouse liver, lung, spleen, and kidney tissues, and Figures 10H-I show the comparison of mouse thymus and spleen tissues. The results showed that mice injected with PBS, CMV-scrR, and CMV-siR^{E} had almost the same ALT, AST contents, thymus weight, spleen weight, and percentage in peripheral blood cells. Compared with mice injected with PBS, mice injected with CMV-siRE also had no tissue damage in the liver, lung, spleen, and kidney.

Therefore, the RNA delivery system provided in this example uses a plasmid as a carrier, and as a mature injection, the plasmid has safety and reliability that have been fully verified, and has a very good druggability. The RNA sequence that exerts the final effect is encapsulated and transported by endogenous exosomes, with no immune responses, and there is no need to verify the safety of the exosomes. The delivery system can deliver all kinds of small molecule RNAs and has strong versatility. Moreover, the preparation of plasmids is much cheaper than the preparation of exosomes, proteins, polypeptides and the like, with good economy. The RNA delivery system provided in this example can tightly bind to AGO₂ and be enriched into a complex (exosomes) after self-assembly in vivo, which not only prevents its premature degradation and maintains its stability in circulation, but also facilitates receptor cell absorption, intracytoplasmic release and lysosomal escape, and only a low dose is required.

### Example 4

As shown in Figure 11A, mice were selected, and injected with mouse lung cancer cells (LLC cells), followed by PBS buffer/CMV-scrR/gefitinib/CMV-siR^{E} every two days for treatment. Survival analysis and tumor evaluation were performed on the mice. Treatment started on the 30th day and ended on the 44th day. Wherein, CMV-scrR represents the control plasmid, and CMV-siR^{E} represents the plasmid carrying the EGFR siRNA gene.

As shown in Figure 11B, the horizontal axis represents time and the vertical axis represents survival rate. From this figure, it can be seen that mice injected with CMV-siR^{E} had the highest survival rate.

As shown in Figure 11C, the figure shows the 3D modeling of mouse lung tissue according to the CT images of mice injected with PBS buffer/CMV-scrR/gefitinib/CMV-siR^{E} before and after treatment. It can be seen that the tumors of mice injected with CMV-siR^{E} were significantly reduced.

As shown in Figure 11D, the figure shows the comparison of the tumor volume (mm³) of mice injected with PBS buffer/CMV-scrR/gefitinib/CMVsiR^{E} before and after treatment. It can be seen that the tumor volume of mice injected with CMV-siR^{E} was significantly reduced, while the tumor volume of mice injected with PBS buffer/CMV scrR/gefitinib not only failed to decrease, but also increased to varying degrees.

As shown in Figure 11E, the figure shows the comparison of western blot results of normal mice and mice injected with PBS buffer/CMV-scrR/gefitinib/CMV-siR^{E}. It can be seen that mice injected with PBS buffer/CMV scrR/gefitinib had significantly higher levels of the EGFR gene.

As shown in Figure 11F, the figure shows the comparison of the levels of related EGFR miRNA in normal mice and mice injected with PBS buffer/CMV-scrR/gefitinib/CMV-siR^{E}. It can be seen that mice injected with PBS buffer/CMV scrR/gefitinib had relatively higher levels of related EGFR miRNA.

In summary, CMV-siR^{E} had a significant therapeutic effect on EGFR-mutated lung cancer tumors.

HE staining and immunohistochemical staining were performed on mice injected with PBS buffer/CMV-scrR/gefitinib/CMV-siR^{E} respectively. The results are shown in Figure 12Aand Figure 12B. EGFR had more expression in mice injected with PBS buffer/CMV scrR/gefitinib. The staining areas of EGFR and PCNA in mice were counted. The results are shown in Figure 12C and Figure 12D. It can be seen that the staining areas of EGFR and PCNA in mice injected with CMV-siR^{E} were the smallest, proving it had the best therapeutic effect on EGFR-mutated lung cancer tumors.

As shown in Figure 13A, KRAS^{G12D} p53^{-/-} mice were selected and inhaled with Adv-Cre. From Day 50 to Day 64 after inhalation, mice were injected with PBS buffer/CMV-scrR/gefitinib/CMV-siR^{K} every two days for treatment, and survival analysis and tumor evaluation of the mice were performed. Wherein, CMV-siR^{K} represents the plasmid carrying an siRNA sequence that inhibits KRAS (siRNA of the KRAS gene has the nucleotide sequence of UGAUUUAGUAUUAUUUAUGGC (SEQ ID NO.: 7).

As shown in Figure 13B, the horizontal axis represents the time after infection, and the vertical axis represents the survival rate. It can be seen from this figure that the survival rate of mice injected with CMV-siR^{K} was higher.

As shown in Figure 13C, the figure shows the 3D modeling of the mouse lung tissue according to the CT images of mice injected with CMV-scrR/CMV-siR^{K} before and after treatment. It can be seen that the injection of CMV-siR^{K} can significantly inhibit the growth of lung cancer tumors.

As shown in Figure 13D, the figure shows the comparison of the number of tumors in mice injected with CMV-scrR/CMV-siR^{K} before and after treatment. It can be seen that the number of tumors in mice injected with CMV-siR^{K} increased significantly less.

As shown in Figure 13E, the figure shows the comparison of tumor volume (mm³) of mice injected with CMV-scrR/CMV-siR^{K} before and after treatment. It can be seen that the tumor volume of mice injected with CMV-siR^{K} grew slowly, while the tumor volume of mice injected with CMV-scrR increased significantly.

As shown in Figure 13F, the figure shows the comparison of western blot results of mice injected with CMV-scrR/CMV-siR^{K}. It can be seen that the level of KRAS gene in mice injected with CMV-scrR was significantly higher.

As shown in Figure 13G, the figure shows the comparison of the levels of related KRAS mRNA in mice injected with CMV-scrR/CMV-siR^{K}. It can be seen that the levels of related KRAS mRNA in mice injected with CMV-scrR were relatively high.

In summary, CMV-siR^{K} had a significant therapeutic effect on KRAS-mutated lung cancer tumors.

HE staining and immunohistochemical staining were performed on mice injected with CMV-scrR/CMV-siR^{K}. The results are shown in Figure 14A, Figure 14D, and Figure 14E. It can be seen that mice injected with CMV-scrR had more expression of KRAS, p-AKT, and p-ERK, and a higher staining percentage. Western blot was used to detect the expression levels of related proteins in mice. As shown in Figure 14B and Figure 14C, mice injected with CMV-scrR had more expression of related proteins. This also shows that CMV-siR^{K} had a significant inhibitory effect on KRAS mutated lung cancer tumors.

The 5' flanking sequence preferably has a sequence that is identical to or more than 80% homologous with ggatcctggaggcttgctgaaggctgtatgctgaattc, including sequences with 85%, 90%, 92%, 95%, 98%, or 99% homology with ggatcctggaggcttgctgaaggctgtatgctgaattc.

The loop sequence preferably has a sequence that is identical to or more than 80% homologous with gttttggccactgactgac, including sequences with 85%, 90%, 92%, 95%, 98%, 99% homology with gttttggccactgactgac.

The 3' flanking sequence preferably has a sequence that is identical to or more than 80% homologous with accggtcaggacacaaggcctgttactagcactcacatggaacaaatggcccagatctggccgcactcgag, including sequences with 85%, 90%, 92%, 95%, 98%, 99% homology with accggtcaggacacaaggcctgttactagcactcacatggaacaaatggcccagatctggccgcactcgag.

The compensation sequence is the reverse complementary sequence of the RNA fragment with any 1-5 bases deleted. In the case where the RNA fragment contains only one RNA sequence, the compensation sequence may be the reverse complementary sequence of the RNA sequence with any 1-5 bases deleted.

Preferably, the compensation sequence is the reverse complementary sequence of the RNA fragment with any 1-3 bases deleted. In the case where the RNA fragment contains only one RNA sequence, the compensation sequence may be the reverse complementary sequence of the RNA sequence with any 1-3 bases deleted.

More preferably, the compensation sequence is the reverse complementary sequence of the RNA fragment with any 1-3 contiguous bases deleted. In the case where the RNA fragment contains only one RNA sequence, the compensation sequence may be the reverse complementary sequence of the RNA sequence with any 1-3 contiguous bases deleted.

Most preferably, the compensation sequence is the reverse complementary sequence of the RNA fragment with the base at position 9 and/or 10 deleted. In the case where the RNA fragment contains only one RNA sequence, the compensation sequence may be a reverse complementary sequence of the RNA sequence with the base at position 9 and/or 10 deleted. Deleting the base at position 9 and/or 10 has the best effect.

It should be noted that the above-mentioned flanking sequence, compensation sequence, and loop sequence are not randomly selected, but are determined based on a large number of theoretical studies and experiments. With the cooperation of the above-mentioned certain flanking sequence, compensation sequence, and loop sequence, the expression rate of RNA fragments can be maximized.

The optional two homologous 5' flanking sequences, two homologous loop sequences and two homologous 3' flanking sequences are as shown in the table below.

| **Name** | **Sequence** |
|---|---|
| 5flank1 | ggataatggaggcttgctgcaggctgtatgctgaattc |
| 5flank2 | ggatactggacgcttgcttaaggctgtatggtgaattc |
| Loop1 | gacttggccactgactgac |
| Loop2 | gttttggccactggctgtc |
| 3flank1 | agccgtcaggacatgaggcctgttactagcactcacgtggctcaaatggcagagatctggctacactccag |
| 3flank2 | actggtcacgacacaaggcctattactagcagtcacattgaacaaatggccaagatctcgccgcactgtag |

In the case where the plasmid carries two or more circuits, adjacent circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAGTG |
| 60 bases | TGGCCGCACTCGAGGTAGTGAGTCGACCAGTGGCCGCACTCGAGGTAGTG AGTCGACCAG |
| 70 bases | TGGCCGCACTCGAGGTAGTGAGTCGACCAGTGGCCGCACTCGAGGTAGTG AGTCGACCAGTGGCCGCACT |
| 80 bases | TGGCCGCACTCGAGGTAGTGAGTCGACCAGTGGCCGCACTCGAGGTAGTG AGTCGACCAGTGGCCGCACTCGAGGTAGTG |

More preferably, in the case where the plasmid carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

siRNA levels of EGFR in the lung after 9 h of intravenous injection of sequence 4 or two sequences with more than 80% homology to sequence 4.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

### Example 5

Construction of virus carrying EGFR siRNA/KRAS-siRNA.

The AAV-5 adeno-associated virus with high-affinity for the liver was used to encapsulate the nucleic acid construct fragment expressing siRNA that inhibited gene.

The required virus was constructed by using the adenovirus packaging kit and services provided by Hanheng Biotechnology (Shanghai) Co., Ltd.. The packaging steps include:
1. Synthesize or clone the target nucleic acid fragment;
2. Select an appropriate restriction enzyme to digest the vector, and recover the purified linearized vector through agarose gel;
3. Carry out enzyme digestion of the target fragment according to the designed primers, and recover the target fragment of the correct size through agarose gel;
4. Connect the linearized vector and the target fragment based on homologous recombination or by T4 ligation;
5. Transform competent DH5a or stbl3, plate the bacterial solution, and culture for 12-16 h;
6. Select single clones for colony verification;
7. Select the positive clones that are correct in colony verification for sequencing;
8. Sequence the correct clone samples for plasmid extraction.

The vector plasmids pAAV-RC and pHelper provided by Hanheng Biotechnology (Shanghai) Co., Ltd., as well as the vector plasmid AAV051 carrying the target nucleic acid construct fragment were used, in which the nucleic acid construct fragment was cloned from plasmids CMV-siR^{E} or CMV-siR^{K} comprising the nucleic acid fragment encoding the siRNA initiated by CMV as described above, wherein the siRNA sequence (the nucleotide sequence shown in SEQ ID NO.: 1: UGUGGCUUCUCUUAACUCCU) inhibiting EGFR or the siRNA sequence (the nucleotide sequence shown in SEQ ID NO.: 7: UGALTUUAGUALTUAULTUAUGGC) inhibiting KRAS was operably linked after the CMV promoter, with ggatcctggaggcttgctgaaggctgtatgctgaattc (SEQ ID NO.: 2) as the 5' flanking sequence, gttttggccactgactgac (SEQ ID NO.: 4) as the stem-loop sequence, and accggtcaggacacaaggcctgttactagcactcacatggaacaaatggcccagatctggccgcactcgag (SEQ ID NO.: 3) as the 3' flanking sequence, and the reverse complement sequence of the RNA fragment with base at position 9 and/or 10 of the RNA fragment deleted.

The AAV-5 viral vector carrying EGFR siRNA or KRAS-siRNA thus obtained was named AAV-CMV-EGFR siRNA or AAV-CMV-KRAS siRNA, respectively.

Herein, unless otherwise specified, AAV-CMV-gene siRNA or AAV-CMV-siR ^{gene abbreviation or initials} represents a viral vector, which has the structure of the AAV-CMV-EGFR siRNA as described above, but differs only in the sequence of the carried RNA that inhibits gene.

### Experiments:

Mice were injected with 100 µL of AAV solution with a titer of 10¹² V g/ml through the tail vein. The in vivo expression of the AAV system was monitored by small animal in vivo imaging. After 3 weeks, it was found that the AAV system was stably expressed in the body, especially in the liver.

In the second experiment, one experimental group and two control groups were set up, wherein the experimental group was the AAV-CMVKRAS-siRNA group, and the control groups were the PBS group and the AAV-CMV-scrR group.

The same number of mice were selected from each group. Mouse lung cancer cells (LLC cells) were injected into the mice, and CT scanning technology was used to observe the progress of mouse model establishment. After 30 days, the successfully established mice were administered once every two days. Specifically, mice in the PBS group/AAV CMV scrR group/AAV-CMV-KRAS siRNA group were injected with PBS buffer/AAV-CMV-scrR/AAV-CMV-KRAS siRNA once every two days for treatment. Survival analysis and tumor evaluation were performed on the mice. Treatment was stopped after 7 administrations.

The survival of mice in each group was counted within 100 days after treatment, and the results are shown in Figure 15A. It can be seen that the survival rates of mice in the PBS group and the AAV-CMV-scrR group were almost the same, while mice in the AAV-CMV-KRAS siRNA group had the highest survival rate.

CT scanning was performed on mice in each group before and after administration. 3D modeling of mouse lung tissue was performed based on the CT images, and the tumor volume was calculated. The results are shown in Figure 15B. In Figure 15B, "PBS pre" indicates the PBS group before administration, "PBS post" indicates the PBS group after administration; "AAV-CMV-scrR pre" indicates the AAV-CMV-scrR group before administration; "AAV-CMV-scrR post" indicates the AAV-CMV-scrR group after administration; "AAV-CMV-KRAS-siRNA pre" indicates the AAV-CMV-KRAS siRNA group before administration; and "AAV-CMV-KRAS-siRNA post" indicates the AAV-CMV-KRAS siRNA group after administration. It can be seen that the tumor volume of mice in the AAV-CMV-KRAS siRNA group decreased significantly after administration, while the tumor volume of mice in the PBS group and AAV-CMV-scrR group not only failed to decrease after administration, but also increased to varying degrees.

The expression levels of KRAS protein and mRNA in the lung of mice in each group were detected by RT-qPCR and Western blotting, respectively, and the results are shown in Figure 15C and Figure 15D. The results showed that the expression of KRAS protein and mRNA in the lung of mice in the AAV-CMV-KRAS siRNA group was reduced compared with the control groups.

The above experiments show that AAV-CMV-KRAS siRNA had a significant therapeutic effect on lung cancer tumors in mice.

In the third experiment, one experimental group and two control groups were set up, wherein the experimental group was the AAV-CMV-EGFR-siRNA group, and the control groups were the PBS group and the AAV-CMV-scrR group.

EGFR-DEL19 mouse model was constructed and fed with doxycycline feed to induce tumor occurrence. After 30 days, the successfully constructed mice were administered once every two days. Specifically, mice in the PBS group/AAV-CMV-scrR group/AAV-CMV-EGFR siRNA group were injected with PBS buffer/AAV CMV scrR/AAV CMV EGFR siRNA once every two days for treatment. Survival analysis and tumor evaluation were performed on the mice. Treatment was stopped after 7 administrations.

The survival of mice in each group was counted within 100 days after treatment, and the results are shown in Figure 16A. It can be seen that the survival rates of mice in the PBS group and the AAV-CMV-scrR group were almost the same, while mice in the AAV-CMV-EGFR siRNA group had the highest survival rate.

CT scanning was performed on mice in each group before and after administration, and the CT images are shown in Figure 16E. 3D modeling of mouse lung tissue was performed based on the CT images, and the tumor volume was calculated. The results are shown in Figure 16B. In Figure 16B, "PBS pre" indicates the PBS group before administration, "PBS post" indicates the PBS group after administration; "AAV-CMV-scrR pre" indicates the AAV-CMV-scrR group before administration; "AAV-CMV-scrR post" indicates the AAV-CMV-scrR group after administration; "AAV-CMV-EGFR-siRNA pre" indicates the AAV-CMV-EGFR siRNA group before administration; and "AAV-CMV-EGFR-siRNA post" indicates the AAV-CMV-EGFR siRNA group after administration. It can be seen that the tumor volume of mice in the AAV-CMV-EGFR siRNA group decreased significantly after administration, while the tumor volume of mice in the PBS group and AAV-CMV-scrR group not only failed to decrease after administration, but also increased to varying degrees.

The expression levels of EGFR protein and mRNA in the lung of mice in each group were detected by RT-qPCR and Western blotting, respectively, and the results are shown in Figure 16C and Figure 16D. The results showed that the expression of EGFR protein and mRNA in the lung of mice in the AAV-CMV-EGFR siRNA group was reduced compared with the control groups.

The above experiments show that AAV-CMV-EGFR siRNA had a significant therapeutic effect on EGFR-mutated lung cancer tumors in mice.

In the fourth experiment, two experimental groups and two control groups were set up, wherein the experimental groups were the AAV-CMVKRAS-siRNA group the AAV-CMV-EGFR-siRNA group, and the control groups were the PBS group and the AAV-CMV-scrR group.

EGFR-DEL19 mouse model was constructed and fed with doxycycline feed to induce tumor occurrence. After 30 days, the successfully constructed mice were administered once every two days. Specifically, mice in the PBS group/AAV CMV scrR group/AAV CMV EGFR siRNA group/AAV CMV KRAS siRNA group were injected with PBS buffer/AAV-CMV-scrR/AAV-CMV-EGFR siRNA/AAV-CMV-KRAS siRNA once every two days for treatment.

After treatment, alanine aminotransferase (ALT), aspartate aminotransferase (AST), total bilirubin (TBIL), serum alkaline phosphatase (ALP), creatinine (CREA) and blood urea nitrogen (BUN) were detected in each group of mice, and the results are shown in Figure 17A-Figure 17F. It can be seen that the contents of the above enzymes in the PBS group, AAV-CMV-scrR group, AAV-CMV-EGFR siRNA group and AAV-CMV-KRAS siRNA group were almost the same.

The above experiments demonstrate that the EGFR siRNA system (AAV-CMV-EGFR siRNA) and KRAS siRNA system (AAV-CMV-KRAS siRNA) encapsulated with AAV-5 type adeno-associated virus with high affinity for the liver is safe and reliable and will not produce negative effects.

The viral vector can also comprise a flanking sequence, a compensation sequence and a loop sequence that facilitate the correct folding and expression of the circuit, and the flanking sequence comprises 5' flanking sequence and 3' flanking sequence; the viral vector comprises a circuit selected from the group consisting of 5' promoter-5' flanking sequence-RNA fragment-loop sequence-compensation sequence-3' flanking sequence, 5'-promoter-targeting tag, and 5' promoter-targeting tag-5' flanking sequence-RNA fragment-loop sequence-compensation sequence-3' flanking sequence, or a combination thereof.

Wherein, the 5' flanking sequence preferably has a sequence that is identical to or more than 80% homologous with ggatcctggaggcttgctgaaggctgtatgctgaattc, including sequences with 85%, 90%, 92%, 95%, 98%, or 99% homology with ggatcctggaggcttgctgaaggctgtatgctgaattc.

The loop sequence preferably has a sequence that is identical to or more than 80% homologous with gttttggccactgactgac, including sequences with 85%, 90%, 92%, 95%, 98%, 99% homology with gttttggccactgactgac.

The 3' flanking sequence preferably has a sequence that is identical to or more than 80% homologous with accggtcaggacacaaggcctgttactagcactcacatggaacaaatggcccagatctggccgcactcgag, including sequences with 85%, 90%, 92%, 95%, 98%, 99% homology with accggtcaggacacaaggcctgttactagcactcacatggaacaaatggcccagatctggccgcactcgag.

The compensation sequence is the reverse complementary sequence of the RNA fragment with any 1-5 bases deleted. In the case where the RNA fragment contains only one RNA sequence, the compensation sequence may be the reverse complementary sequence of the RNA sequence with any 1-5 bases deleted.

Preferably, the compensation sequence is the reverse complementary sequence of the RNA fragment with any 1-3 bases deleted. In the case where the RNA fragment contains only one RNA sequence, the compensation sequence may be the reverse complementary sequence of the RNA sequence with any 1-3 bases deleted.

More preferably, the compensation sequence is the reverse complementary sequence of the RNA fragment with any 1-3 contiguous bases deleted. In the case where the RNA fragment contains only one RNA sequence, the compensation sequence may be the reverse complementary sequence of the RNA sequence with any 1-3 contiguous bases deleted.

Most preferably, the compensation sequence is the reverse complementary sequence of the RNA fragment with the base at position 9 and/or 10 deleted. In the case where the RNA fragment contains only one RNA sequence, the compensation sequence may be a reverse complementary sequence of the RNA sequence with the base at position 9 and/or 10 deleted. Deleting the base at position 9 and/or 10 has the best effect.

It should be noted that the above-mentioned flanking sequence, compensation sequence, and loop sequence are not randomly selected, but are determined based on a large number of theoretical studies and experiments. With the cooperation of the above-mentioned certain flanking sequence, compensation sequence, and loop sequence, the expression rate of RNA fragments can be maximized.

In the case where the viral vector carries two or more circuits, adjacent gene circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

siRNA levels of EGFR in the lung after 9 h of intravenous injection of the delivery system in which sequence 4 or two sequences 4-1 and 4-2 with more than 80% homology to sequence 4 was constructed into an AAV vector.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

### Example 6

The plasmid CMV-siR^{V} carrying VEGFR siRNA that inhibits VEGFR expression and the plasmid CMV-siR^{mT} carrying mTOR siRNA that inhibits mTOR expression were constructed according to the method described in Example 2. Wherein, the siRNA of the VEGFR gene has the nucleotide sequence of ALTCTUGAAGAGLTUGUALTUAGCC (SEQ ID NO.: 8), and the siRNA of the mTOR gene has the nucleotide sequence of AGAUAGUUGGCAAAUCUGCCA (SEQ ID NO. 9).

Different mice were injected with PBS buffer/control plasmid/VEGFR siRNA plasmid/mTOR siRNA plasmid/MIX siRNA plasmid (combined use of VEGFR siRNA and mTOR siRNA)/sunitinib/everolimus. The development of kidney cancer tumors in mice was observed, and the results are shown in Figure 18 and Figure 19. It can be seen that the development of kidney cancer in mice injected with MIX siRNA plasmid was most significantly inhibited, while the development of kidney cancer in mice injected with PBS buffer/control plasmid was more rapid.

In summary, the combined use of VEGFR siRNA and mTOR siRNA had a significant therapeutic effect on kidney cancer tumors.

In the case where the viral vector carries two or more circuits, adjacent gene circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

In the case where the linker is the above sequence 4, or sequence 4-1 and sequence 4-2 with more than 80% homology with sequence 4, detection after 9 h of the injection of the delivery system containing the above sequence shows that it also has the enrichment, self-assembly and therapeutic effects on cancers.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

### Example 7

The plasmid CMV-siR^{TNF-α} carrying TNF-α siRNA that inhibits TNF-α expression, the plasmid CMV-siR^{mtegrin-α} carrying anti-integrin-α siRNA that inhibits integrin-α expression, and the plasmid CMVsiR^{B7} carrying B7 siRNA that inhibits B7 expression were constructed according to the method described in Example 2. Wherein, TNF-α siRNA has the nucleotide sequence of AAAACAUAAUCAAAAGAAGGC (SEQ ID NO. 10, integrin-α siRNA has the nucleotide sequence of AUAAUCAUCUCCAUUAAUGUC (SEQ ID NO. 11), and B7 siRNA has the nucleotide sequence of ULTCTUCLTCTUGGGUAAUCLTUCAG (SEQ ID NO. 12).

In addition, the plasmid CMV-si^{mix}, that is, CMV-siR^{TNF-α+integrin-α+B7} carrying siRNAs of TNF-α siRNA, integrin-α siRNA and B7 siRNA simultaneously was constructed according to the method described in Example 2.

In the first experiment, 3 experimental groups and 3 control groups were set up. The experimental groups were anti-TNF-α (0.5) group, anti-TNF-α (5) group and anti-TNF-α (20) group; and the control groups were mock group, scr-RNA group and IFX group.

Among them, the anti-TNF-α (0.5) group, anti-TNF-α (5) group, and anti-TNF-α (20) group were plasmids carring TNF-α siRNA (CMV-siR^{TNF-α}). 0.5 µL, 5 µL, and 20 µL of CMV-siR^{TNF-α} solutions were injected into the mice through the tail vein.

The mock group was the negative control group, and the scr-RNA group and IFX group were injected with scr-RNA plasmid and IFX (infliximab) through the tail vein of mice respectively.

DSS-induced chronic colitis model was then constructed, during which weights were recorded every day. The results are shown in Figure 20 A. It can be seen that mice in the scr-RNA group experienced the most rapid weight loss, while mice in the anti-TNF-α (0.5) group, anti-TNF-α (5) group, and anti-TNF-α (20) group experienced a slower weight loss, and the higher the dose of TNF-α siRNA solution, the slower the weight loss of the mice, showing that the plasmid-encapsulated TNF-α siRNA system can reduce weight loss in colitis mice.

After the model was constructed, the in vivo expression of the plasmid system was monitored by small animal in vivo imaging, and then the mice were sacrificed for observation of the colon. The results are shown in Figure 20 B. It can be seen that mice in the scr-RNA group had the shortest colon length, while mice in the anti-TNF-α (0.5) group, anti-TNF-α (5) group, and anti-TNF-α (20) group had relatively longer colon length, and the higher the dose of TNF-α siRNA injected, the longer the mouse colon length, showing that the plasmid-encapsulated TNF-α siRNA system can improve the shortening of colon length caused by chronic inflammation to varying degrees.

The disease activity index of the mice was evaluated, and the results are shown in Figure 20 C. It can be seen that mice in the scr-RNA group, anti-TNF-α (0.5) group, and anti-TNF-α (5) group had a higher disease activity index, while mice in the anti-TNF-α (20) group and IFX group had a smaller disease activity index.

TNF-α mRNA was detected in mouse colon, and the results are shown in Figure 20D. It can be seen that the CMV-siR^{TNF-α} system can reduce the expression and secretion of colon TNF-α. TNF-α was detected in mouse colon, and the results are shown in Figure 17E. It can be seen that the AAV system can produce a certain amount of TNF-α. The pro-inflammatory factors IL-6, IL-12p70, IL-17A, and IL-23 in the colon were detected, and the results are shown in Figure 20F. It can be seen that the secretion of inflammatory factors in the high-dose group was lower than that in the control group as a whole.

HE staining of mouse colon sections and pathological score statistics were performed, and the results are shown in Figure 21A and Figure 21B. It can be seen that mice in the anti-TNF-α (0.5) group, anti-TNF-α (5) group, anti-TNF-α (20) group, especially the anti-TNF-α (20) group, had higher colon mucosal integrity, shallower immune cell infiltration, and significantly less colonic crypt abscesses and colon congestion and bleeding compared to the control groups.

The above experiments can prove that the use of plasmid-encapsulated TNF-α siRNA system treatment provided by the present invention was more effective than IFX in improving the manifestations of colitis.

In the second experiment, 4 experimental groups and 3 control groups were set up. The experimental groups were anti-TNF-α group, anti-integrin-α group, anti-B7 group, and anti-mix group. The control groups were mock group, PBS group, and scr-RNA group.

The anti-TNF-α group, anti-integrin-α group, anti-B7 group, and anti-mix group used plasmids to carry TNF-α siRNA (CMV-siR^{TNF-α}), integrin-α siRNA (CMV-siR^{integrin-α}), B7 siRNA (CMV-siR^{B7}), and siRNAs of TNF-α siRNA, integrin-α siRNA and B7 siRNA simultaneously (CMV-si^{mix}, i.e., CMV-siR^{TNF-α+integrin-α+B7}). 20 µL was injected into the mice through tail vein, and the expression of the system was monitored in vivo in small animals. It can be seen that the above system was stably expressed in vivo, especially in the liver.

The mock group was the negative control group, and mice in the scr-RNA group and PBS group were injected with scr-RNA plasmid and PBS solution (phosphate buffered saline solution) through the tail vein, respectively.

DSS-induced chronic colitis model was then constructed, during which weights were recorded every day. The results are shown in Figure 22A. It can be seen that the weight gain of mice in the anti-mix group was the most stable, indicating that the plasmid-encapsulated CMV-siR^{TNF-α+integrin-α+B7} gene circuit can significantly reduce the weight loss of mice with chronic colitis. The mice in the anti-TNF-α group, anti-integrin-α group, and anti-B7 group also had a significantly faster weight recovery than the scr-RNA group and PBS group during the inflammation remission period.

After the model construction was completed, the in vivo expression of the plasmid system was monitored by small animal in vivo imaging, and then the mice were sacrificed for observation of the colon. The results are shown in Figure 22B. It can be seen that the redness and swelling of the colon of mice in the four experimental groups were alleviated to varying degrees. The shortening of colon length caused by chronic inflammation also improved to varying degrees.

The disease activity index of mice was evaluated, and the results are shown in Figure 22C. It can be seen that the disease activity index of mice in the scr-RNA group and PBS group was higher, while the disease activity index of mice in the anti-TNF-α group, anti-integrin-α group, anti-B7 group, and anti-mix group decreased in sequence.

TNF-α mRNA, integrin mRNA and B7 mRNA were detected in mouse plasma, liver and colon. The results are shown in Figure 22D-Figure 22F. It can be seen that the system generated a certain amount of stably expressed RNA in plasma, liver and colon, and the system significantly reduced expression of TNF-α, integrin and B7 mRNA in colon.

HE staining was performed on mouse colon sections. The results are shown in Figure 23. It can be seen that mice in the four experimental groups, especially mice in the anti-mix group, had higher colon mucosal integrity, shallower immune cell infiltration, and significantly less colonic crypt abscesses and colon congestion and bleeding compared to the control groups.

### Example 8

The virus AAV-CMV-siR^{TNF-α} carrying TNF-α siRNA that inhibits TNF-α expression, the virus AAV-CMVsiR^{integrin-α} carrying anti-integrin-α siRNA that inhibits integrin-α expression, and the virus AAV-CMV-siR^{B7} carrying B7 siRNA that inhibits B7 expression were constructed according to the method described in Example 5. Virus. Wherein, TNF-α siRNA has the nucleotide sequence of AAAACAUAAUCAAAAGAAGGC (SEQ ID NO. 10), integrin-α siRNA has the nucleotide sequence of AUAAUCAUCUCCAUUAAUGUC (SEQ ID NO. 11), and B7 siRNA has the nucleotide sequence of LTCTCTUCLTCTUGGGUAAUCLTUCAG (SEQ ID NO. 12). In addition, a virus carrying TNF-α siRNA, integrin-α siRNA and B7 siRNA simultaneously was constructed (AAV-CMV-si^{mix}, i.e., CMV-siR^{TNF-a+integrin-α+B7}).

In the first experiment, we set up 3 experimental groups and 2 control groups. The experimental groups were AAV-CMV-siR^{TNF-α} (low) group, AAV-CMV-siR^{TNF-α} (medium) group, andAAV-CMV-siR^{TNF-α} (high) group; and the control groups were Normal group and AAV-CMV-scrR group.

The experimental protocol is shown in Figure 24A. The AAV-CMV-siR^{TNF-α} (low) group, AAV-CMV-siR^{TNF-α} (medium) group, and AAV-CMV-siR^{TNF-α} (high) group used AAV-5 type adeno-associated virus with high affinity for the liver to encapsulate the TNF-α siRNA gene circuit (AAV-CMV-siR^{TNF-α}). Mice were injected with 25 µL, 50 µL and 100 µL of AAV solution with a titer of 10¹² V g/ml through the tail vein.

The in vivo expression of the AAV system was monitored by small animal in vivo imaging. The results are shown in Figure 24B. It can be seen that after 3 weeks, the AAV system was stably expressed in the body, especially in the liver. Among them, the AAV-CMV-siR^{TNF-α} (high) group had an average radiation up to 8.42*10⁵ (p/sec/cm²/sr), and the expression site was in the liver, showing that the expression of the AAV system has a dose-dependent effect.

DSS-induced chronic colitis model was then constructed, during which weights was recorded every two days. The results are shown in Figure 24C. It can be seen that the AAV-encapsulated CMV-siR^{TNF-α} system can reduce the weight loss of mice with chronic colitis, and the mice in the three experimental groups had a significantly faster weight recovery than the AAV-CMV-scrR group during the inflammation remission period.

At the end of the tenth week of model construction, the in vivo expression of the AAV system was monitored by small animal in vivo imaging, and then the mice were sacrificed for observation of the colon. The results are shown in Figure 24D. It can be seen that the redness and swelling of the colon of mice in the AAV-CMV-siR^{TNF-α} (low) group, AAV-CMV-siR^{TNF-α} (medium) group, and AAV-CMV-siR^{TNF-α} (high) group were alleviated to varying degrees, and the shortening of colon length caused by chronic inflammation also improved to varying degrees, wherein the improvement of inflammation in the AAV-CMV-siR^{TNF-α} (high) group was the most significant.

The disease index of mice in each group was scored and counted, and the results are shown in Figure 24E. It can be seen that the disease index of mice in the AAV-CMV-siR^{TNF-α}(high) group was smaller than that of AAV-CW-siR^{TNF-α} (low) group, AAV-CMV-siR^{TNF-α} (medium) group and AAV-CMV-scrR group.

The levels of TNF-α siRNA in mice of each group were detected, and the results are shown in Figure 24F. It can be seen that the levels of TNF-α siRNA in mice of the three experimental groups were all high, while mice in the control group AAV-CMV-scrR group had almost no expression of TNF-α siRNA, showing that the above-mentioned AAV system can produce a certain amount of TNF-α siRNA.

The levels of TNF-α mRNA in mice of each group were detected, and the results are shown in Figure 24G. It can be seen that the levels of TNF-α mRNA in mice of the Normal group and the three experimental groups were all low, while the level of TNF-α mRNA in mice of the AAV-CMV-scrR group was high, showing that the AAV system can reduce the expression and secretion of TNF-α in the colon.

The pro-inflammatory cytokines IL-6, IL-12 and IL-23 in the mouse colon were detected, and the results are shown in Figure 25A. It can be seen that mice in the Normal group and the AAV-CMV-siR^{TNF-α} (high) group secreted the least pro-inflammatory cytokines, and mice in the AAV-CMV-scrR group secreted the most pro-inflammatory cytokines.

HE staining and pathological scoring statistics were performed on mouse colon sections, and the results are shown in Figure 25B and Figure 25C. It can be seen that mice in the experimental groups, especially mice in the AAV-CMVsiR^{TNF-α} (high) group, had higher colon mucosal integrity, shallower immune cell infiltration, and significantly less colonic crypt abscesses and colon congestion and bleeding compared to the AAV-CMV-scrR group.

The above experiments show that the use of AAV with high affinity for the liver to encapsulate CMVsiR^{TNF-α}can achieve long-term expression of TNF-α siRNA and long-term TNF-α silencing, and can alleviate colitis to a certain extent, with great drug potential and clinical research value.

In the second experiment, we set up three experimental groups and two control groups. Wherein, the experimental groups were AAV-CMV-siR^{T+B+I} (low) group, AAV-CMV-siR^{T+B+I} (medium) group, and AAV-CMV-siR^{T+B+I} (high) group; and the control groups were Normal group and AAV-CMV-scrR group.

The AAV-CMV-siR^{T+B+I} (low) group, AAV-CMV-siR^{T+B+I} (medium) group, and AAV-CMV-siR^{T+B+I} (high) group used AAV-5 type adeno-associated virus with high affinity for the liver to encapsulate the TNF-α siRNA, B7-siRNA and Integrin α4 siRNA element tandem drug delivery system (AAV-CMV-siR^{T+B+I}). Mice were injected with 25 µL, 50 µL and 100 µL of AAV solution with a titer of 10¹² V g/ml through the tail vein.

The in vivo expression of the AAV system was monitored by small animal in vivo imaging. The results are shown in Figure 26A. It can be seen that after 3 weeks, the AAV system was stably expressed in the body, especially in the liver, and that the expression of the AAV system has a dose-dependent effect.

DSS-induced chronic colitis model was then constructed, during which weights was recorded every two days. The results are shown in Figure 26B. It can be seen that the AAV-encapsulated CMV-siR^{T+B+I} system can reduce the weight loss of mice with chronic colitis, and the mice in the three experimental groups had a significantly faster weight recovery than the AAV-CMV-scrR group during the inflammation remission period.

At the end of the tenth week of model construction, the in vivo expression of the AAV system was monitored by small animal in vivo imaging, and then the mice were sacrificed for observation of the colon. The results are shown in Figure 26C. It can be seen that the redness and swelling of the colon of mice in the AAV-CMV-siR^{T+B+I} (low) group, AAV-CMV-siR^{T+B+I} (medium) group, and AAV-CMV-siR^{T+B+I} (high) group were alleviated to varying degrees, and the shortening of colon length caused by chronic inflammation also improved to varying degrees, wherein the improvement of inflammation in the AAV-CMV--siR^{T+B+I} (high) group was the most significant.

The disease index of mice in each group was scored and counted, and the results are shown in Figure 27A. It can be seen that the disease index of mice in the AAV-CMV--siR^{T+B+I} (high) group was smaller than that of AAV-CMV-siR^{T+B+I} (low) group, AAV-CMV-siR^{T+B+I} (medium) group and AAV-CMV-scrR group.

The levels of TNF-α siRNA, B7 siRNA and integrin α4 siRNA were detected in mouse plasma, and the results are shown in Figure 27B, Figure 27C, and Figure 27D. It can be seen that AAV-encapsulated CMV-siR^{T+B+I} system produced a certain amount of siRNA in mouse plasma with a dose-dependent effect.

The levels of TNF-α siRNA, B7 siRNA and integrin α4 siRNA were detected in mouse liver, and the results are shown in Figure 27E, Figure 27F, and Figure 27G. It can be seen that the AAV-encapsulated CMV-siR^{T+B+I} system produced a certain amount of siRNA in mouse liver with a dose-dependent effect.

The levels of TNF-α siRNA, B7 siRNA and integrin α4 siRNA were detected in mouse colon, and the results are shown in Figure 28A, Figure 28B, and Figure 28C. It can be seen that the AAV-encapsulated CMV-siR^{T+B+I} system produced a certain amount of siRNA in mouse colon with a dose-dependent effect.

The levels of TNF-α mRNA, B7 mRNA and integrin α4 mRNA were detected in mouse colon, and the results are shown in Figure 28D, Figure 28E, and Figure 28F. It can be seen that the AAV-encapsulated CMV-siR^{T+B+I} system significantly reduced expression of TNF-α mRNA, B7 mRNA and integrin α4 mRNA.

HE staining and pathological scoring statistics were performed on mouse colon sections, and the results are shown in Figure 29A and Figure 29B. It can be seen that mice in the experimental groups, especially mice in the AAV-CMV-siR^{T+B+I} (high) group, had higher colon mucosal integrity, shallower immune cell infiltration, and significantly less colonic crypt abscesses and colon congestion and bleeding compared to the AAV-CMV-scrR group.

The above experiments show that the use of AAV with high affinity for the liver to encapsulate CMV-siR^{T+B+I} can achieve long-term expression of TNF-α siRNA, B7 siRNA and integrinα4 siRNA and multiple target gene silencing, and can significantly alleviate the degree of colon inflammation, with great drug potential and clinical research value.

The sequences involved above are specifically shown in the table below.

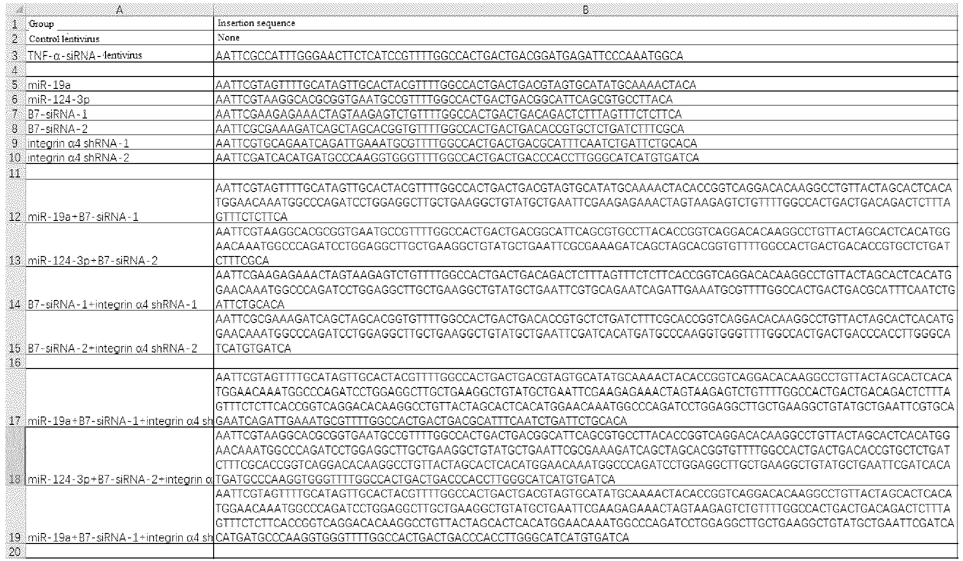

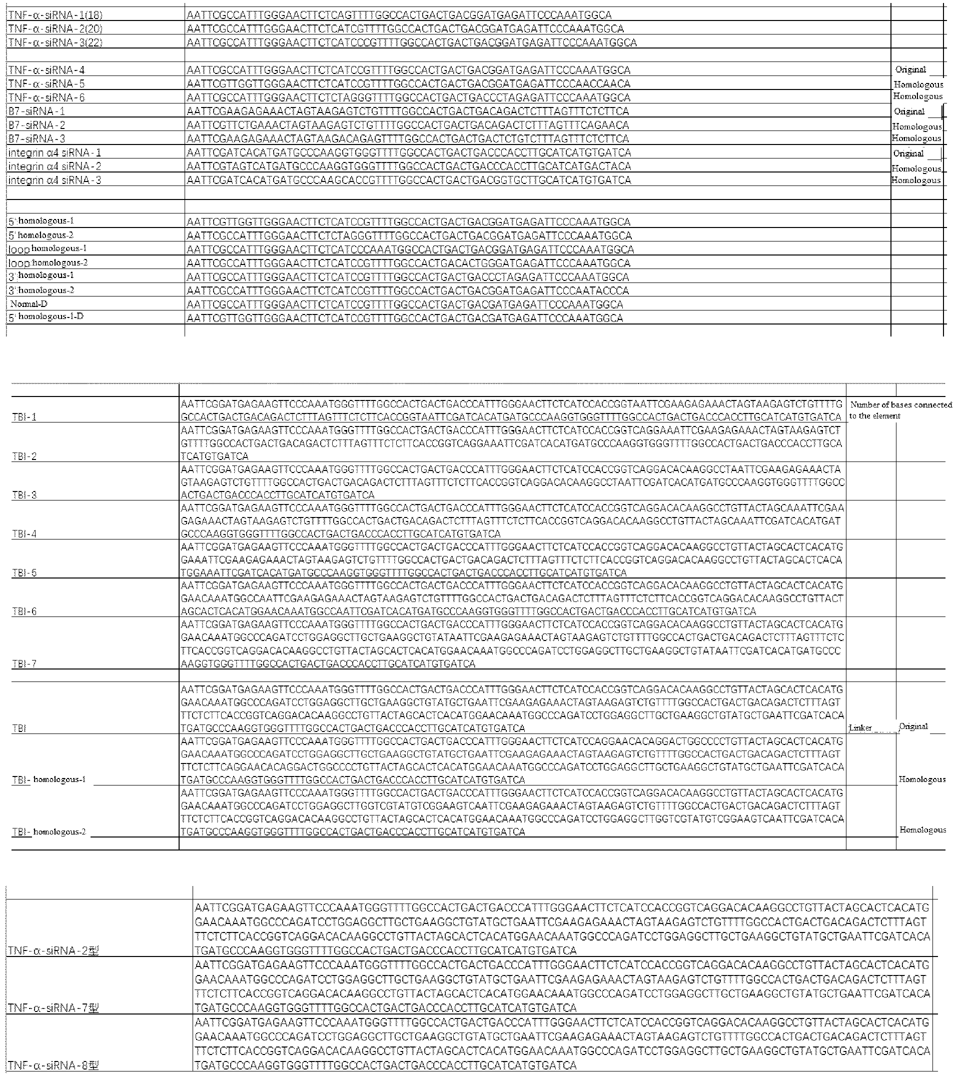

### Example 9

The plasmid CMV-siR^{miR-21} carrying the miR-21 siRNA that inhibits miR-21 expression and the plasmid CMV-siR^{TGF-β1} carrying the TGF-β1 siRNA that inhibits TGF-β1 expression were constructed according to the method described in Example 2. Wherein, miR-21 siRNA has the antisense strand of miR-21, and TGF-β1 siRNA has the nucleotide sequence of ACGGAAAUAACCUAGAUGGGC (SEQ ID NO. 13).

In addition, the plasmid CMV-siR^{miR-21+TGF-β1} carrying both miR-21 siRNA and TGF-β1 siRNA was constructed according to the method described in Example 2.

In this example, 8 experimental groups and 3 control groups were set up. The experimental groups were Anti-miR-21 (1 mg/kg) group, Anti-miR-21 (5 mg/kg) group, Anti-miR-21 (10 mg/kg) group, TGF-β1 siRNA (1 mg/kg) group, TGF-β1 siRNA (5 mg/kg) group, TGF-β1 siRNA (10 mg/kg) group, Anti-miR-21+TGF-β1 siRNA (10 mg/kg) group, Pirfenidone (300 mg/kg) group; and the control group were Normal group, PBS group and scrRNA group.

Among them, mice with pulmonary fibrosis in the Anti-miR-21 (1 mg/kg) group, Anti-miR-21 (5 mg/kg) group, and Anti-miR-21 (10 mg/kg) group were injected into the tail vein with 1 mg/kg, 5 mg/kg, 10 mg/kg of miR-21 siRNA plasmid; mice with pulmonary fibrosis in TGF-β1 siRNA (1 mg/kg) group, TGF-β1 siRNA (5 mg/kg) group, TGF-β1 siRNA (10 mg/kg) group were injected into the tail vein with 1 mg/kg, 5 mg/kg, 10 mg/kg of TGF-β1 siRNA plasmid; mice with pulmonary fibrosis in Anti-miR-21+TGF-β1 siRNA (10 mg/kg) group were injected into the tail vein with 10 mg/kg Anti-miR-21 and TGF-β1 siRNA plasmids; and mice with pulmonary fibrosis in Pirfenidone (300 mg/kg) group was injected into the tail vein with 300 mg/kg Pirfenidone. The Normal group was the normal control group, and mice with pulmonary fibrosis in the PBS group and the scrRNA group were injected into the tail vein with PBS solution and control plasmid, respectively.

The hydroxyproline content of mice in each group was detected respectively, and the results are shown in Figure 30. Hydroxyproline is the main component of collagen, and its content reflects the degree of pulmonary fibrosis. As can be seen from Figure 30, mice in the Anti-miR-21 (5 mg/kg) group, Anti-miR-21 (10 mg/kg) group, TGF-β1 siRNA (10 mg/kg) group, and Anti-miR-21+TGF-β1 siRNA (10 mg/kg) group had relatively low hydroxyproline content, indicating that their pulmonary fibrosis was inhibited.

Fluorescent staining was performed on the lung of mice in each group, and the results are shown in Figure 31. In the figure, the green part represents type I collagen (Collagen I), the red part represents α-SMA, and the blue part represents DAPI. It can be seen that the mice in the PBS group and scrRNA group had more type I collagen and α-SMA, while mice in the experimental group had relatively less type I collagen and α-SMA, and in particular, mice in the Anti-miR-21 (5 mg/kg) group, Anti-miR-21 (10 mg/kg) group, Anti-miR-21+TGF-β1 siRNA (10 mg/kg) group had almost no expression of type I collagen and α-SMA.

Masson's trichrome staining was performed on the lung of mice in each group, and the results are shown in Figure 32. It can be seen that the alveolar space of mice in the PBS group and scrRNA group was severely damaged, resulting in pulmonary interstitial collagen, while the experimental groups significantly alleviated these conditions.

H&E staining was performed on the lung of mice in each group, and the results are shown in Figure 33. It can be seen that mice in the PBS group and scrRNA group had widened alveolar space, infiltrated inflammatory cells and damaged alveolar structure, while the lung tissue of the experimental groups was relatively normal.

The TGF-β1 protein level and TGF-β1 mRNA level were detected in mice in the Normal group, PBS group, scrRNA group, TGF-β1 siRNA (1 mg/kg) group, TGF-β1 siRNA (5 mg/kg) group, TGF-β1 siRNA (10 mg/kg) group, and Pirfenidone (300 mg/kg) by western blot, and the results are shown in Figure 34A-Figure 34C. It can be seen that the TGF-β1 protein level and TGF-β1 mRNA level of mice in the TGF-β1 siRNA (10 mg/kg) group were the lowest, showing that after tail vein injection of the corresponding siRNA expression plasmid, TGF-β1 can be successfully delivered to the lung to function.

The relative miR-21 level was detected in mice in the Normal group, PBS group, scrRNA group, Anti-miR-21 (1 mg/kg) group, Anti-miR-21 (5 mg/kg) group, and Anti-miR-21 (10 mg/kg) group, and the results are shown in Figure 34D. It can be seen that the relative miR-21 level of mice in the Anti-miR-21 (10 mg/kg) group was the highest, showing that after tail vein injection of the corresponding antisense chain expression plasmid, the antisense chain of miR-21 can be successfully delivered to the lung to function.

The above experiments show that the use of the plasmid with high affinity for the liver to encapsulate CMV-siR^{miR-21}, CMV-siR^{TGF-β1}, and CMV-siR^{miR-21+TGF-β1} can significantly alleviate the degree of pulmonary fibrosis, with great drug potential and clinical research value.

Plasmids comprising sequence fragments of three different 5' flanking sequences, loop sequences and 3' flanking sequences also have in vivo enrichment, self-assembly and therapeutic effects on pulmonary fibrosis. The sequences include:
1. Three 5' flanking sequences with more than 80% homology;
2. Three loop sequences with more than 80% homology;
3. Three 3' flanking sequences with more than 80% homology. The sequences are specifically shown in the table below.

| | |
|---|---|
| 5' flanking sequence-1 | CTGGAGGCTTGCTGAAGGCTGTATGCTGAATTCG |
| 5' flanking sequence-2 | CTGGAGGCTTGCTGAAGGCTGTATGCTGTTAACG |
| 5' flanking sequence-3 | CTGGAGGCTTGCTGAAGGCTGTATGCTGGCAACG |
| loop-1 | GTTTTGGCCACTGACTGAC |
| loop-2 | GTTAAGGCCACTGACTGAC |
| loop-3 | GAATTGGCCACTGACTGAC |
| 3' flanking sequence-1 | CACCGGTCAGGACACAAGGCCTGTTACTAGCACTCACATGGA ACAAATGGCC |
| 3' flanking sequence-2 | CAGGCCTCAGGACACAAGGCCTGTTACTAGCACTCACATGGA ACAAATGGCC |
| 3' flanking sequence-3 | CAGCGCTCAGGACACAAGGCCTGTTACTAGCACTCACATGGA ACAAATGGCC |

In the case where the RNA plasmid delivery system carries multiple circuits, the linker between adjacent circuits is sequence 2, which can be composed of multiple bases. In this case, the plasmid also has an enrichment effect after injection. Sequence 2 is specifically shown in the table below.

| | |
|---|---|
| 20 bases | TGGCCGCACTCGAGGTAGTG |

| | |
|---|---|
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

The sequences are specifically shown in the table below, in which sequence 4-1 is the above-mentioned sequence 4, and sequences 4-2/4-3/4-4 are homologous sequences with more than 80% homology to sequence 4-1.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

The plasmid delivery system comprising RNA sequence of lengths 18, 19, and 21 have in vivo enrichment, self-assembly, and therapeutic effects on pulmonary fibrosis.

The sequences are specifically shown in the table below.

| | |
|---|---|
| Sequence of 21 nt | TATCTTTGCTGTCACAAGAGC |
| Sequence of 19 nt | TAAAGTCAATGTACAGCTG |
| Sequence of 18 nt | TTCATGTCATGGATGGTG |

The plasmid delivery system, in the case of carrying the RNA fragment, has in vivo enrichment effect, effect of spontaneously forming into a complex and therapeutic effects on pulmonary fibrosis. The grouping of RNA fragments includes but is not limited to:
1) siRNA1 alone, siRNA2 alone, shRNA1 alone, shRNA2 alone, miRNA1 alone, and miRNA2 alone;
2) RNA fragments containing any two RNA sequences in the above 1); and
3) RNA fragments containing any three RNA sequences in the above 1).

The sequences are specifically shown in the table below.

| | |
|---|---|
| siRNA-1 precursor | TATCTTTGCTGTCACAAGAGCGTTTTGGCCACTGACTGACGCTCTTGTC AGCAAAGATAA |
| siRNA-2 precursor | TAAAGTCAATGTACAGCTGGTTTTGGCCACTGACTGACCAGCTGTATTG ACTTTA |
| shRNA-1 precursor | TATCTTTGCTGTCACAAGAGCGTTTTGGCCACTGACTGACGCTCTTGTG ACAGCAAAGATA |
| shRNA-2 precursor | TAAAGTCAATGTACAGCTGGTTTTGGCCACTGACTGACCAGCTGTACA TTGACTTTA |
| miRNA-1 | miR-21 |
| miRNA-2 | miR-34a |

### Example 10

The virus AAV-CMV-siR^{miR-21} carrying miR-21 siRNA that inhibits miR-21 expression and the virus AAV-CMVsiR^{TGF-β1} carrying TGF-β1 siRNA that inhibits TGF-β1 expression were constructed according to the methods described in Example 5 and Example 9. Wherein, miR-21 siRNA has the antisense strand of miR-21, and TGF-β1 siRNA has the nucleotide sequence of ACGGAAAUAACCUAGAUGGGC (SEQ ID NO. 13). In addition, the virus AAV-CMV-MIX, that is, AAV-CMV-siR^{miR-21+TGF-β1} carrying both miR-21 siRNA and TGF-β1 siRNA was constructed.

Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

Mice were then selected for modeling. After the modeling was successful, PBS buffer/AAV-scrR/AAV-anti-miR21/AAV-TGF-β1 siRNA/AAV-MIX (10 mg/kg) was injected into mice to obtain PBS group/AAV-scrR group/AAV-anti-miR21 group/AAV-TGF-β1 siRNA group/AAV-MIX group, respectively.

The relative TGF-β1 mRNA level was detected in mice in the Normal group, PBS group, AAV-scrR group, and AAV-TGF-β1 siRNA group, and the results are shown in Figure 35B. It can be seen that the relative TGF-β1 mRNA level of mice in the AAV-TGF-β1 siRNA group was relatively low.

The relative miR21 mRNA level was detected in mice in the Normal group, PBS group, AAV-scrR group, and AAV-anti-miR21 group, and the results are shown in Figure 35C. It can be seen that the relative miR21 mRNA level of mice in the AAV-anti-miR21 group was relatively low.

The hydroxyproline content of mice in each group was detected, and the results are shown in Figure 35A. It can be seen that mice in the PBS group and the AAV-scrR group had the highest hydroxyproline content, while mice in the AAV-anti-miR21 group, AAV-TGF-β1 siRNA group, and AAV-MIX group all had low hydroxyproline content, indicating that pulmonary fibrosis was inhibited in mice in the AAV-anti-miR21 group, AAV-TGF-β1 siRNA group, and AAV-MIX group.

Further, the viral vector can also comprise a flanking sequence, a compensation sequence and a loop sequence that facilitate the correct folding and expression of the circuit, and the flanking sequence comprises 5' flanking sequence and 3' flanking sequence; the viral vector comprises a circuit selected from the group consisting of 5' promoter-5' flanking sequence-RNA fragment-loop sequence-compensation sequence-3' flanking sequence, 5'-promoter-targeting tag, and 5' promoter-targeting tag-5' flanking sequence-RNA fragment-loop sequence-compensation sequence-3' flanking sequence, or a combination thereof.

Wherein, the 5' flanking sequence preferably has a sequence that is identical to or more than 80% homologous with ggatcctggaggcttgctgaaggctgtatgctgaattc, including sequences with 85%, 90%, 92%, 95%, 98%, or 99% homology with ggatcctggaggcttgctgaaggctgtatgctgaattc.

The loop sequence preferably has a sequence that is identical to or more than 80% homologous with gttttggccactgactgac, including sequences with 85%, 90%, 92%, 95%, 98%, 99% homology with gttttggccactgactgac.

The 3' flanking sequence preferably has a sequence that is identical to or more than 80% homologous with accggtcaggacacaaggcctgttactagcactcacatggaacaaatggcccagatctggccgcactcgag, including sequences with 85%, 90%, 92%, 95%, 98%, 99% homology with accggtcaggacacaaggcctgttactagcactcacatggaacaaatggcccagatctggccgcactcgag.

The compensation sequence is the reverse complementary sequence of the RNA fragment with any 1-5 bases deleted. In the case where the RNA fragment contains only one RNA sequence, the compensation sequence may be the reverse complementary sequence of the RNA sequence with any 1-5 bases deleted.

Preferably, the compensation sequence is the reverse complementary sequence of the RNA fragment with any 1-3 bases deleted. In the case where the RNA fragment contains only one RNA sequence, the compensation sequence may be the reverse complementary sequence of the RNA sequence with any 1-3 bases deleted.

More preferably, the compensation sequence is the reverse complementary sequence of the RNA fragment with any 1-3 contiguous bases deleted. In the case where the RNA fragment contains only one RNA sequence, the compensation sequence may be the reverse complementary sequence of the RNA sequence with any 1-3 contiguous bases deleted.

Most preferably, the compensation sequence is the reverse complementary sequence of the RNA fragment with the base at position 9 and/or 10 deleted. In the case where the RNA fragment contains only one RNA sequence, the compensation sequence may be a reverse complementary sequence of the RNA sequence with the base at position 9 and/or 10 deleted. Deleting the base at position 9 and/or 10 has the best effect.

It should be noted that the above-mentioned flanking sequence, compensation sequence, and loop sequence are not randomly selected, but are determined based on a large number of theoretical studies and experiments. With the cooperation of the above-mentioned certain flanking sequence, compensation sequence, and loop sequence, the expression rate of RNA fragments can be maximized.

Adenoviral vectors comprising three homologous sequences also have in vivo enrichment, self-assembly and therapeutic effects on pulmonary fibrosis. The sequences are grouped as follows:
1. Three 5' flanking sequences with more than 80% homology;
2. Three loop sequences with more than 80% homology;
3. Three 3' flanking sequences with more than 80% homology. The sequences are specifically shown in the table below.

| | |
|---|---|
| 5' flanking sequence-1 | CTGGAGGCTTGCTGAAGGCTGTATGCTGAATTCG |
| 5' flanking sequence-2 | CTGGAGGCTTGCTGAAGGCTGTATGCTGTTAACG |
| 5' flanking sequence-3 | CTGGAGGCTTGCTGAAGGCTGTATGCTGGCAACG |
| loop-1 | GTTTTGGCCACTGACTGAC |
| loop-2 | GTTAAGGCCACTGACTGAC |
| loop-3 | GAATTGGCCACTGACTGAC |
| 3' flanking sequence-1 | CACCGGTCAGGACACAAGGCCTGTTACTAGCACTCACATGGA ACAAATGGCC |
| 3' flanking sequence-2 | CAGGCCTCAGGACACAAGGCCTGTTACTAGCACTCACATGGA ACAAATGGCC |
| 3' flanking sequence-3 | CAGCGCTCAGGACACAAGGCCTGTTACTAGCACTCACATGGA ACAAATGGCC |

In the case where the viral vector carries two or more circuits, adjacent gene circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

In the case where the adenoviral vector carries multiple circuits and adjacent circuits are linked via sequence 1-sequence 2-sequence 3, with sequence 2 containing multiple bases, the constructed delivery system also has in vivo enrichment, self-assembly and therapeutic effects on pulmonary fibrosis.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

In the case where the linker is sequence 4, or sequence with more than 80% homology with sequence 4, the constructed delivery system also has in vivo enrichment, self-assembly and therapeutic effects on pulmonary fibrosis, wherein sequence 4-1 is the above-mentioned sequence 4, and sequences 4-2/4-3/4-4 are homologous sequences to sequence 4-1. The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

The RNA fragment may comprise one, two or more RNA sequences with medical significance. The RNA sequence can be expressed in the target receptor, and the compensation sequence cannot be expressed in the target receptor. The RNA sequence may be an siRNA sequence, shRNA sequence or miRNA sequence, preferably an siRNA sequence.

The length of an RNA sequence is 15-25 nucleotides (nt), preferably 18-22 nt, such as 18 nt, 19 nt, 20 nt, 21 nt, or 22 nt. The range of the sequence length is not chosen arbitrarily but was determined after repeated trials. A large number of experiments have proved that in the case where the length of the RNA sequence is less than 18 nt, especially less than 15 nt, the RNA sequence is mostly invalid and will not play a role. In the case where the length of the RNA sequence is greater than 22 nt, especially greater than 25 nt, not only does the cost of the circuit increase greatly, but the effect is no better than that of an RNA sequence with a length of 18-22 nt, and the economic benefits are poor. Therefore, in the case where the length of the RNA sequence is 15-25 nt, especially 18-22 nt, the cost and function can be balanced with the best effect.

The constructed delivery system constructed with RNA sequence of lengths 18, 19, and 21 have in vivo enrichment, self-assembly, and therapeutic effects on pulmonary fibrosis.

The sequences are specifically shown in the table below.

| | |
|---|---|
| Sequence of 21 nt | TATCTTTGCTGTCACAAGAGC |
| Sequence of 19 nt | TAAAGTCAATGTACAGCTG |
| Sequence of 18 nt | TTCATGTCATGGATGGTG |

The viral vector system, in the case of carrying multiple RNA fragments, has in vivo enrichment, self-assembly and therapeutic effects on pulmonary fibrosis, as shown in Figure 5. The grouping of RNA fragments includes but is not limited to:
1) Use of six RNAs alone: siRNA-1 alone, siRNA-2 alone, shRNA-1 alone, shRNA-2 alone, miRNA-1 alone, and miRNA-2 alone;
2) RNA fragments containing any two RNA sequences: siRNA-1+siRNA-2, shRNA-1+shRNA-2, miRNA-1+miRNA-2; and
3) RNA fragments containing any three RNA sequences: siRNA-1+siRNA-2+shRNA-1, siRNA-1+siRNA-2+shRNA-2, siRNA-1+siRNA-2+miRNA-1, siRNA-1+siRNA-2+miRNA-2.

The RNA sequences are specifically shown in the table below.

| | |
|---|---|
| siRNA-1 precursor | TATCTTTGCTGTCACAAGAGCGTTTTGGCCACTGACTGACGCTCTTGTC AGCAAAGATAA |
| siRNA-2 precursor | TAAAGTCAATGTACAGCTGGTTTTGGCCACTGACTGACCAGCTGTATTG ACTTTA |
| shRNA-1 precursor | TATCTTTGCTGTCACAAGAGCGTTTTGGCCACTGACTGACGCTCTTGTG ACAGCAAAGATA |
| shRNA-2 precursor | TAAAGTCAATGTACAGCTGGTTTTGGCCACTGACTGACCAGCTGTACA TTGACTTTA |
| miRNA-1 | miR-21 |
| miRNA-2 | miR-34a |

### Example 11

The effect of the plasmid carrying RVG targeting peptide prepared according to Example 2 on glioblastoma was detected.

In the first experiment, 5 experimental groups and 3 control groups were set up. The experimental groups were CMV-siR^{E} group, CMV-siR^{T} group, CMV-RVG-siR^{E+T} group, CMV-siR^{E+T} group, CMV-Flag-siR^{E+T} group, where "E" represents EGFR, "T" represents TNC, and the control groups were PBS group, CMV-scrR group, and CMV-Flag-scrR group. The specific experimental protocol is shown in Figure 36A.

The expression levels of CD63 protein and siRNA of mice in each group were detected. The results are shown in Figure 36B-Figure 36D, indicating that intravenous injection of CMV-RVG-siR^{E+T} can deliver siRNA to the brain.

In the second experiment, 2 experimental groups and 2 control groups were set up. The experimental groups were CMV-RVG-siR^{E} group and CMV-RVG-siR^{E+T} group, and the control groups were PBS group and CMV-scrR group.

The specific experimental protocol is shown in Figure 37A. Mice were selected and injected with glioblastoma cells (U-87 MG-Luc cells). From the 7th day to the 21st day, mice were injected with PBS buffer/CMV-scrR/CMV-RVG-siR^{E}/CMV-RVG-siR^{E+T} (5 mg/kg) every two days for treatment, and survival analysis and tumor evaluation were performed on the mice. On days 7, 14, 28 and 35, the mice were subjected to BLI in vivo imaging detection.

As shown in Figure 37B, the figure shows the comparison of mouse BLI in vivo imaging detection on days 7, 14, 28, and 35. It can be seen that mice in the CMV-RVG-siR^{E+T} group had the most significant inhibitory effect on glioblastoma.

As shown in Figure 37C, the figure shows the comparison of the survival rates of mice in each group. It can be seen that mice in the CMV-RVG-siR^{E+T} group had the longest survival time.

As shown in Figure 37D, the figure shows the comparison of fluorescence images of mice in each group, which was obtained using luciferase by in vivo bioluminescence imaging, where the ordinate reflects the intensity of lucifer fluorescence signal. Because the gene had been artificially integrated into the implanted tumors, the figure is able to reflect tumor progression. It can be seen that the tumors of mice in the control groups developed relatively rapidly, while the tumors of mice in the experimental group were suppressed to a great extent.

As shown in Figure 37E, the figure shows the comparison of the relative siRNA of mice in each group. It can be seen that the level of EGFR siRNA in mice in CMV-RVG-siR^{E} group was high, and the levels of both EGFR siRNA and TNC siRNA in mice in CMVRVG-siR^{E+T} were high.

As shown in Figure 37F, the figure shows the comparison of western blot results of mice in each group. It can be seen that mice in the PBS group, CMV-scrR group, and CMV-RVG-siR^{E} group had high levels of EGFR and TNC genes.

The above experimental data demonstrate that intravenous injection of CMV-RVG-siR^{E+T} plasmid can deliver siRNA to the brain and inhibit the growth of glioblastoma.

The brains of mice in each group were subjected to immunohistochemical staining, and the proportion of EGFR, TNC, and PCNA staining in each visual field was counted. The results are shown in Figures 38. It can be seen that mice in the CMV-RVG-siR^{E+T} group had the lowest EGFR, TNC, and PCNA contents in the brain, and mice in the CMV-RVG-siR^{E} group had low EGFR and PCNA contents in the brain. It can be seen that the injection of CMV-RVG-siR^{E} plasmid can inhibit the expression of EGFR and PCNA in the brain, and the injection of CMV-RVG-siR^{E+T} plasmid can inhibit the expression of EGFR, TNC and PCNA in the brain.

The plasmid does have the effect of enriching in vivo and spontaneously forming a complex structure containing an RNA fragment. The present invention provides a random group of experimental data of plasmids carrying four of the circuits, in which the adjacent circuits are linked via sequence 1-sequence 2-sequence 3, with sequence 2 of 5 bases, 10 bases, 20 bases, 30 bases, 40 bases, 50 bases or 80 bases. The enrichment and self-assembly effects of the plasmids were verified through experiments.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the plasmid carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

In order to prove that the plasmid does have the effects of in vivo enrichment and spontaneously forming a complex structure containing an RNA fragment, the present invention provides a random group of experimental data of plasmids containing the linker of sequence 4 or at least two sequences with a homology of more than 80% to sequence 4. The enrichment and self-assembly effects of the plasmids were verified through experiments.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

### Example 12

The effects of viruses carrying RVG targeting peptide prepared as described in Examples 2 and 5 on glioblastoma were detected.

Material: AAV-CMV-RVG-siR^{E} and AAV-CMV-RVG-siR^{E+T}. Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

Mice were selected and injected with glioblastoma cells (U-87 MG-Luc cells). From the 7th day to the 21st day, mice were injected with PBS buffer/AAV CMV scrR/AAV CMV RVG-siR^{E}/AAV-CMV-RVG-siR^{E+T} (5 mg/kg) every two days for treatment, to obtain PBS group/AAV-scrR group/AAV-CMV-RVG-siR^{E} group/AAV-CMVRVG-siR^{E+T} group, respectively.

Survival analysis was performed on mice in each group, and the survival rate of mice in each group was counted at 20 days, 40 days, 60 days, and 80 days after receiving treatment. The results are shown in Figure 39A. It can be seen that mice in the AAV-CMVRVG-siR^{E+T} group had the longest survival time, followed by the AAV-CMV-RVG-siR^{E} group.

Tumor evaluation was performed on mice in each group. Specifically, BLI in vivo imaging was performed on mice on days 7, 14, 28, and 35. The results are shown in Figure 39B. It can be seen that the AAV-CMV-RVG-siR^{E+T} group had the most significant inhibitory effect on glioblastoma in mice.

In order to prove that the viral vector does have the effects of in vivo enrichment and self-assembly, the present invention provides a random group of experimental data of viral vectors carrying four of the circuits, in which the adjacent circuits are linked via sequence 1-sequence 2-sequence 3, with sequence 2 of 5 bases, 10 bases, 20 bases, 30 bases, 40 bases, 50 bases or 80 bases. The enrichment and self-assembly effects of the viral vectors were verified through experiments.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

In order to prove that the viral vector does have the effects of in vivo enrichment and self-assembly, the present invention provides a random group of experimental data of viral vectors containing the linker of sequence 4 or at least two sequences with a homology of more than 80% to sequence 4. The enrichment and self-assembly effects of the viral vectors were verified through experiments.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

### Example 13

The plasmid CMV-siR^{P} carrying PTP1B siRNA that inhibits PTP1B expression, and the plasmid CMV-RVG-siR^{P} carrying targeting peptide RVG and PTP1B siRNA were constructed according to the method described in Example 2. Similarly, the virus AAV-CMV-siR^{P} carrying PTP1B siRNA that inhibits PTP1B expression and the virus AAV-CMV-RVG-siR^{P} carrying targeting peptide RVG were constructed according to the method described in Example 5. Wherein, the siRNA of PTP1B gene has the nucleotide sequence of UGAUAUAGUCALTUAUCLTUCLTU (SEQ ID NO. 14).

In the first experiment, 2 experimental groups and 1 control group were set up. The experimental groups were CMV-siR^{P} group and CMV-RVG-siR^{P} group, and the control group was CMV-scrR group, where "P" means PTP1B.

Mice in the CMV-siR^{P} group, CMV-RVG-siR^{P} group, and CMV-scrR group were injected with 5 mg/kg CMV-siR^{P} plasmid, CMV-RVG-siR^{P} plasmid, and CMV-scrR plasmid respectively, and then fluorescence microscope images of the hypothalamus and liver of mice in each group were obtained respectively. The results are shown in Figure 40 that PTP1B siRNA can be delivered to the hypothalamus.

In the second experiment, 2 experimental groups and 2 control groups were set up. The experimental groups were CMV-siR^{P} group the comparison of the body weights of mice in each group. It can be seen that the body weight of mice in the CMV-RVG-siR^{P} group was the most stable.

As shown in Figure 41C, the figure shows the comparison of the weights of the epididymal fat pads of mice in each group. It can be seen that the weight of the epididymal fat pads of mice in the CMV-RVG-siR^{P} group was the lightest.

The oxygen consumption, respiratory exchange ratio, activity volume, and heat production of differently treated mice were continuously detected for 72 h using metabolic cages, and then the average values were drawn for statistical analysis. The results are shown in Figure 41D-Figure 41G. The results show that the CMV-RVG-siR^{P} plasmid can effectively increase the oxygen consumption of mice, which means that mice in this group were in a high-energy metabolic state compared with mice in other groups. Normal mice mainly use glucose as their own energy source. CMV-RVG-siR^{P} plasmid can reduce the respiratory exchange ratio of mice, which means that mice in this group were more inclined to use protein as their own energy source compared with mice in other groups. The activity level of mice injected with CMV-RVG-siR^{P} plasmid was significantly increased. Moreover, the heat production of mice in the CMV-RVG-siR^{P} group was also significantly increased.

As shown in Figure 41H, this figure shows the comparison of the initial body weight curves of mice in each group. It can be seen that the mice in the CMV-RVG-siR^{P} group had the lightest body weight.

As shown in Figure 41I, this figure shows the comparison of the initial food intake curves of mice in each group. It can be seen that the mice in the CMV-RVG-siR^{P} group had the least food intake.

As shown in Figure 41J, this figure shows the comparison of serum leptin levels in mice of each group. It can be seen that the serum leptin levels of the mice in the CMV-RVG-siR^{P} group were the lowest.

As shown in Figure 41K, this figure shows the comparison of western blot results of mice in each group. It can be seen that the PTP1B protein content of mice in the CMV-RVG-siR^{P} group was the lowest.

As shown in Figure 41L, this figure shows the comparison of blood glucose change curves of mice in each group. It can be seen that the blood glucose content of mice in the CMV-RVG-siR^{P} group was the lowest.

As shown in Figure 41M, this figure shows the comparison of the basal glucose change curves of mice in each group. It can be seen that the basal glucose content of mice in the CMV-RVG-siR^{P} group was the lowest.

From the above experiments, it can be concluded that intravenous injection of CMV-RVG-siR^{P} plasmid can reduce obesity in obese mouse models.

The serum total cholesterol (TC), triglycerides (TG), and low-density lipoprotein (LDL) of mice in each group were measured, and the results are shown in Figure 42A. It can be seen that the mice in the CMV-RVG-siR^{P} group had the lowest TC, TG, and LDL contents.

The body length of mice in each group was measured, and the results are shown in Figure 42B. It can be seen that the body length of mice in the four groups was almost the same.

The HFD food intake of mice in each group was counted, and the results are shown in Figure 42C. It can be seen that the HFD food intake of mice in the four groups was almost the same.

The liver tissue of mice in each group was collected after treatment and compared with the normal control. The results are shown in Figure 31D. Obvious pathological characteristics of fatty liver can be seen in the liver tissue pathological sections of mice in the PBS group and CMV scrR group, while mice in the CMV-siR^{P} group had milder fatty liver.

The above experiments show that intravenous injection of CMV-RVG-siR^{P} plasmid can reduce fatty liver in obese mice.

In addition, C57BL/6 mice were selected and injected with PBS buffer/AAV-CMV-scrR/AAV-CMV-siR^{P}/AAV-CMV-RVG-siR^{P} 12 weeks later, and once every two days for 24 days to obtain PBS group/AAV-CMVscrR group/AAV-CMV-siR^{P} group/AAV-CMV-RVG-siR^{P} group, respectively. The changes in body weight, weight of epididymal fat pads, initial food intake, serum leptin content, blood sugar content, basal glucose content, serum total cholesterol (TC), triglyceride (TG), low-density lipoprotein protein (LDL), body length, and food intake of mice in each group were detected and counted. The results are as follows.

As shown in Figure 43A, the figure shows the comparison of the body weights of mice in each group. It can be seen that the body weight of mice in the AAV-CMV-RVG-siR^{P} group was the most stable.

As shown in Figure 43B, the figure shows the comparison of the weights of the epididymal fat pads of mice in each group. It can be seen that the weight of the epididymal fat pads of mice in the AAV-CMVRVG-siR^{P} group was the lightest.

As shown in Figure 43C, the figure shows the comparison of the initial food intake curves of mice in each group. It can be seen that mice in the AAV-CMV-RVG-siR^{P} group had the least food intake.

As shown in Figure 43D, this figure shows the comparison of serum leptin levels in mice of each group. It can be seen that the serum leptin levels of mice in the AAV-CMVRVG-siR^{P} group was the lowest.

As shown in Figure 43E, this figure shows the comparison of blood glucose change curves of mice in each group. It can be seen that the blood glucose level of mice in the AAV-CMV-RVG-siR^{P} group was the lowest.

As shown in Figure 43F, this figure shows the comparison of the basal glucose change curves of mice in each group. It can be seen that mice in the AAV-CMV-RVG-siR^{P} group had the lowest basal glucose content.

As shown in Figure 44A-Figure 44C, the three figures show the comparison of serum total cholesterol (TC), triglyceride (TG), and low-density lipoprotein (LDL) of mice in each group. It can be seen that mice in the AAV-CMVRVG-siR^{P} group had the lowest TC, TG, and LDL.

As shown in Figure 44D, the figure shows the comparison of the body lengths of the mice in each group, it can be seen that the body lengths of the mice in the four groups were almost the same.

As shown in Figure 44E, the figure shows the comparison of the HFD food intake of the mice in each group, it can be seen that the HFD food intake of mice in the four groups was almost the same.

The above experiments can demonstrate that AAV-CMV-siR^{P} and AAV-CMV-RVG-siR^{P} had inhibitory effect on obesity.

Adenoviral vectors comprising three homologous sequences also have in vivo enrichment, self-assembly and therapeutic effects on obesity, as shown in Figure 8. The sequences are grouped as follows:
1. Three 5' flanking sequences with more than 80% homology;
2. Three loop sequences with more than 80% homology;
3. Three 3' flanking sequences with more than 80% homology. The sequences are specifically shown in the table below.

| | |
|---|---|
| 5' flanking sequence-1 | CTGGAGGCTTGCTGAAGGCTGTATGCTGAATTCG |
| 5' flanking sequence-2 | CTGGAGGCTTGCTGAAGGCTGTATGCTGTTAACG |
| 5' flanking sequence-3 | CTGGAGGCTTGCTGAAGGCTGTATGCTGGCAACG |
| loop-1 | GTTTTGGCCACTGACTGAC |
| loop-2 | GTTAAGGCCACTGACTGAC |
| loop-3 | GAATTGGCCACTGACTGAC |
| 3' flanking sequence-1 | CACCGGTCAGGACACAAGGCCTGTTACTAGCACTCACATGGA ACAAATGGCC |
| 3' flanking sequence-2 | CAGGCCTCAGGACACAAGGCCTGTTACTAGCACTCACATGGA ACAAATGGCC |
| 3' flanking sequence-3 | CAGCGCTCAGGACACAAGGCCTGTTACTAGCACTCACATGGA ACAAATGGCC |

In the case where the viral vector carries two or more circuits, adjacent gene circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

In the case where the adenoviral vector carries multiple circuits and adjacent circuits are linked via sequence 1-sequence 2-sequence 3, with sequence 2 containing multiple bases, the constructed delivery system also has in vivo enrichment, self-assembly and therapeutic effects on obesity.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

In the case where the linker is sequence 4, or sequence with more than 80% homology with sequence 4, the constructed delivery system also has in vivo enrichment, self-assembly and therapeutic effects on obesity, wherein sequence 4-1 is the above-mentioned sequence 4, and sequences 4-2/4-3/4-4 are homologous sequences to sequence 4-1. The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

The RNA fragment may comprise one, two or more RNA sequences with medical significance. The RNA sequence can be expressed in the target receptor, and the compensation sequence cannot be expressed in the target receptor. The RNA sequence may be an siRNA sequence, shRNA sequence or miRNA sequence, preferably an siRNA sequence.

The length of an RNA sequence is 15-25 nucleotides (nt), preferably 18-22 nt, such as 18 nt, 19 nt, 20 nt, 21 nt, or 22 nt. The range of the sequence length is not chosen arbitrarily but was determined after repeated trials. A large number of experiments have proved that in the case where the length of the RNA sequence is less than 18 nt, especially less than 15 nt, the RNA sequence is mostly invalid and will not play a role. In the case where the length of the RNA sequence is greater than 22 nt, especially greater than 25 nt, not only does the cost of the circuit increase greatly, but the effect is no better than that of an RNA sequence with a length of 18-22 nt, and the economic benefits are poor. Therefore, in the case where the length of the RNA sequence is 15-25 nt, especially 18-22 nt, the cost and function can be balanced with the best effect.

The constructed delivery system constructed with RNA sequence of lengths 18, 20, and 21 have in vivo enrichment, self-assembly, and therapeutic effects on obesity.

The sequences are specifically shown in the table below.

| | |
|---|---|
| Sequence of 21 nt | CTAACTTCAGTGTCTGGACTC |
| Sequence of 20 nt | CTAACTTCAGTGTCTGGACT |
| Sequence of 18 nt | CTAACTTCAGTGTCTGGA |

The viral vector system, in the case of carrying multiple RNA fragments, has in vivo enrichment, self-assembly and therapeutic effects on obesity, as shown in Figure 6. The grouping of RNA fragments includes but is not limited to:
1) Use of six RNAs alone: siRNA-1 alone, siRNA-2 alone, shRNA-1 alone, shRNA-2 alone, miRNA-1 alone, and miRNA-2 alone;
2) RNA fragments containing any two RNA sequences: siRNA-1+siRNA-2, shRNA-1+shRNA-2, miRNA-1+miRNA-2; and
3) RNA fragments containing any three RNA sequences: siRNA-1+siRNA-2+shRNA-1, siRNA-1+siRNA-2+shRNA-2, siRNA-1+siRNA-2+miRNA-1, siRNA-1+siRNA-2+miRNA-2.

The RNA sequences are specifically shown in the table below.

| | |
|---|---|
| siRNA-1 precursor | CTGGAGGCTTGCTGAAGGCTGTATGCTGAATTCGCTAACTTCAGTGTCT GGACTCGTTTTGGCCACTGACTGACGAGTCCAGACTGAAGTTAGCACC GGTCAGGACACAAGGCCTGTTACTAGCACTCACATGGAACAAATGGCC |
| siRNA-2 precursor | CTGGAGGCTTGCTGAAGGCTGTATGCTGAATTCGTCTTGTCCATCAGTA AGAGGCGTTTTGGCCACTGACTGACGCCTCTTAGATGGACAAGACACC GGTCAGGACACAAGGCCTGTTACTAGCACTCACATGGAACAAATGGCC |
| shRNA-1 precursor | CTGGAGGCTTGCTGAAGGCTGTATGCTGAATTCGCTAACTTCAGTGTCT GGACTCGTTTTGGCCACTGACTGACGAGTCCAGACACTGAAGTTAGCA |
| | CCGGTCAGGACACAAGGCCTGTTACTAGCACTCACATGGAACAAATGG CC |
| shRNA-2 precursor | CTGGAGGCTTGCTGAAGGCTGTATGCTGAATTCGTCTTGTCCATCAGTA AGAGGCGTTTTGGCCACTGACTGACGCCTCTTACTGATGGACAAGACA CCGGTCAGGACACAAGGCCTGTTACTAGCACTCACATGGAACAAATGG CC |
| miRNA-1 | miR-193a-3p |
| miRNA-2 | miR-338-3p |

### Example 14

The plasmid CMV-siR^{mHTT} carrying mHTT siRNA that inhibits mHTT expression, and the plasmid CMV-RVG-siR^{P} carrying targeting peptide RVG and mHTT siRNA were constructed according to the method described in Example 2. Similarly, the virus AAV-CMV-siR^{mHTT} carrying mHTT siRNA that inhibits mHTT expression and the virus AAV-CMV-RVG-siR^{mHTT} carrying targeting peptide RVG were constructed according to the method described in Example 5. Wherein, the siRNA of mHTT gene has the nucleotide sequence of UAUGUUUUCACAUAUUGUCAG (SEQ ID NO. 15).

In the first experiment, 2 experimental groups and 2 control groups were set up. The experimental groups were CMV-siR^{mHTT} group and CMV-RVG-siR^{mHTT} group, and the control groups were PBS group and CMV-scrR group.

The experimental protocol is shown in Figure 45A. Mice with Huntington's disease in the CMV-siR^{mHTT} group, CMV-RVG-siR^{mHTT} group, PBS group, and CMV-scrR group were injected with CMV-siR^{mHTT} plasmid, CMV-RVG-siR^{mHTT} plasmid, PBS solution, and CMV-scrR plasmid through the tail vein, respectively. The plasma exosomes were then isolated, labeled with PKH26 dye, and co-cultured with cells to observe the absorption of exosomes by cells.

As shown in Figure 45B, the figure shows the comparison of siRNA levels in plasma exosomes of mice in each group. It can be seen that the levels of siRNA in plasma exosomes of mice in the two experimental groups were higher.

After injecting plasmid/solution into mice in each group, plasma exosomes were extracted, labeled with PKH26, co-cultured with cells and photographed using a confocal microscope. The results are shown in Figure 45C, showing that siRNA-encapsulated exosomes entered cells.

After co-culturing the extracted plasma exosomes from mice in each group with cells, the changes in HTT protein levels and mRNA levels of mice in each group were detected. As shown in Figure 45D-Figure 45F, the results show that CMV-siR^{mHTT} and CMV-RVG-siR^{mHTT} can reduce HTT protein levels, indicating that siRNA assembled into exosomes can still exert gene silencing function.

After co-culturing the extracted plasma exosomes from mice in each group with cells, the aggregation of HTT protein of mice in each group was observed and counted. As shown in Figure 45G-Figure 45H, the results show that CMV-siR^{mHTT} and CMV-RVG-siR^{mHTT} can reduce the aggregation of pathological HTT proteins in the Huntington HTT aggregation cell model, indicating that siRNA assembled into exosomes can still exert gene silencing function and can effectively reduce the aggregation of mutant proteins.

The absolute siRNA expression levels in the liver, plasma, cortex, and striatum of mice in each group were detected respectively. As shown in Figure 46A, this figure shows the comparison of the absolute siRNA levels in the liver of mice in each group. It can be seen that the absolute siRNA levels of mice in the CMV-siR^{mHTT} group and CMV-RVG-siR^{mHTT} group were higher. As shown in Figure 46B, this figure shows the comparison of the absolute siRNA levels in the plasma of mice in each group. It can be seen that the absolute siRNA levels of mice in the CMV-siR^{mHTT} group and CMV-RVG-siR^{mHTT} group were higher. As shown in Figure 46C, this figure shows the comparison of the absolute siRNA levels in the cortex and striatum of mice in each group. It can be seen that the absolute siRNA levels of mice injected with CMV-RVG-siR^{mHTT} were higher.

As shown in Figure 46D, this figure shows the in situ hybridization of mouse liver, cortex and striatum tissue in each group. It can be seen that the liver tissue sections of mice in the CMV siR^{mHTT} group and CMV-RVG-siR^{mHTT} group showed obvious fluorescence, and the cortex and striatum tissue sections of mice in the CMV-RVG-siR^{mHTT} group showed obvious fluorescence. This shows that RVG can guide exosomal siRNA to enter and pass through the blood-brain barrier and exert its function.

In the second experiment, 2 experimental groups and 2 control groups were set up. The experimental groups were CMV-siR^{GFP} group and CMVRVG-siR^{GFP} group, and the control groups were PBS group and CMV-scrR group. GFP transgenic mice in the CMV-siR^{GFP} group, CMV RVG-siR^{GFP} group, PBS group, and CMV-scrR group were injected with CMV-siR^{GFP} plasmid, CMV-RVG-siR^{GFP} plasmid, PBS solution, and CMV-scrR plasmid through the tail vein, respectively.

As shown in Figure 46E and Figure 46F, the figure shows the sections of the liver, cortex and striatum of mice in each group. It can be seen that the transgenic mice injected with CMV siR^{GFP}/CMV-RVG-siR^{GFP} in the liver had reduced GFP fluorescence level, and the transgenic mice injected with CMV-RVG-siR^{GFP} in the cortex and striatum had reduced GFP fluorescence level. This shows that RVG can guide exosomal siRNA to enter and pass through the blood-brain barrier and exert its function.

In the third experiment, two experimental groups and one control group were set up. The experimental groups were CMV-siR^{mHTT} group and CMV-RVG-siR^{mHTT} group, and the control group was CMV-scrR group.

The experimental protocol is shown in Figure 47A. The 8-week-old N17182Q mice were selected, and mice with Huntington's disease in the CMV-siR^{mHTT} group, CMV-RVG-siR^{mHTT} group, and the CMV-scrR group were injected with CMV-siR^{mHTT} plasmid, CMV-RVG-siR^{mHTT} plasmid and CMV-scrR plasmid through the tail vein, respectively. Rotation test was performed on days 0 and 14, and mice were sacrificed after 14 days for analysis.

As shown in Figure 47B, this figure shows the comparison of the descending latency of wild-type mice and mice in the CMV-scrR group and CMV-RVG-siR^{mHTT} group. It can be seen that on day 0, the descending latency of mice in the CMV-scrR group and CMV-RVG-siR^{mHTT} group was relatively consistent. On day 14, the descending latency of mice in the CMV-scrR group was the shortest.

As shown in Figure 47C and Figure 47D, Figure 47C shows the western blot results of the striatum of mice in the CMV-scrR group and CMV-RVG-siR^{mHTT} group, and Figure 47D shows the comparison of the relative mHTT mRNA levels in the striatum of mice in the CMV-scrR group and CMV-RVG-siR^{mHTT} group. It can be seen that mice in the CMV-scrR group had higher N171-mHTT protein level and higher relative mHTT mRNA level in the striatum.

In the fourth experiment, one experimental group and one control group were set up. The experimental group was CMV-RVG-siR^{mHTT} group, and the control group was CMV-scrR group.

The experimental protocol is shown in Figure 47E. The 3-month-old BACHD mice were selected. Mice with Huntington's disease in the CMV-RVG-siR^{mHTT} group and CMV-scrR group were injected with CMV-RVG-siR^{mHTT} plasmid and CMV-scrR plasmid through the tail vein, respectively, and were sacrificed after 14 days for analysis.

As shown in Figure 47F, Figure 47F shows the western blot results of the cortex and striatum of mice in the CMV-scrR group and CMV-RVG-siR^{mHTT} group, and it can be seen that mice in the CMV-RVG-siR^{mHTT} group had less mutant HTT and endogenous HTT in the cortex and striatum.

As shown in Figure 47G, Figure 47G shows the comparison of the relative mHTT protein levels in the cortex and striatum of mice in the CMV-scrR group and the CMVRVG-siR^{mHTT} group. It can be seen that whether in the mouse cortex or the striatum, the relative mHTT protein levels of mice in the CMV-RVG-siR^{mHTT} group were lower.

As shown in Figure 47H and Figure 47I, Figure 47H shows the immunofluorescence images of mice in the CMV-scrR group and CMV-RVG-siR^{mHTT} group, and Figure 47I shows the comparison of the relative mHTT mRNA levels in the cortex and striatum of mice in the CMV scrR group and the CMV-RVG-siR^{mHTT} group. It can be seen that whether in the mouse cortex or the striatum, the relative mHTT mRNA levels of mice in the CMVRVG-siR^{mHTT} group were lower.

The above experiments demonstrate that intravenous injection of CMV-RVG-siR^{mHTT} plasmid helps suppress mHTT in the striatum and cortex, thereby improving exercise capacity and alleviating neuropathology in HD mice.

In the fifth experiment, one experimental group and one control group were set up. The experimental group was the CMV-RVG-siR^{mHTT} group, and the control group was the CMV-scrR group.

The experimental protocol is shown in Figure 48A. The 6-week-old YAC128 mice were selected. Mice with Huntington's disease in the CMV-RVG-siR^{mHTT} group and CMV-scrR group were injected with CMV-RVG-siR^{mHTT} plasmid and CMV-scrR plasmid through the tail vein, respectively. The rotation test was performed on the 0th day, 4th week, and 8th week of the experiment, and then the mice were sacrificed for analysis.

As shown in Figure 48B, this figure shows the comparison of the descending latency of wild-type mice and mice in the CMV-RVG-siR^{mHTT} group and CMV-scrR group. It can be seen that on day 0, the descending latency of mice in the CMV-RVG-siR^{mHTT} group and CMV-scrR group was relatively consistent. At the fourth and eighth weeks, the descending latency of mice in the CMV-scrR group was the shortest.

As shown in Figure 48C, this figure shows the western blot results of the cortex and striatum of mice in the CMV-RVG-siR^{mHTT} group and CMV-scrR group. It can be seen that mice in the CMV-RVG-siR^{mHTT} group had lower mutant HTT and endogenous HTT contents in the cortex, and had lower mutant HTT and higher endogenous HTT contents in the striatum.

As shown in Figure 48D and Figure 48E, the two figures show the relative mHTT mRNA levels and the relative mHTT protein levels in the cortex and striatum of mice in the CMV-RVG-siR^{mHTT} group and CMV-scrR group. It can be seen that the relative mHTT mRNA levels and the relative mHTT protein levels of mice in the CMV-RVG-siR^{mHTT} group were relatively lower in both cortex and striatum.

As shown in Figure 48F, this figure show immunofluorescence images of the cortex and striatum of mice in the CMV-RVG-siR^{mHTT} group and CMV-scrR group. It can be seen that the expression levels of NeuN and EM48 of mice in the CMV-RVG-siR^{mHTT} group were lower than those in the CMV-scrR group.

The above experiments can demonstrate that intravenous injection of CMVRVG-siR^{mHTT} plasmid can help reduce mHTT protein and toxic aggregates in the striatum and cortex, thereby improving behavioral defects and neuropathology in the striatum and cortex.

In addition, similar experiments were performed on viral vectors. Mice were injected with 100 µL of AAV solution with a titer of 10¹² V g/ml through the tail vein. The in vivo expression of the AAV system was monitored by small animal in vivo imaging, and after 3 weeks, the AAV system was stably expressed in the body, especially in the liver.

Mice were then selected for modeling. After the completion of modeling, mice were injected with PBS buffer/AAV-CMV-scrR/AAV-CMV-siR^{mHTT}/AAV-CMV-RVG-siR^{mHTT} to obtain PBS group/AAV CMV scrR group/AAV-CMV-siR^{mHTT} group/AAV-CMV-RVG-siR^{mHTT} group. After the above solution was injected into the tail vein, the plasma exosomes were isolated, labeled with PKH26 dye, and co-cultured with cells to observe the absorption of exosomes by cells. The results are as follows.

As shown in Figure 49A, the figure shows the comparison of siRNA levels in plasma exosomes of mice in each group. It can be seen that the levels of siRNA in plasma exosomes of mice in the AAV-CMV-siR^{mHTT} group and AAV-CMV-RVG-siR^{mHTT} group were higher.

As shown in Figure 49B, this figure shows the comparison of the relative mHTT mRNA levels of mice in each group after co-culturing the mouse plasma exosomes with cells. It can be seen that the mice in the AAV-CMV-siR^{mHTT} group and AAV-CMV-RVG-siR^{mHTT} group had lower relative mHTT mRNA levels, indicating that AAV-CMVsiR^{mHTT} and AAV-CMV-RVG-siR^{mHTT} could reduce HTT mRNA levels, meaning siRNA assembled into exosomes could still exert gene silencing function.

As shown in Figure 49C, this figure shows the comparison of absolute levels of siRNA in mouse liver. It can be seen that the absolute levels of siRNA of mice in the AAV-CMV-siR^{mHTT} group and AAV-CMVRVG-siR^{mHTT} group were higher.

As shown in Figure 49D, this figure shows the comparison of absolute levels of siRNA in mouse plasma. It can be seen that the absolute levels of siRNA of mice in the AAV-CMV-siR^{mHTT} group and AAV-CMVRVG-siR^{mHTT} group were higher.

As shown in Figure 49E, this figure shows the comparison of the descending latency of wild-type mice (WT) and mice in the AAV-CMV-siR^{mHTT} group and AAV-CMV-RVG-siR^{mHTT} group. It can be seen that at 0 weeks, the descending latency of mice in the three group was relatively consistent. At the 4th and 8th weeks, the descending latency of mice in the CMV-scrR group was the shortest.

As shown in Figure 49F, this figure shows the comparison of the relative mHTT mRNA levels in the cortex and striatum of mice in the AAV-CMV-scrR group and AAV-CMV-RVG-siR^{mHTT} group. It can be seen that whether in the cortex or the striatum, the mHTT mRNA levels of mice in the AAV-CMV-RVG-siR^{mHTT} group were lower than those in the AAV-CMV-scrR group.

The above experiments can demonstrate that intravenous injection of AAV-CMV-RVG-siR^{mHTT} can help reduce mHTT protein and toxic aggregates in the striatum and cortex, thereby exerting a therapeutic effect on Huntington's disease.

In the case where the viral vector carries two or more circuits, adjacent gene circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

### Example 15

The plasmid CMV-siR^{LRRK2} carrying LRRK2 siRNA that inhibits LRRK2 expression, and the plasmid CMV-RVG-siR^{LRRK2} carrying targeting peptide RVG and LRRK2 siRNA were constructed according to the method described in Example 2. Wherein, the siRNA of LRRK2 gene is AUUAACAUGAAAAUAUCACUU (SEQ ID NO. 16).

Use of the vector system in the treatment of Parkinson's disease was investigated.

In this experiment, LRRK2R1441G transgenic mice were selected for the experiment when they were 3 months old, and an LPS intervention group and an LPS non-intervention group were set up. The LPS intervention group was treated with CMV-scrR/CMV-RVG-siR^{LRRK2} 7 days after LPS intervention.

As shown in Figure 50A and Figure 50B, Figure 50A shows the western blot results of LRRK2R1441G transgenic mice injected with CMV-scrR/CMV-RVG-siR^{LRRK2}, and Figure 50B shows the protein grayscale analysis of LRRK2R1441G transgenic mice injected with CMV-scrR/CMV-RVG-siR^{LRRK2}. It can be seen that the levels of LRRK2 protein and S935 protein in mice injected with CMV-RVG-siR^{LRRK2} were reduced, indicating that CMV-RVG-siR^{LRRK2} can cross the blood-brain barrier and reduce the expression of deep brain proteins after siRNA was released from the liver and assembled into exosomes.

As shown in Figure 50C, this figure shows immunofluorescence images of TH+ neurons in the substantia nigra area of LRRK2R1441G transgenic mice injected with CMV-scrR/CMV-RVG-siR^{LRRK2}. The results show that the loss of TH neurons was rescued in mice injected with CMV-RVG-siR^{LRRK2}, indicating that CMV-RVG-siR^{LRRK2} can cross the blood-brain barrier and enter the deep brain to function after siRNA was released from the liver and assembled into exosomes.

As shown in Figure 50D, this figure shows immunofluorescence images of microglial activation levels in LRRK2R1441G transgenic mice injected with CMV-scrR/CMV-RVG-siR^{LRRK2}. The results show that microglia activation can be inhibited in mice injected with CMV-RVG-siR^{LRRK2}, indicating that CMV-RVG-siR^{LRRK2} can cross the blood-brain barrier and enter the deep brain to function after siRNA was released from the liver and assembled into exosomes.

The above experiments can demonstrate that intravenous injection of CMV-RVG-siR^{LRRK2} plasmid helps suppress LRRK2 in dopaminergic neurons, thereby reducing the development of neuropathology in mice with Parkinson's disease.

In the case where the plasmid carries two or more circuits, adjacent circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAGTG |
| 60 bases | TGGCCGCACTCGAGGTAGTGAGTCGACCAGTGGCCGCACTCGAGGTAGTG AGTCGACCAG |
| 70 bases | TGGCCGCACTCGAGGTAGTGAGTCGACCAGTGGCCGCACTCGAGGTAGTG AGTCGACCAGTGGCCGCACT |
| 80 bases | TGGCCGCACTCGAGGTAGTGAGTCGACCAGTGGCCGCACTCGAGGTAGTG AGTCGACCAGTGGCCGCACTCGAGGTAGTG |

More preferably, in the case where the plasmid carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

In the case where the viral vector carries two or more circuits, adjacent gene circuits can be linked via sequence 1-sequence 2-sequence 3; wherein, sequence 1 is preferably CAGATC, sequence 2 may be a sequence of 5-80 bases, such as 10, 15, 20, 25, 30, 35, 40, 45, 50, 55, 60, 65, 70, 75 bases, preferably a sequence of 10-50 bases, more preferably a sequence of 20-40 bases, and sequence 3 is preferably TGGATC.

Sequence 2 is specifically shown in the table below.

| | |
|---|---|
| 20 bases | TGGCCGCACTCGAGGTAGTG |
| 30 bases | TGGCCGCACTCGAGGTAGTGAGTCGACCAG |
| 40 bases | TGGCCGCACTCGAGGTAGTGAGTCGACCAGTGGCCGCACT |
| 50 bases | TGGCCGCACTCGAGGTAGTGAGTCGACCAGTGGCCGCACTCGAGGTAG TG |
| 60 bases | TGGCCGCACTCGAGGTAGTGAGTCGACCAGTGGCCGCACTCGAGGTAG TGAGTCGACCAG |
| 70 bases | TGGCCGCACTCGAGGTAGTGAGTCGACCAGTGGCCGCACTCGAGGTAG TGAGTCGACCAGTGGCCGCACT |
| 80 bases | TGGCCGCACTCGAGGTAGTGAGTCGACCAGTGGCCGCACTCGAGGTAG TGAGTCGACCAGTGGCCGCACTCGAGGTAGTG |

More preferably, in the case where the viral vector carries two or more circuits, adjacent gene circuits are linked via sequence 4 or a sequence with more than 80% homology to sequence 4; wherein sequence 4 is CAGATCTGGCCGCACTCGAGGTAGTGAGTCGACCAGTGGATC.

The sequences are specifically shown in the table below.

| | |
|---|---|
| Sequence 4-1 | CAGATCTGGCCGCACTCGAGGTAGTGAGTCGACCAGTGGATC |
| Sequence 4-2 | CAGATCTGGCCGCACTCGTAGAGGTGAGTCGACCAGTGGATC |
| Sequence 4-3 | CAGATCTGGCACCCGTCGAGGTAGTGAGTCGACCAGTGGATC |
| Sequence 4-4 | CAGATCTGGCCGCACAGGTCGTAGTGAGTCGACCAGTGGATC |

### Example 16 Safety detection

The RNA delivery vector system provided by the present invention was studied in more detail in Macaca fascicularis, a well-known non-human primate model used in safety assessment studies.

With ethical approval, 4 adult macaques were used for intravenous injection of 5 mg/kg CMV-siR^{E} plasmid, and blood samples were collected before injection or at different time points after injection. One month later, these macaques were intravenously injected with 5 mg/kg CMV siR^{E} plasmid daily for a total of 5 times, and blood samples were collected before injection or at different time points after the last injection.

As shown in Figure 51, Figure 51A shows siRNA concentration changes in whole blood of Macaca fascicularis with a single injection. Figure 51B shows siRNA concentration changes in whole blood of Macaca fascicularis with multiple injections. It can be seen that the concentration of siRNA in Macaca fascicularis with a single injection reached a peak 6 h after intravenous injection, and then decreased. The concentration of siRNA in Macaca fascicularis with multiple injections reached a peak 3 h after intravenous injection, and then decreased. The siRNA concentration of Macaca fascicularis with multiple injections decreased more slowly.

The above experiments can show that CMV-siR^{E} plasmid can be safe and effective for primates such as Macaca fascicularis.

The above is a description of the present invention, and it cannot be regarded as a limitation of the present invention. Unless otherwise indicated, the practice of the present invention will employ conventional techniques of organic chemistry, polymer chemistry, biotechnology, etc. It will be apparent that the present invention may be carried out in other ways than those specifically described in the above description and examples. Other aspects and modifications within the scope of the invention will be apparent to those skilled in the art to which this invention belongs. Many modifications and variations are possible in light of the teachings of the present invention and are therefore within the scope of the present invention.

## Claims

1. An isolated nucleic acid comprising a nucleotide sequence encoding an RNA capable of inhibiting gene expression, comprising:
(a) a nucleotide sequence encoding one or more RNAs that inhibit gene expression, and the RNA is selected from the group consisting of miRNA, shRNA, siRNA, mRNA, ncRNA, sgRNA, and a combination thereof;
wherein, the nucleotide sequence comprises an RNA fragment sequence targeting the gene, and one or more of a flanking sequence (such as a 5' flanking sequence and a 3' flanking sequence), a stem-loop sequence and a compensation sequence of the RNA fragment sequence;
preferably, the nucleotide sequence encoding one or more RNAs that inhibit gene expression comprises successively: 5' flanking sequence, RNA fragment sequence, stem-loop sequence, compensation sequence and 3' flanking sequence; and
optionally, (b) a nucleotide sequence encoding a targeting protein.

2. The nucleic acid according to claim 1, wherein (a) is a nucleotide sequence encoding an RNA that inhibits gene expression.

3. The nucleic acid according to claim 1, wherein (a) is a nucleotide sequence encoding a plurality of RNAs that inhibit gene expression, preferably, the plurality of RNAs that inhibit gene expression are 2-4 RNAs that inhibit gene expression.

4. The nucleic acid according to any one of claims 1-3, wherein the RNA is siRNA.

5. The nucleic acid according to any one of claims 1-3, wherein the nucleotide sequence encoding one or more RNAs that inhibit gene expression has 15-29 nucleotides in length, preferably 21-23 nucleotides in length.

6. The nucleic acid according to claim 1, wherein the targeting protein is a target tissue-specific targeting peptide.

7. The nucleic acid according to claim 1, wherein the targeting protein is a fusion protein of a target tissue-specific targeting peptide and a membrane protein.

8. The nucleic acid according to claim 6 or 7, wherein the target tissue-specific targeting peptide is selected from the group consisting of RVG targeting peptide, GE11 targeting peptide, PTP targeting peptide, TCP-1 targeting peptide, and MSP targeting peptide.

9. The nucleic acid according to claim 6 or 7, wherein the membrane protein is selected from the group consisting of a receptor protein (such as a growth factor receptor), LAMP1 or LAMP2 (such as LAMP2B), and an antibody or an antigen-binding fragment thereof.

10. A vector of RNA that inhibits gene expression, comprising:
(a) a nucleotide sequence encoding one or more RNAs that inhibit gene expression, and the RNA is selected from the group consisting of miRNA, shRNA, siRNA, mRNA, ncRNA, sgRNA, and a combination thereof; and
optionally, (b) a nucleotide sequence encoding a targeting protein.

11. The vector according to claim 10, which is a plasmid.

12. The vector according to claim 10, which is a viral vector, such as a baculovirus expression vector, an adenoviral vector, a retroviral vector, a herpes viral vector or a lentiviral vector, preferably an adenoviral vector.

13. A pharmaceutical composition comprising the nucleic acid according to any one of claims 1-9 or the vector according to any one of claims 10-12.

14. A method of treating a disease, comprising administering to a subject the nucleic acid according to any one of claims 1-9 or the vector according to any one of claims 10-12.
